# EUROPEAN PATENT SPECIFICATION

(11) **EP 1 635 837 B1**
(45) Date of publication and mention of the grant of the patent: **16.07.2008**
(21) Application number: 04735028.5
(22) Date of filing: 27.05.2004
(51) Int. Cl.: A61K 31/517, C07D 403/14, C07D 403/12, C01B 25/26, A61P 43/00

(54) **(3-((QUINAZOLIN-4-YL)AMINO)-1H-PYRAZOL-1-YL)ACETAMIDE DERIVATIVES AND RELATED COMPOUNDS AS AURORA KINASE INHIBITORS FOR THE TREATMENT OF PROLIFERATIVE DISEASES SUCH AS CANCER**
(3-((CHINAZOLIN-4-YL)AMINO)-1H-PYRAZOL-1-YL)ACETAMID DERIVATE UND VERWANDTE VERBINDUNGEN ALS AURORA KINASE INHIBITOREN ZUR BEHANDLUNG VON PROLIFERATIVEN ERKRANKUNGEN WIE KREBS
DERIVES DE (3-((QUINAZOLIN-4-YL) AMINO)-1H-PYRAZOL-1-YL) ACETAMIDE ET COMPOSES ASSOCIES EN TANT QU'INHIBITEURS DES KINASES AURORA POUR LE TRAITEMENT DE MALADIES PROLIFERATIVES TELLES QUE LE CANCER

(30) Priority: 02.06.2003 EP 03291314
(43) Date of publication of application: 22.03.2006
(73) Proprietor: AstraZeneca AB, 151 85 Södertälje (SE)
(72) Inventor: MORTLOCK, Andrew Austen, Macclesfield, Cheshire SK10 4TG (GB); HERON, Nicola Murdoch, Macclesfield, Cheshire SK10 4TG (GB); JUNG, Frederic Henri, AstraZeneca Pharma, 51689 Reims (FR); PASQUET, Georges Rene, AstraZeneca Pharma, 51689 Reims (FR)
(86) International application number: PCT/GB2004/002281
(87) International publication number: WO 2004/105764

(56) References cited:
- WO-A-01/21596
- WO-A-02/00649
- WO-A-02/50065
- WO-A-03/055491

## Description

The present invention relates to quinazoline derivatives for use in the treatment of disease, in particular proliferative diseases such as cancer and in the preparation of medicaments for use in the treatment of proliferative diseases, and to processes for their preparation, as well as pharmaceutical compositions containing them as active ingredient.

Cancer (and other hyperproliferative diseases) are characterised by uncontrolled cellular proliferation. This loss of the normal regulation of cell proliferation often appears to occur as the result of genetic damage to cellular pathways that control progress through the cell cycle.

In eukaryotes, an ordered cascade of protein phosphorylation is thought to control the cell cycle. Several families of protein kinases that play critical roles in this cascade have now been identified. The activity of many of these kinases is increased in human tumours when compared to normal tissue. This can occur by either increased levels of expression of the protein (as a result of gene amplification for example), or by changes in expression of co activators or inhibitory proteins.

The first identified, and most widely studied of these cell cycle regulators have been the cyclin dependent kinases (or CDKs). Activity of specific CDKs at specific times is essential for both initiation and coordinated progress through the cell cycle. For example, the CDK4 protein appears to control entry into the cell cycle (the G0-G1-S transition) by phosphorylating the retinoblastoma gene product pRb. This stimulates the release of the transcription factor E2F from pRb, which then acts to increase the transcription of genes necessary for entry into S phase. The catalytic activity of CDK4 is stimulated by binding to a partner protein, Cyclin D. One of the first demonstrations of a direct link between cancer and the cell cycle was made with the observation that the Cyclin D1 gene was amplified and cyclin D protein levels increased (and hence the activity of CDK4 increased) in many human tumours (Reviewed in Sherr, 1996, Science 274: 1672-1677; Pines, 1995, Seminars in Cancer Biology 6: 63-72). Other studies (Loda et al., 1997, Nature Medicine 3(2): 231-234; Gemma et al., 1996, International Journal of Cancer 68(5): 605-11; Elledge et al. 1996, Trends in Cell Biology 6; 388-392) have shown that negative regulators of CDK function are frequently down regulated or deleted in human tumours again leading to inappropriate activation of these kinases.

More recently, protein kinases that are structurally distinct from the CDK family have been identified which play critical roles in regulating the cell cycle and which also appear to be important in oncogenesis. They include the human homologues of the *Drosophila* aurora and *S. cerevisiae* Ipl1 proteins. The three human homologues of these genes Aurora-A, Aurora-B and Aurora-C (also known as aurora2, auroral and aurora3 respectively) encode cell cycle regulated serine-threonine protein kinases (summarised in Adams et al., 2001, Trends in Cell Biology. 11(2): 49-54). These show a peak of expression and kinase activity through G2 and mitosis. Several observations implicate the involvement of human aurora proteins in cancer. This evidence is strong for Aurora-A. The Aurora-A gene maps to chromosome 20q13, a region that is frequently amplified in human tumours including both breast and colon tumours. Aurora-A may be the major target gene of this amplicon, since Aurora-A DNA is amplified and mRNA overexpressed in greater than 50% of primary human colorectal cancers. In these tumours Aurora-A protein levels appear greatly elevated compared to adjacent normal tissue. In addition, transfection of rodent fibroblasts with human Aurora-A leads to transformation, conferring the ability to grow in soft agar and form tumours in nude mice (Bischoff et al., 1998, The EMBO Journal. 17(11): 3052-3065). Other work (Zhou et al., 1998, Nature Genetics. 20(2): 189-93) has shown that artificial overexpression of Aurora-A leads to an increase in centrosome number and an increase in aneuploidy, a known event in the development of cancer. Further work has shown an increase in expression of Aurora-B (Adams et al., 2001, Chromsoma. 110(2):65-74) and Aurora-C (Kimura et al., 1999, Journal of Biological Chemistry, 274(11): 7334-40) in tumour cells when compared to normal cells.

Importantly, it has also been demonstrated that abrogation of Aurora-A expression and function by antisense oligonucleotide treatment of human tumour cell lines (WO 97/22702 and WO 99/37788) leads to cell cycle arrest and exerts an antiproliferative effect in these tumour cell lines. Additionally, small molecule inhibitors of Aurora-A and Aurora-B have been demonstrated to have an antiproliferative effect in human tumour cells (Keen et al. 2001, Poster #2455, American Association of Cancer research annual meeting), as has selective abrogation of Aurora-B expression alone by siRNA treatment (Ditchfield etal., 2003, Journal of Cell Biology, 161(2):267-280). This indicates that inhibition of the function of Aurora-A and/or Aurora-B will have an antiproliferative effect that may be useful in the treatment of human tumours and other hyperproliferative diseases. Further, inhibition of Aurora kinases as a therapeutic approach to these diseases may have significant advantages over targeting signalling pathways upstream of the cell cycle (e.g. those activated by growth factor receptor tyrosine kinases such as epidermal growth factor receptor (EGFR) or other receptors). Since the cell cycle is ultimately downstream of all of these diverse signalling events, cell cycle directed therapies such as inhibition of Aurora kinases would be predicted to be active across all proliferating tumour cells, whilst approaches directed at specific signalling molecules (e.g. EGFR) would be predicted to be active only in the subset of tumour cells which express those receptors. It is also believed that significant "cross talk" exists between these signalling pathways meaning that inhibition of one component may be compensated for by another.

A number of quinazoline derivatives have been proposed hitherto for use in the inhibition of Aurora kinases. For example, WO 01/21594, WO 01/21595 and WO 01/215968 describe the use of certain phenyl-quinazoline compounds as Aurora-A kinase inhibitors, which may be useful in the treatment of proliferative diseases and WO 01/21597 discloses other quinazoline derivatives as inhibitors of Aurora-A kinase. Additionally, WO 02/00649 discloses quinazoline derivative bearing a 5-membered heteroaromatic ring where the ring is, in particular, substituted thiazole or substituted thiophene. However despite the compounds of WO 02/00649 there still exists a need for further compounds having Aurora kinase inhibitory properties.

The applicants have been successful in finding a novel series of compounds which inhibit the effects of the Aurora kinases and in particular Aurora-A kinase and/or Aurora-B kinase which are thus of use in the treatment of proliferative diseases such as cancer. In particular, the compounds may be used to treat either solid or haematological tumours and more particularly colorectal, breast, lung, prostate, bladder, renal or pancreatic cancer or leukaemia or lymphoma. In addition certain aspects of the invention make them useful in the formulation of medicaments for the treatment of disease.

According to one aspect of the invention there is provided a compound of formula (I) 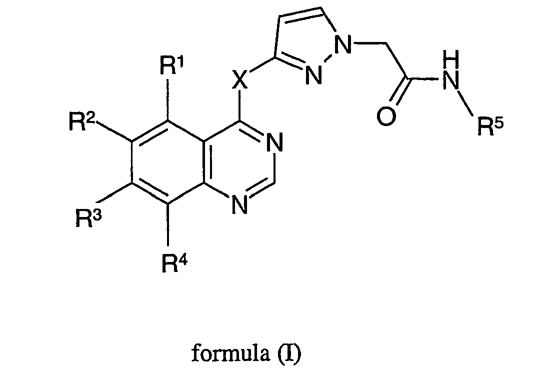 or a salt or ester thereof;
where:
**X** is O or NR⁶;
**R⁶** is hydrogen or C₁₋₄alkyl;
**R¹** is hydrogen, halo, or -X¹R¹¹;
**X¹** is a direct bond, -O-, -NH- or -N(C₁₋₆alkyl)-;
**R¹¹** is hydrogen, heterocyclyl or a group selected from C₁₋₆alkyl, C₂₋₆alkenyl, C₂₋₆alkynyl, C₃₋₆ cycloalkyl and C₃₋₆cycloalkenyl which group is optionally substituted by heterocyclyl, halo, hydroxy, C₁₋₄alkoxy or -NR⁹R¹⁰;
**R²** is hydrogen, halo, nitro, cyano or -X²R¹²;
**X² is** a direct bond, -O-, -NH- or -N(C₁₋₆alkyl)-;
**R¹²** is hydrogen, heterocyclyl or a group selected from aryl, C₁₋₆alkyl, C₂₋₆alkenyl, C₂₋₆ alkynyl, C₃₋₆cycloalkyl and C₃₋₆cycloalkenyl which group is optionally substituted by aryl, heterocyclyl, halo, hydroxy or -NR¹⁵R¹⁶;
**R³** is hydrogen, halo or -X³R¹³;
**X³** is a direct bond, -CH₂=CH₂-, -O-, -NH- or -N(C₁₋₆alkyl)-;
**R¹³** is hydrogen, heterocyclyl or a group selected from C₁₋₆alkyl, C₂₋₆alkenyl, C₂₋₆alkynyl, C₃₋₆ cycloalkyl and C₃₋₆cycloalkenyl which group is optionally substituted by -NR⁷R⁸, heterocyclyl, halo, hydroxy or C₁₋₄alkoxy;
**R⁷** and **R⁸** are independently selected from hydrogen, heterocyclyl, C₁₋₆alkyl, hydroxyC₁₋₆ alkyl, C₁₋₃alkoxyC₁₋₆alkyl, C₃₋₆cycloalkyl, C₃₋₆cycloalkylC₁₋₃alkyl, hydroxyC₃₋₆cycloalkyl, hydroxyC₁₋₄alkylC₃₋₆cycloalkyl, hydroxyC₃₋₆cycloalkylC₁₋₃alkyl, C₁₋₃alkoxyC₃₋₆Cycloalkyl, C₁₋₃alkoxyC₃₋₆cycloalkylC₁₋₃alkyl, haloC₁₋₆alkyl, haloC₃₋₆cycloalkyl, haloC₃₋₆cycloalkylC₁₋₃ alkyl, C₂₋₆alkenyl, C₂₋₆alkynyl, cyanoC₁₋₄alkyl, aminoC₁₋₆alkyl, C₁₋₃alkylaminoC₁₋₆alkyl and bis(C₁₋₃alkyl)aminoC₁₋₆alkyl;
or **R⁷** and **R⁸** together with the nitrogen to which they are attached form a heterocyclic ring which ring comprises 4 to 7 ring atoms of which one is nitrogen and of which another is optionally selected from N, NH, O, S, SO and SO₂, and which ring is optionally substituted on carbon or nitrogen by 1 or 2 groups independently selected from C₁₋₄alkyl, hydroxy, C₁₋₄ alkoxy, hydroxyC₁₋₄alkyl, hydroxyC₁₋₄alkoxyC₁₋₄alkyl and C₁₋₄alkoxyC₁₋₄alkoxy, and where a ring -CH₂- is optionally replaced with -C(O)-;
**R⁴** is selected from hydrogen, halo or -X⁴R¹⁴;
**X⁴** is a direct bond, -O-, -NH- or -N(C₁₋₆alkyl)-;
**R¹⁴** is selected from hydrogen, C₁₋₆alkyl, C₂₋₆alkenyl and C₂₋₆alkynyl;
**R⁵** is aryl or heteroaryl optionally substituted by 1, 2 or 3 substituents independently selected from halo, hydroxy, cyano, nitro, amino, C₁₋₄alkylamino, bis(C₁₋₄alkyl)amino, C₁₋₄alkyl, C₂₋₄ alkenyl, C₂₋₄alkynyl, C₁₋₄alkoxy, C(O)NHR¹⁷, NHC(O)R¹⁸ and S(O)ₚR¹⁹ where p is 0, 1 or 2;
**R⁹**, **R¹⁰**, **R¹⁵** and **R¹⁶** are independently selected from hydrogen, C₁₋₆alkyl, C₃₋₆cycloalkyl, C₃₋₆ cycloalkylC₁₋₃alkyl, hydroxyC₁₋₆alkyl, haloC₁₋₆alkyl, aminoC₁₋₆alkyl, C₁₋₆alkylaminoC₁₋₆alkyl and bis(C₁₋₆alkyl)aminoC₁₋₆alkyl;
**R¹⁷**, **R¹⁸** and **R¹⁹** are independently selected from hydrogen, C₁₋₄alkyl, C₃₋₆cycloalkyl, C₂₋₄ alkenyl and C₂₋₄alkynyl.

As a further aspect a compound of formula (I) or a pharmaceutically acceptable salt thereof is provided.

In another aspect the invention provides a compound of formula (IA) 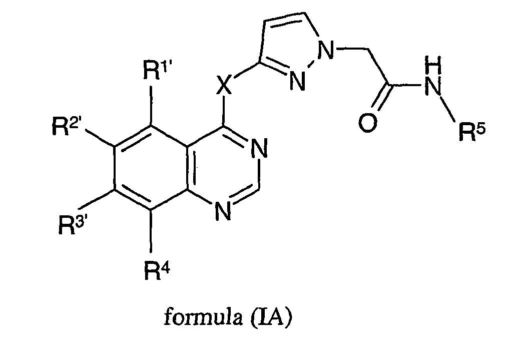 or a salt or ester thereof ;
where X, X¹, X², X³, R⁴ and R⁵ are as defined in relation to formula (I) and
**R^{1'}** is hydrogen, halo, or-X¹R^{11'};
**R^{11'}** is hydrogen, phosphonooxy, heterocyclyl or a group selected from C₁₋₆alkyl, C₂₋₆alkenyl, C₂₋₆alkynyl, C₃₋₆cycloalkyl and C₃₋₆cycloalkenyl which group is optionally substituted by phosphonooxy, heterocyclyl, halo, hydroxy, C₁₋₄alkoxy or -NR^{9'}R^{10'};
**R^{2'}** is hydrogen, halo, nitro, cyano or -X²R^{12'};
**R^{12'}** is hydrogen, phosphonooxy, heterocyclyl or a group selected from aryl, C₁₋₆alkyl, C₂₋₆ alkenyl, C₂₋₆alkynyl, C₃₋₆cycloalkyl and C₃₋₆cycloalkenyl which group is optionally substituted by phosphonooxy, aryl, heterocyclyl, halo, hydroxy or -NR^{15'}R^{16'};
R^{3'} is hydrogen, halo or -x³R^{13'};
**R^{13'}** is phosphonooxy or a group selected from C₁₋₆alkyl, C₂₋₆alkenyl, C₂₋₆alkynyl, C₃₋₆ cycloalkyl and C₃₋₆cycloalkenyl which group is substituted by -NR^{7'}R^{8'}, heterocyclyl, halo, hydroxy, phosphonooxy or C₁₋₄alkoxy;
**R^{7'}** and **R^{8'}** are independently selected from hydrogen, heterocyclyl, C₁₋₆alkyl, hydroxyC₁₋₆ alkyl, phosphonooxyC₁₋₆alkyl, C₁₋₃alkoxyC₁₋₆alkyl, phosphonooxyC₁₋₄alkoxyC₁₋₄alkyl, C₃₋₆ cycloalkyl, C₃₋₆cycloalkylC₁₋₃alkyl, hydroxyC₃₋₆cycloalkyl, phosphonooxyC₃₋₆cycloalkyl, hydroxyC₁₋₄alkylC₃₋₆cycloalkyl, phosphonooxyC₁₋₄alkylC₃₋₆cycloalkyl, hydroxyC₃₋₆ cycloalkylC₁₋₃alkyl, phosphonooxyC₃₋₆cycloalkylC₁₋₃alkyl, C₁₋₃alkoxyC₃₋₆cycloalkyl, C₁₋₃ alkoxyC₃₋₆cycloalkylC₁₋₃alkyl, haloC₁₋₆alkyl, haloC₃₋₆cycloalkyl, haloC₃₋₆cycloalkylC₁₋₃alkyl, C₂₋₆alkenyl, C₂₋₆alkynyl, cyanoC₁₋₄alkyl, aminoC₁₋₆alkyl, C₁₋₃alkylaminoC₁₋₆alkyl and bis(C₁₋₃ alkyl)aminoC₁₋₆alkyl;
or **R⁷'** and **R⁸'** together with the nitrogen to which they are attached form a heterocyclic ring which ring comprises 4 to 7 ring atoms of which one is nitrogen and of which another is optionally selected from N, NH, O, S, SO and SO₂, and which ring is substituted on carbon or nitrogen by 1 or 2 groups independently selected from C₁₋₄alkyl, hydroxy, phosphonooxy, C₁₋₄ alkoxy, hydroxyC₁₋₄alkyl, phoshonooxyC₁₋₄alkyl, hydroxyC₁₋₄alkoxyC₁₋₄alkyl, phosphonooxyC₁₋₄alkoxyC₁₋₄alkyl and C₁₋₄alkoxyC₁₋₄alkoxy, and where a ring -CH₂- is optionally replaced with a -C(O)-;
**R^{9'}**, **R^{10'}**, **R^{15'}** and **R**^{**16**'} are independently selected from hydrogen, C₁₋₆alkyl, C₃₋₆cycloalkyl, C₃₋₆cycloalkylC₁₋₃alkyl, hydroxyC₁₋₆alkyl, phosphonooxyC₁₋₆alkyl, haloC₁₋₆alkyl, aminoC₁₋₆ alkyl, C₁₋₆alkylaminoC₁₋₆alkyl and bis(C₁₋₆alkyl)aminoC₁₋₆alkyl;
provided that a compound of formula (IA) contains at least one phosphonooxy group.

In a preferred embodiment a compound of formula (IA) contains only one phosphonooxy group.

As a further aspect a compound of formula (IA) or a pharmaceutically acceptable salt thereof is provided.

In this specification the term alkyl when used either alone or as a suffix or prefix or otherwise includes straight-chain and branched-chain saturated structures comprising carbon and hydrogen atoms. References to individual alkyl groups such as propyl are specific for the straight-chain version only and references to individual branched-chain alkyl groups such as *tert*-butyl are specific for the branched chain version only. An analogous convention applies to other generic terms such as alkenyl and alkynyl.

Cycloalkyl is a monocyclic alkyl group, and cycloalkenyl and cycloalkynyl are monocyclic alkenyl and alkynyl groups respectively.

The prefix Cₘ₋ₙ in Cₘ₋ₙalkyl and other terms (where m and n are integers) indicates the range of carbon atoms that are present in the group, for example C₁₋₃alkyl includes C₁alkyl (methyl), C₂alkyl (ethyl) and C₃alkyl (propyl or isopropyl).

The term Cₘ₋ₙalkoxy comprises -O-Cₘ₋ₙalkyl groups.

The term halo includes fluoro, chloro, bromo and iodo.

Aryl groups are aromatic carbocyclic groups which may be monocyclic or bicyclic.

Unless otherwise stated heteroaryl groups are monocyclic or bicyclic aromatic rings containing 5 to 10 ring atoms of which 1, 2, 3 or 4 ring atoms are chosen from nitrogen, sulphur or oxygen where a ring nitrogen or sulphur may be oxidised.

Heterocyclyl is a saturated, unsaturated or partially saturated, monocyclic or bicyclic ring containing 4 to 7 ring atoms, of which 1, 2 or 3 ring atoms are selected from nitrogen, sulphur or oxygen, which ring may be carbon or nitrogen linked, wherein a -CH₂- group can optionally be replaced by a -C(O)- group; wherein a ring nitrogen or sulphur atom is optionally oxidised to form the N-oxide or S-oxide(s); and wherein a ring -NH- is optionally substituted by acetyl, formyl, methyl or mesyl. When the term heterocyclyl is used within the definition of groups of formula (I), this heterocyclyl ring is optionally substituted by 1 or 2 groups selected from C₁₋₄alkyl, C₁₋₄alkoxy, hydroxyC₁₋₄alkyl, hydroxy and haloC₁₋₄alkyl, and preferably the ring is so substituted. When the term heterocyclyl is used within the definition of groups of formula (IA), this heterocyclyl ring is optionally substituted by 1 or 2 groups selected from C₁₋₄alkyl, C₁₋₄alkoxy, hydroxyC₁₋₄alkyl, phosphonooxyC₁₋₄alkyl, hydroxy, phosphonooxy and haloC₁₋₄alkyl, and preferably the ring is so substituted. When the term heterocyclyl is used within the definition of R³ in formula (I), in one aspect of the invention it is a saturated monocyclic ring containing 4 to 7 ring atoms of which 1 or 2 are nitrogen and which ring is optionally substituted by C₁₋₄alkyl, hydroxyC₁₋₄alkyl and hydroxy. When the term heterocyclyl is used within the definition of R^{3'} in formula (IA), in one aspect of the invention it is a saturated monocyclic ring containing 4 to 7 ring atoms of which 1 or 2 are nitrogen and which ring is optionally substituted by C₁₋₄alkyl, hydroxyC₁₋₄alkyl, phosphonoooxyC₁₋₄alkyl, hydroxy and phosphonooxy.

Phosphonooxy is in one aspect a group of formula -OP(O)(OH)₂. However the term phosphonooxy also includes salts such as those formed with alkali metal ions such as sodium or potassium ions or alkaline earth metal ions, for example calcium or magnesium ions.

This specification also makes use of several composite terms to describe groups comprising more than one functionality. Such terms are to be interpreted as is understood in the art. For example Cₘ₋ₙcycloalkylCₘ₋ₙalkyl comprises Cₘ₋ₙalkyl substituted by Cₘ₋ₙ cycloalkyl and Cₘ₋ₙalkoxyCₘ₋ₙcycloalkylCₘ₋ₙalkyl comprises Cₘ₋ₙalkyl substituted by Cₘ₋ₙ cycloalkyl, which Cₘ₋ₙcycloalkyl is substituted by Cₘ₋ₙalkoxy.

HaloCₘ₋ₙalkyl is a Cₘ₋ₙalkyl group that is substituted by 1, 2 or 3 halo substituents. Similarly other generic terms containing halo such as haloCₘ₋ₙcycloalkyl and haloCₘ₋ₙ cycloalkcylCₘ₋ₙalkyl groups may contain 1, 2 or 3 halo substituents.

HydroxyCₘ₋ₙalkyl is a Cₘ₋ₙalkyl group that is substituted by 1, 2 or 3 hydroxy substituents. Similarly other generic terms containing hydroxy such as hydroxyCₘ₋ₙ cycloalkyl, hydroxyCₘ₋ₙalkylCₘ₋ₙcycloalkyl, hydroxyCₘ₋ₙcycloalkylCₘ₋ₙalkyl and hydroxyCₘ₋ₙ alkoxyCₘ₋ₙalkyl groups may contain 1, 2 or 3 hydroxy substituents.

Cₘ₋ₙalkoxyCₘ₋ₙalkyl is a Cₘ₋ₙalkyl group that is substituted by 1, 2 or 3 Cₘ₋ₙalkoxy substituents. Similarly other generic terms containing Cₘ₋ₙalkoxy such as Cₘ₋ₙalkoxyCₘ₋ₙ alkoxy, Cₘ₋ₙalkoxyCₘ₋ₙcycloalkyl and Cₘ₋ₙalkoxyCₘ₋ₙcycloalkylCₘ₋ₙalkyl groups may contain 1, 2 or 3 Cₘ₋ₙalkoxy substituents.

Where optional substituents are chosen from, for example 1 or 2 or from 1, 2, or 3 groups or substituents it is to be understood that this definition includes all substituents being chosen from one of the specified groups i.e. all substituents being the same or the substituents being chosen from two or more of the specified groups i.e. the substituents not being the same.

Unless specifically stated the bonding atom of a group may be any atom of that group so for example propyl includes prop-1-yl and prop-2-yl.

Compounds of the present invention have been named with the aid of computer software (ACD/Name version 6.6 or ACD Name Batch version 6.0).

Suitable values for any R group or any part or substituent for such groups include:
- for C₁₋₄alkyl:: methyl, ethyl, propyl, isopropyl, butyl, isobutyl and tert-butyl;
- for C₁₋₆alkyl:: C₁₋₄alkyl, pentyl, neopentyl, dimethylbutyl and hexyl;
- for C₂₋₄alkenyl:: vinyl, allyl and but-2-enyl;
- for C₂₋₆alkenyl:: C₂₋₄alkenyl, 3-methylbut-2-enyl and 3-methylpent-2-enyl;
- for C₂₋₄alkynyl:: ethynyl, propargyl and prop-1-ynyl;
- for C₂₋₆alkynyl:: C₂₋₄alkynyl, pent-4-ynyl and 2-methylpent-4-ynyl;
- for C₃₋₆cycloalkyl:: cyclopropyl, cyclobutyl, cyclopentyl and cyclohexyl;
- for C₃₋₆cycloalkenyl:: cyclobutenyl, cyclopentenyl, cyclohexenyl and cyclohex-1,4-dienyl;
- for C₃₋₆cycloalkylC₁₋₃alkyl:: cyclopropylmethyl, cyclobutylmethyl, cyclopentylmethyl, 2-cyclopropylethyl and 2-cyclobutylethyl;
- for C₁₋₄alkoxy:: methoxy, ethoxy, propoxy, butoxy and *tert*-butoxy;
- for C₁₋₃alkoxyC₁₋₄alkyl:: methoxymethyl, 2-methoxyethyl, 2-methoxypropyl and 2-ethoxyethyl;
- for C₁₋₃alkoxyC₁₋₆alkyl :: C₁₋₃alkoxyC₁₋₄alkyl, 4-methoxybutyl, 3-ethoxybutyl and 4,4-diethoxybutyl;
- for C₁₋₃alkcoxyC₃₋₆cycloalkyl:: 2-methoxycyclobutyl, 3-methoxycyclopentyl and 3-ethoxycyclopentyl;
- for C₁₋₃alkoxyC₃₋₆cycloalkylC₁₋₃alkyl:: 2-methoxycyclobutylmethyl and 2-methoxycyclopentylmethyl;
- for C₁₋₄alkoxyC₁₋₄alkoxy:: methoxymethoxy, 2-methoxyethoxy and 2-ethoxyethoxy;
- for hydroxyC₁₋₄alkyl:: hydroxymethyl, 2-hydroxyethyl, 2,2-dihydroxyethyl and 3-hydroxypropyl;
- for hydroxyC₁₋₆alkyl:: hydroxyC₁₋₄alkyl, 3-hydroxypentyl and 6-hydroxyhexyl;
- for hydroxyC₃₋₆Cycloalkyl:: 2-hydroxycyclopropyl, 2-hydroxycyclobutyl and 2-hydroxycyclopentyl;
- for hydroxyC₃₋₆cycloalkylC₁₋₃alkyl:: 2-hydroxycyclopropylmethyl and 2-hydroxycyclobutylmethyl;
- for hydroxyC₁₋₄alkylC₃₋₆cycloalkyl:: 1-(hydroxymethyl)cyclopentyl;
- for hydroxyC₁₋₄alkoxyC₁₋₄alkyl:: 2-(2-hydroxyethoxy)ethyl;
- for haloC₁₋₆alkyl:: chloromethyl, trifluoromethyl and 3,3,3-trifluoropropyl;
- for haloC₃₋₆cycloalkyl:: 2-chlorocyclopropyl and 2-chlorocyclobutyl;
- for haloC₃₋₆cycloalkylC₁₋₃alkyl:: 2-chlorocyclopropylmethyl and 2-chlorocyclobutylmethyl;
- for cyanoC₁₋₄alkyl:: cyanomethyl and 2-cyanoethyl;
- for aminoC₁₋₆alkyl:: aminomethyl, 2-aminoethyl, 2-aminopropyl and 4-aminobutyl;
- for C₁₋₃alkylaminoC₁₋₆alkyl:: 2-(methylamino)ethyl and 3-(ethylamino)propyl;
- for bis(C₁₋₃alkyl)aminoC₁₋₆alkyl:: 2-(dimethylamino)ethyl 2-[methyl(ethyl)amino]ethyl and 2-(diethylamino)ethyl;
- for C₁₋₄alkylamino:: methylamino, ethylamino, propylamino and isopropylamino;
- for bis(C₁₋₄alkyl)amino:: dimethylamine, methyl(ethyl)amino and diethylamino;
- C₁₋₆alkylaminoC₁₋₆alkyl:: 3-(methylamino)propyl, 4-[methylamino]butyl and 2-(ethylamino)ethyl;
- bis(C₁₋₆alkyl)aminoC₁₋₆alkyl:: 3-(dimethylamino)propyl, 4-[methyl(ethyl)amino]butyl and 2-(diethylamino)ethyl;
- for aryl:: phenyl and naphthyl
- for heteroaryl:: furyl, thienyl, pyrrolyl, pyrazolyl, pyridyl, pyrazinyl, pyridazinyl, pyrimidinyl, quinazolinyl and quinolinyl
- for heterocyclyl:: azetidinyl, pyrrolidinyl, imidazolidinyl, piperidinyl, piperazinyl, azepanyl, diazepanyl, pyridyl, imidazolyl, tetrahydrofuranyl, tetrahydropyranyl, furanyl, pyranyl, tetrahydrothienyl, thienyl, tetrahydro-2H-pyranyl and morpholinyl.
- for phosphonooxyC₁₋₄alkyl:: phosphonooxymethyl and 2-phosphonooxyethyl;
- for phosphonooxyC₁₋₆alkyl:: phosphonooxyC₁₋₄alkyl and 3-phosphonooxypentyl;
- for phosphonooxyC₃₋₆cycloalkyl:: 2-phosphonooxycyclopropyl and 2-phosphonooxycyclobutyl;
- for phosphonooxyC₃₋₆cycloalkylC₁₋₃alkyl:: 2-phosphonooxycyclopropylmethyl and 2-phosphonooxycyclobutylmethyl;
- for phosphonooxyC₁₋₄alkylC₃₋₆cycloalkyl:: 1-(phosphonooxymethyl)cyclopentyl;
- for phosphonooxyC₁₋₄alkoxyC₁₋₄alkyl:: 2-(2-phosphonooxyethoxy)ethyl.

Within the present invention, it is to be understood that, insofar as certain of compounds of formula (I) or formula (IA) herein defined may exist in optically active or racemic forms by virtue of one or more asymmetric carbon or sulphur atoms, the invention includes in its definition any such optically active or racemic form which possesses aurora kinase inhibitory activity and in particular Aurora-A and/or Aurora-B kinase inhibitory activity. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to herein.

Within the present invention it is to be understood that a compound of formula (I) or formula (IA) may exhibit the phenomenon of tautomerism and that the formulae drawings within this specification can represent only one of the possible tautomeric forms. It is to be understood that the invention encompasses any tautomeric form which has Aurora kinase inhibitory activity and in particular Aurora-A and/or Aurora-B kinase inhibitory activity and is not to be limited merely to any one tautomeric form utilised within the formulae drawings.

It is also to be understood that certain compounds of formula (I) or formula (IA) can exist in solvated as well as unsolvated forms such as, for example, hydrated forms. It is to be understood that the invention encompasses all such solvated forms which have Aurora kinase inhibitory activity and in particular Aurora-A and/or Aurora-B kinase inhibitory activity.

The present invention relates to the compounds of formula (I) or formula (IA) as herein defined as well as to the salts thereof. Salts for use in pharmaceutical compositions will be pharmaceutically acceptable salts, but other salts may be useful in the production of the compounds of formula (I) or formula (IA) and their pharmaceutically acceptable salts. Pharmaceutically acceptable salts of the invention may, for example, include acid addition salts of compounds of formula (I) or formula (IA) as herein defined which are sufficiently basic to form such salts. Such acid addition salts include but are not limited to furmarate, methanesulphonate, hydrochloride, hydrobromide, citrate and maleate salts and salts formed with phosphoric and sulphuric acid. In addition where compounds of formula (I) or formula (IA) are sufficiently acidic, salts are base salts and examples include but are not limited to, an alkali metal salt for example sodium or potassium, an alkaline earth metal salt for example calcium or magnesium, or organic amine salt for example triethylamine, ethanolamine, diethanolamine, triethanolamine, morpholine, *N*-methylpiperidine, *N*-ethylpiperidine, dibenzylamine or amino acids such as lysine.

The compounds of formula (I) or formula (IA) may also be provided as *in vivo* hydrolysable esters. An *in vivo* hydrolysable ester of a compound of formula (I) or formula (IA) containing carboxy or hydroxy group is, for example a pharmaceutically acceptable ester which is cleaved in the human or animal body to produce the parent acid or alcohol. Such esters can be identified by administering, for example, intravenously to a test animal, the compound under test and subsequently examining the test animal's body fluid.

Suitable pharmaceutically acceptable esters for carboxy include C₁₋₆alkoxymethyl esters for example methoxymethyl, C₁₋₆alkanoyloxymethyl esters for example pivaloyloxymethyl, phthalidyl esters, C₃₋₈cycloalkoxycarbonyloxyC₁₋₆alkyl esters for example 1-cyclohexylcarbonyloxyethyl; 1,3-dioxolen-2-onylmethyl esters for example 5-methyl-1,3-dioxolen-2-onylmethyl; and C₁₋₆alkoxycarbonyloxyethyl esters for example 1-methoxycarbonyloxyethyl and may be formed at any carboxy group in the compounds of this invention.

Suitable pharmaceutically acceptable esters for hydroxy include inorganic esters such as phosphate esters (including phosphoramidic cyclic esters) and α-acyloxyalkyl ethers and related compounds which as a result of the *in vivo* hydrolysis of the ester breakdown to give the parent hydroxy group/s. Examples of α-acyloxyalkyl ethers include acetoxymethoxy and 2,2-dimethylpropionyloxymethoxy. A selection of *in vivo* hydrolysable ester forming groups for hydroxy include C₁₋₁₀alkanoyl, for example formyl, acetyl; benzoyl; phenylacetyl; substituted benzoyl and phenylacetyl, C₁₋₁₀alkoxycarbonyl (to give alkyl carbonate esters), for example ethoxycarbonyl; di-C₁₋₄alkylcarbamoyl and *N*-(di-C₁₋₄alkylaminoethyl)-*N-*C₁₋₄alkylcarbamoyl (to give carbamates); di-C₁₋₄alkylaminoacetyl and carboxyacetyl. Examples of ring substituents on phenylacetyl and benzoyl include aminomethyl, C₁₋₄ alkylaminomethyl and di-(C₁₋₄alkyl)aminomethyl, and morpholino or piperazino linked from a ring nitrogen atom via a methylene linking group to the 3- or 4- position of the benzoyl ring. Other interesting *in vivo* hydrolysable esters include, for example, R^{A}C(O)OC₁₋₆alkyl-CO-, wherein R^{A} is for example, benzyloxy-C₁₋₄alkyl, or phenyl). Suitable substituents on a phenyl group in such esters include, for example, 4-C₁₋₄piperazino-C₁₋₄alkyl, piperazino-C₁₋₄alkyl and morpholino-C₁₋₄alkyl.

The compounds of the formula (I) may be also be administered in the form of a prodrug which is broken down in the human or animal body to give a compound of the formula (I). Examples of prodrugs include in vivo hydrolysable esters of a compound of the formula (I). Various forms of prodrugs are known in the art. For examples of such prodrug derivatives, see:
a) Design of Prodrugs, edited by H. Bundgaard, (Elsevier, 1985) and Methods in Enzymology, Vol. 42, p. 309-396, edited by K. Widder, et al. (Academic Press, 1985);
b) A Textbook of Drug Design and Development, edited by Krogsgaard-Larsen and H. Bundgaard, Chapter 5 "Design and Application of Prodrugs", by H. Bundgaard p. 113-191 (1991);
c) H. Bundgaard, Advanced Drug Delivery Reviews, 8, 1-38 (1992);
d) H. Bundgaard, et al., Journal of Pharmaceutical Sciences, 77, 285 (1988); and
e) N. Kakeya, et al., Chem Pharm Bull, 32, 692 (1984).

Particular values of X, R¹, R^{1'}, R², R^{2'}, R³, R^{3'}, R⁴ and R⁵ for compounds of formula (I) and formula (IA) are as follows. Such values may be used where appropriate with any of the definitions, claims or embodiments defined herein.

In one aspect of the invention X is NR⁶. In another aspect X is NH.

In one aspect of the invention R⁶ is hydrogen or methyl. In another aspect R⁶ is hydrogen.

In one aspect of the invention R¹ is hydrogen or -OR¹¹. In another aspect R¹ is hydrogen.

In one aspect of the invention X¹ is a direct bond or -O-. In another aspect X¹ is a direct bond.

In one aspect of the invention R¹¹ is hydrogen, heterocyclyl selected from piperidinyl and pyrrolidinyl or C₁₋₄alkyl (optionally substituted by hydroxy, C₁₋₄alkoxy, amino, C₁₋₄ alkylamino or bis(C₁₋₄alkyl)amino). In another aspect R¹¹ is hydrogen, C₁₋₄alkyl or C₁₋₄ alkoxy. In another aspect R¹¹ is hydrogen.

In one aspect of the invention R² is hydrogen or -OR¹². In another aspect R² is hydrogen or methoxy. In a further aspect R² is hydrogen. In yet a further aspect R² is methoxy.

In one aspect of the invention X² is a direct bond or -O-.

In one aspect of the invention R¹² is hydrogen, C₁₋₄alkyl (optionally substituted by heterocyclyl) or heterocyclyl. In another aspect R¹² is hydrogen or C₁₋₄alkyl. In another aspect of the invention R¹² is hydrogen. In a further aspect of the invention R¹² is methyl.

In one aspect of the invention R³ is-X³R¹³. In a further aspect R³ is 3-(2-hydroxymethylpyrrolidin-1-yl)propoxy or 3-[(2-hydroxyethyl)(propyl)amino]propoxy.

In one aspect of the invention X³ is -CH₂=CH₂-, -O- or -NH-. In another aspect X³ is - O-.

In one aspect of the invention R¹³ is C₁₋₆alkyl substituted by -NR⁷R⁸, heterocyclyl or halo. In a further aspect of the invention R¹³ is ethyl or propyl, both of which are substituted by -Nk⁷R⁸, heterocyclyl or halo. In yet a further aspect of the invention R¹³ is propyl substituted by chloro, -NR⁷R⁸ or a heterocyclyl selected from pyrrolidinyl, piperidinyl, piperazinyl, diazepanyl and azetidinyl which heterocyclyl is optionally substituted by hydroxy, methyl, hydroxymethyl or 2-hydroxyethyl. In another aspect R¹³ is propyl substituted by chloro or -NR⁷R⁸. In a further aspect R¹³ is propyl substituted by -NR⁷R⁸.

In one aspect of the invention R⁷ and R⁸ are independently selected from hydrogen, heterocyclyl, C₁₋₆alkyl, hydroxyC₁₋₆alkyl, hydroxyC₁₋₄alkylC₃₋₆cycloalkyl, C₁₋₃alkoxyC₁₋₄alkyl, C₃₋₆cycloalkyl, C₃₋₆cycloalkylC₁₋₃alkyl, haloC₁₋₆alkyl, C₂₋₆alkenyl, C₂₋₆alkynyl, cyanoC₁₋₄alkyl and bis(C₁₋₃alkyl)aminoC₁₋₆alkyl; or R⁷ and R⁸ together with the nitrogen to which they are attached form a heterocyclic ring which ring comprises 4 to 7 ring atoms of which one is nitrogen and of which another is optionally NH and which ring is optionally substituted on carbon or nitrogen by a group selected from C₁₋₄alkyl, hydroxy, hydroxyC₁₋₄alkyl and hydroxyC₁₋₄alkoxyC₁₋₄alkyl, and where a ring -CH₂- is optionally replaced with -C(O)-. In a further aspect R⁷ and R⁸ are independently selected from hydrogen, methyl, ethyl, propyl, isopropyl, butyl, isobutyl, tert-butyl, pentyl, neopentyl, hydroxymethyl, 2-hydroxyethyl, 3-hydroxy-1,1-dimethylpropyl, methoxymethyl, 2-methoxyethyl, 2-ethoxyethyl, cyclopropyl, cyclobutyl, cyclopentyl, cyclopropylmethyl, cyclobutylmethyl, cyclopentylmethyl, trifluoromethyl, 2,2,2-trifluoroethyl, 3,3,3-trifluoropropyl, allyl, propargyl, 2-(dimethylamino)ethyl and 2-(diethylamino)ethyl; or R⁷ and R⁸ together with the nitrogen to which they are attached form a heterocyclic ring selected from pyrrolidinyl, piperidinyl, piperazinyl, diazepanyl and azetidinyl where the ring is optionally substituted by hydroxy, methyl, hydroxymethyl or 2-hydroxyethyl. In yet another aspect R⁷ and R⁸ are independently selected from hydrogen, methyl, ethyl, propyl, hydroxymethyl, 2-hydroxyethyl or 3-hydroxypropyl ; or R⁷ and R⁸ together with the nitrogen to which they are attached form a heterocyclic ring selected from pyrrolidinyl, piperidinyl and piperazinyl, where the ring is optionally substituted by hydroxymethyl or 2-hydroxyethyl.

In one aspect of the invention R⁴ is hydrogen.

In one aspect of the invention R⁵ is aryl optionally substituted by 1 or 2 halo. In another aspect R⁵ is phenyl optionally substituted by 1 or 2 fluoro or chloro. In a further aspect R⁵ is phenyl optionally substituted by 1 or 2 fluoro. In yet another aspect R⁵ is 2,3-difluorophenyl or 3-fluorophenyl.

In one aspect of the invention R^{1'} is hydrogen or -OR^{11'}. In another aspect R^{1'} is hydrogen.

In one aspect of the invention R^{11'} is hydrogen, heterocyclyl selected from piperidinyl or pyrrolidinyl or C₁₋₄alkyl (optionally substituted by hydroxy, phosphonooxy, C₁₋₄alkoxy, amino, C₁₋₄alkylamino or bis(C₁₋₄alkyl)amino). In another aspect R^{11'} is hydrogen, C₁₋₄alkyl or C₁₋₄alkoxy. In another aspect R^{11'} is hydrogen.

In one aspect of the invention R^{2'} is hydrogen or-OR^{12'}. In another aspect R^{2'} is hydrogen or methoxy. In a further aspect R^{2'} is hydrogen. In yet a further aspect R^{2'} is methoxy.

In one aspect of the invention R^{12'} is hydrogen, C₁₋₄alkyl (optionally substituted by heterocyclyl) or heterocyclyl. In another aspect R^{12'} is hydrogen or C₁₋₄alkyl. In another aspect of the invention R^{12'} is hydrogen. In a further aspect of the invention R¹²' is methyl.

In one aspect of the invention R^{3'} is -X³R^{13'}.

In one aspect of the invention R^{13'} is C₁₋₆alkyl substituted by -NR^{7'}R^{8'}. In a further aspect of the invention R^{13'} is propyl substituted by -NR^{7'}R^{8'},

In one aspect of the invention R^{7'} is selected from hydrogen, heterocyclyl, C₁₋₆alkyl, C₁₋₃alkoxyC₁₋₆alkyl, cyanoC₁₋₄alkyl and C₃₋₆cycloalkyl. In another aspect R^{7'} is ethyl, propyl, cyclobutyl or 2-methoxyethyl,

In one aspect of the invention R^{8'} is phosphonooxyC₁₋₄alkyl or phosphonooxyC₁₋₄ alkylC₃₋₆cycloalkyl, In another aspect R^{8'} is 2-phosphonooxyethyl or 1,1-dimethyl-3-phosphonooxypropyl.

In one aspect of the invention R^{7'} and R^{8'} together with the nitrogen to which they are attached form a heterocyclic ring selected from pyrrolidinyl, piperidinyl and piperazinyl which ring is substituted on carbon or nitrogen by a group selected from phosphonooxy, phosponooxymethyl and 2-phoshonooxyethyl. In a further aspect R^{7'} and R^{8'} together with the nitrogen to which they are attached form a pyrrolidinyl ring which ring is substituted on carbon by phosponooxymethyl.

A particular class of compounds is of formula (I) wherein:
X is NR⁶;
R⁶ is hydrogen or methyl;
R¹ is hydrogen or -OR¹¹;
X¹ is a direct bond or -O-;
R¹¹ is hydrogen, heterocyclyl selected from piperidinyl and pyrrolidinyl, or C₁₋₄alkyl optionally substituted by hydroxy, C₁₋₄alkoxy, amino, C₁₋₄alkylamino or bis(C₁₋₄alkyl)amino; R² is hydrogen or -OR¹²;
R¹² is hydrogen, C₁₋₄alkyl (optionally substituted by heterocyclyl) or heterocyclyl;
R³ is -X³R¹³;
X³ is -CH₂=CH₂-, -O- or -NH-;
R¹³ is C₁₋₆alkyl substituted by -NR⁷R⁸, heterocyclyl or halo;
R⁷ and R⁸ are independently selected from hydrogen, heterocyclyl, C₁₋₆alkyl, hydroxyC₁₋₆ alkyl, hydroxyC₁₋₄alkylC₃₋₆cycloalkyl, C₁₋₃alkoxyC₁₋₄alkyl, C₃₋₆cycloalkyl, C₃₋₆cycloalkylC₁₋₃ alkyl, haloC₁₋₆alkyl, C₂₋₆alkenyl, C₂₋₆alkynyl, cyanoC₁₋₄alkyl and bis(C₁₋₃alkyl)aminoC₁₋₆ alkyl; or R⁷ and R⁸ together with the nitrogen to which they are attached form a heterocyclic ring which ring comprises 4 to 7 ring atoms of which one is nitrogen and of which another is optionally NH and which ring is optionally substituted on carbon or nitrogen by a group selected from C₁₋₄alkyl, hydroxy, hydroxyC₁₋₄alkyl and hydroxyC₁₋₄alkoxyC₁₋₄alkyl, and where a ring -CH₂- is optionally replaced with -C(O)-;
R⁴ is hydrogen; and
R⁵ is aryl optionally substituted by 1 or 2 halo;
or is a salt, ester or prodrug thereof

A further particular class of compounds is of formula (I) wherein:
X is NH;
R¹ is hydrogen;
R² is hydrogen or methoxy;
R³ is -X³R¹³;
X³ is -O-;
R¹³ is propyl substituted by chloro or -NR⁷R⁸;
R⁷ and R⁸ are independently selected from hydrogen, methyl, ethyl, propyl, isopropyl, butyl, isobutyl, tert-butyl, pentyl, neopentyl, hydroxymethyl, 2-hydroxyethyl, 3-hydroxy-1,1-dimethylpropyl, methoxymethyl, 2-methoxyethyl, 2-ethoxyethyl, cyclopropyl, cyclobutyl, cyclopentyl, cyclopropylmethyl, cyclobutylmethyl, cyclopentylmethyl, trifluoromethyl, 2,2,2-trifluoroethyl, 3,3,3-trifluoropropyl, allyl, propargyl, 2-(dimethylamino)ethyl and 2-(diethylamino)ethyl; or R⁷ and R⁸ together with the nitrogen to which they are attached form a heterocyclic ring selected from pyrrolidinyl, piperidinyl, piperazinyl, diazepanyl and azetidinyl where the ring is optionally substituted by hydroxy, methyl, hydroxymethyl or 2-hydroxyethyl;
R⁴ is hydrogen; and
R⁵ is 2,3-difluorophenyl or 3-fluorophenyl;
or is a salt, ester or prodrug thereof.

A particular class of compounds is of formula (IA) wherein:
X is NR⁶;
R⁶ is hydrogen or methyl;
R^{1'} is hydrogen or -OR^{11'};
R^{11'} is hydrogen, heterocyclyl selected from piperidinyl or pyrrolidinyl, C₁₋₄alkyl optionally substituted by hydroxy, phosphonooxy, C₁₋₄alkoxy, amino, C₁₋₄alkylamino or bis(C₁₋₄ alkyl)amino;
R^{2'} is hydrogen or -OR^{12'};
R^{12'} is hydrogen, C₁₋₄alkyl (optionally substituted with heterocyclyl) or heterocyclyl;
R³ is -X³R^{13'};
X³ is -CH₂=CH₂-, -O- or -NH-;
R^{13'} is C₁₋₆alkyl substituted by -NR^{7'} R^{8'};
R^{7'} is selected from hydrogen, heterocyclyl, C₁₋₆alkyl, C₁₋₃alkoxyC₁₋₆alkyl, cyanoC₁₋₄alkyl and C₃₋₆cycloalkyl;
R^{8'} is phosphonooxyC₁₋₄alkyl or phosphonooxyC₁₋₄alkylC₃₋₆cycloalkyl;
or R^{7'} and R^{8'} together with the nitrogen to which they are attached form a heterocyclic ring selected from pyrrolidinyl, piperidinyl and piperazinyl which ring is substituted on carbon or nitrogen by a group selected from phosphonooxy, phosponooxymethyl and 2-phoshonooxyethyl;
R⁴ is hydrogen; and
R⁵ is aryl optionally substituted by 1 or 2 halo;
or is a salt or ester thereof.

A further particular class of compounds is of formula (IA) wherein:
X is NH;
R^{1'} is hydrogen;
R^{2'} is hydrogen or methoxy;
R^{3'} is -X³R^{13'};
X³ is -O-;
R^{13'} is propyl substituted by -NR^{7'}R^{8'};
R^{7'} is ethyl, propyl, cyclobutyl or 2-methoxyethyl;
R^{8'} is 2-phosphonooxyethyl or 1,1-dimethyl-3-phosphonooxypropyl;
or R^{7'} and R^{8'} together with the nitrogen to which they are attached form a heterocyclic ring selected from pyrrolidinyl, piperidinyl and piperazinyl which ring is substituted on carbon or nitrogen by a group selected from phosphonooxy, phosponooxymethyl and 2-phoshonooxyethyl;
R⁴ is hydrogen; and
R⁵ is 2,3-difluorophenyl or 3-fluorophenyl;
or is a salt or ester thereof.

Particular compounds of the invention are any one of:
*N*-(3-fluorophenyl)-2-{3-[(7-{3-[(2*R*)-2-(hydroxymethyl)pyrrolidin-1-yl]propoxy}quinazolin-4-yl)amino]-1*H*-pyrazol-1-yl}acetamide;
*N*-(3-fluorophenyl)-2-{3-[(7-{3-[(2-hydroxyethyl)(propyl)amino]propoxy}quinazolin-4-yl)amino]-1*H*-pyrazol-1-yl}acetamide;
*N*-(2,3-difluorophenyl)-2-{3-[(7-{3-[(2*R*)-2-(hydroxymethyl)pyrrolidin-1-yl]propoxy}quinazolin-4-yl)amino]-1*H*-pyrazol-1-yl}acetamide;
*N*-(2,3-difluorophenyl)-2-{3-[(7-{3-[(2-hydroxyethyl)(propyl)amino]propoxy}quinazolin-4-yl)amino]-1*H*-pyrazol-1-yl}acetamide;
*N*-(3-fluorophenyl)-2-{3-[(7-{3-[(2*R*)-2-(hydroxymethyl)pyrrolidin-1-yl]propoxy}-6-methoxyquinazolin-4-yl)amino]-1*H*-pyrazol-1-yl}acetamide;
*N*-(3-fluorophenyl)-2-{3-[(7-{3-[(2-hydroxyethyl)(propyl)amino]propoxy}-6-methoxyquinazolin-4-yl)amino]-1*H*-pyrazol-1-yl}acetamide;
*N*-(2,3-difluorophenyl)-2-{3-[(7-{3-[(2*R*)-2-(hydroxymethyl)pyrrolidin-1-yl]propoxy}-6-methoxyquinazolin-4-yl)amino]-1*H*-pyrazol-1-yl}acetamide; and
*N*-(2,3-difluorophenyl)-2-{3-[(7-{3-[(2-hydroxyethyl)(propyl)amino]propoxy}-6-methoxyquinazolin-4-yl)amino]-1*H*-pyrazol-1-yl}acetamide;
or a salt, ester or prodrug thereof and more particularly a pharmaceutically acceptable salt thereof.

Other particular compounds of the invention are any one of:
{(2*R*)-1-[3-({4-[(1-{2-[(3-fluorophenyl)amino]-2-oxoethyl}-1*H*-pyrazol-4-yl)amino]quinazolin-7-yl}oxy)propyl]pyrrolidin-2-yl}methyl dihydrogen phosphate;
{(2*R*)-1-[3-({4-[(1-(2-[(2,3-difluorophenyl)amino]-2-oxoethyl}-1*H*-pyrazol-3-yl)amino]quinazolin-7-yl}oxy)propyl]pyrrolidin-2-yl}methyl dihydrogen phosphate;
{(2*R*)-1-[3-({4-[(1-{2-[(3-fluorophenyl)amino]-2-oxoethyl}-1*H*-pyrazol-3-yl)amino]-6-methoxyquinazolin-7-yl}oxy)propyl]pyrrolidin-2-yl}methyl dihydrogen phosphate; and
{(2*R*)-1-[3-({4-[(1-{2-[(2,3-difluorophenyl)amino]-2-oxoethyl}-1*H*-pyrazol-3-yl)amino]-6-methoxyquinazolin-7-yl}oxy)propyl]pyrrolidin-2-yl}methyl dihydrogen phosphate;
or a salt or ester thereof and more particularly a pharmaceutically acceptable salt thereof.

The present invention also provides a process for the preparation of a compound of formula (I) or a salt or ester thereof, which process comprises reacting a compound of formula (II) 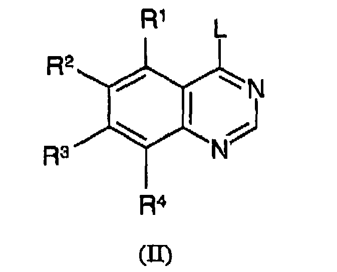 where L is a suitable leaving group such as chloro, bromo, SMe etc.
with a compound of formula (III) 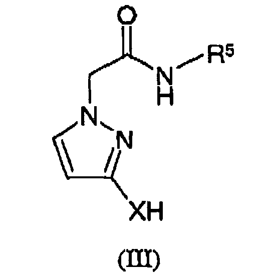 in the presence of hydrochloric acid in dioxane,
and thereafter if necessary:
i) converting a compound of the formula (I) into another compound of the formula (I); and/or
ii) removing any protecting groups; and/or
iii) forming a salt or ester thereof.
The reaction is suitably effected in an organic solvent such as dimethyl acetamide or isopropanol at elevated temperatures of from 50°C to 120°C for 30 minutes to 2 hours.

The process may further comprise a process for the preparation of a compound of formula (II) from a compound of formula (IV) 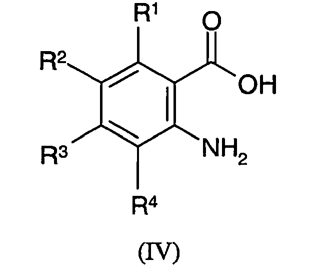 by reacting it with a reagent such as formamidine acetate in a solvent such as 2-methoxyethanol under reflux to yield a compound of formula (V) 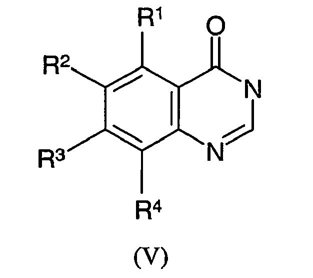 which may then be reacted with a reagent such as thionyl chloride in a solvent such as dimethyl formamide at elevated temperatures to yield a compound of formula (II) where L is, for example chloro.
Compounds of formula (IV) are either known in the art or can be derived from other compounds known in the art by conventional methods which would be apparent from the literature.

The process may further comprise a process for the preparation of a compound of formula (III) which process comprises the reaction of a compound of formula (VI) 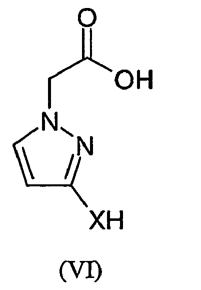 with a compound of formula (VII)

R⁵-NH₂ (VII)

The reaction is suitably effected in an organic solvent such as tetrahydrofuran (THF) or dichloromethane (DCM) at temperatures from 0°C to 25°C in the presence of a base such as sodium hydroxide or pyridine under an inert atmosphere for 1 to 5 hours.

Further provided is a process for the preparation of a compound of formula (IA) or a salt or ester thereof, which process comprises phosphorylation of a suitable compound of formula (I) by reacting a compound of formula (I) and tetrazole with di-tert-butyl diethylphosphoramidite in an appropriate organic solvent such as dimethylformamide or dimethylacetamide under an inert atmosphere, followed by (after 1 to 5 hours) the addition of hydrogen peroxide and sodium metabisulphite. Deprotection of the phosphate group then yields a compound of formula (IA). Deprotection is suitably effected with hydrochloric acid in dioxane or dichloromethane (DCM) at ambient temperature for 6 to 30 hours.

Suitable reaction conditions are illustrated herein.

It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogen group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.

It will also be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or *tert*-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *tert*-butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a *tert*-butyl group which may be removed, for example, by treatment with an acid, for example an organic acid such as trifluoroacetic acid, or for example a benzyl group which may be removed, for example, by hydrogenation over a catalyst such as palladium-on-carbon.

The protecting groups may be removed at any convenient stage in the synthesis using conventional techniques well known in the chemical art.

According to a further aspect of the invention there is provided a pharmaceutical composition which comprises a compound formula (I), or a pharmaceutically acceptable salt or ester thereof, as defined herein in association with a pharmaceutically acceptable diluent or carrier.

Also provided is a pharmaceutical composition which comprises a compound of formula (IA), or a pharmaceutically acceptable salt or ester thereof, as defined herein in association with a pharmaceutically acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

Suitable pharmaceutically acceptable excipients for a tablet formulation include, for example, inert diluents such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal track, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, soya bean oil, coconut oil, or preferably olive oil, or any other acceptable vehicle.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxyethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as ethyl or propyl p-hydroxybenzoate, anti-oxidants (such as ascorbic acid), colouring agents, flavouring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavouring agents may be added to provide a palatable oral preparation. These compositions may be preserved by the addition of an anti-oxidant such as ascorbic acid.

Dispersible or lyophilised powders and granules suitable for preparation of an aqueous suspension or solution by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavouring and colouring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, solutions, emulsions or particular systems, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in polyethylene glycol.

Suppository formulations may be prepared by mixing the active ingredient with a suitable non-irritating excipient which is solid at ordinary temperatures but liquid at the rectal temperature and will therefore melt in the rectum to release the drug. Suitable excipients include, for example, cocoa butter and polyethylene glycols.

Topical formulations, such as creams, ointments, gels and aqueous or oily solutions or suspensions, may generally be obtained by formulating an active ingredient with a conventional, topically acceptable, vehicle or diluent using conventional procedure well known in the art.

Compositions for administration by insufflation may be in the form of a finely divided powder containing particles of average diameter of, for example, 30µm or much less preferably 5µm or less and more preferably between 5µm and 1µm, the powder itself comprising either active ingredient alone or diluted with one or more physiologically acceptable carriers such as lactose. The powder for insufflation is then conveniently retained in a capsule containing, for example, 1 to 50mg of active ingredient for use with a turbo-inhaler device, such as is used for insufflation of the known agent sodium cromoglycate.

Compositions for administration by inhalation may be in the form of a conventional pressurised aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

For further information on formulation the reader is referred to Chapter 25.2 in Volume 5 of Comprehensive Medicinal Chemistry (Corwin Hansch; Chairman of Editorial Board), Pergamon Press 1990.

Therefore in a further aspect of the invention there is provided a compound of formula (I), or a pharmaceutically acceptable salt or ester thereof, for use in therapy. In addition a compound of formula (IA), or a pharmaceutically acceptable salt or ester thereof, is provided for use in therapy.

Further provided is a compound of formula (I), or a pharmaceutically acceptable salt or ester thereof, for use as a medicament and also provided is a compound of formula (IA), or a pharmaceutically acceptable salt or ester thereof, for use as a medicament. Another aspect of the invention provides a compound of formula (I), or a pharmaceutically acceptable salt or ester thereof, for use as a medicament for the treatment of a hyperproliferative disease such as cancer and in particular colorectal, breast, lung, prostate, bladder, renal or pancreatic cancer or leukaemia or lymphoma. Also provided is a compound of formula (IA), or a pharmaceutically acceptable salt or ester thereof, for use as a medicament for the treatment of a hyperproliferative disease such as cancer and in particular colorectal, breast, lung, prostate, bladder, renal or pancreatic cancer or leukaemia or lymphoma.

Additionally a compound of formula (I), or a pharmaceutically acceptable salt or ester thereof is provided for use in a method of treatment of a warm-blooded animal such as man by therapy. A compound of formula (IA), or a pharmaceutically acceptable salt or ester thereof, is also provided for use in a method of treatment of a warm-blooded animal such as man by therapy. Another aspect of the invention provides a compound of formula (I), or a pharmaceutically acceptable salt or ester thereof, for use in a method of treatment of a hyperproliferative disease such as cancer, and in particular colorectal, breast, lung, prostate, bladder, renal or pancreatic cancer or leukaemia or lymphoma. Also provided is a compound of formula (IA), or a pharmaceutically acceptable salt or ester thereof, for use in a method of treatment of a hyperproliferative disease such as cancer, and in particular colorectal, breast, lung, prostate, bladder, renal or pancreatic cancer or leukaemia or lymphoma.

In another aspect of the invention, there is provided the use of a compound of formula (I), or a pharmaceutically acceptable salt or ester thereof, in the preparation of a medicament for the treatment of a disease where the inhibition of one or more Aurora kinase(s) is beneficial. The use of a compound of formula (IA), or a pharmaceutically acceptable salt or ester thereof, in the preparation of a medicament for the treatment of a disease where the inhibition of one or more Aurora kinase(s) is beneficial is also provided. In particular it is envisaged that inhibition of Aurora-A kinase and/or Aurora-B kinase may be beneficial. Preferably inhibition of Aurora-B kinase is beneficial.

In another aspect of the invention, there is provided the use of a compound of formula (I) or a pharmaceutically acceptable salt or ester thereof, in the preparation of a medicament for the treatment of a hyperproliferative disease such as cancer, and in particular colorectal, breast, lung, prostate, bladder, renal or pancreatic cancer or leukaemia or lymphoma. Also provided is the use of a compound of formula (IA) or a pharmaceutically acceptable salt or ester thereof in the preparation of a medicament for the treatment of a hyperproliferative disease such as cancer, and in particular colorectal, breast, lung, prostate, bladder, renal or pancreatic cancer or leukaemia or lymphoma.

According to yet another aspect, there is provided a compound of formula (I), or a pharmaceutically acceptable salt or ester thereof for use in the method of treating a human suffering from a disease in which the inhibition of one or more Aurora kinases is beneficial, comprising the steps of administering to a person in need thereof a therapeutically effective amount of a compound of formula (I) or a pharmaceutically acceptable salt or ester thereof, Further provided is a compound of formula (IA) or a pharmaceutically acceptable salt thereof for use in the method of treating a human suffering from a disease in which the inhibition of one or more Aurora kinases is beneficial, comprising the steps of administering to a person in need thereof a therapeutically effective amount of a compound of formula (IA) or a pharmaceutically acceptable salt thereof. In particular it is envisaged that inhibition of Aurora-A kinase and/or Aurora-B kinase may be beneficial. Preferably inhibition of Aurora-B kinase is beneficial. Further provided is a compound of formula (I) or a pharmaceutically acceptable salt or ester thereof for use in the method of treating a human suffering from a hyperproliferative disease such as cancer, and in particular colorectal, breast, lung, prostate, bladder, renal or pancreatic cancer or leukaemia or lymphoma, comprising the steps of administering to a person in need thereof a therapeutically effective amount of a compound of formula (I) or a pharmaceutically acceptable salt, ester or prodrug thereof. A compound of formula (IA) is also provided for use in the method of treating a human suffering from a hyperproliferative disease such as cancer and in particular colorectal, breast, lung, prostate, bladder, renal or pancreatic cancer or leukaemia or lymphoma, comprising the steps of administering to a person in need thereof a therapeutically effective amount of a compound of formula (IA) or a pharmaceutically acceptable salt or ester thereof. The use of a compound of formula (I) or a pharmaceutically acceptable salt or ester thereof in any of the methods of treating a human described above also form aspects of this invention.

For the above mentioned therapeutic uses the dose administered will vary with the compound employed, the mode of administration, the treatment desired, the disorder indicated and the age and sex of the animal or patient. The size of the dose would thus be calculated according to well known principles of medicine.

In using a compound of formula (I) or formula (IA) for therapeutic or prophylactic purposes it will generally be administered so that a daily dose in the range, for example, 0.05 mg/kg to 50 mg/kg body weight is received, given if required in divided doses. In general lower doses will be administered when a parenteral route is employed. Thus, for example, for intravenous administration, a dose in the range, for example, 0.05 mg/kg to 25 mg/kg body weight will generally be used. Similarly, for administration by inhalation, a dose in the range, for example, 0.05 mg/kg to 25 mg/kg body weight will be used.

The treatment defined herein may be applied as a sole therapy or may involve, in addition to the compound of the invention, conventional surgery or radiotherapy or chemotherapy. Such chemotherapy may include one or more of the following categories of anti-tumour agents :-
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea; antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, arnsacrine, topotecan and camptothecin);
(ii) cytostatic agents such as antioestrogens (for example tamoxifen, toremifene, raloxifene, droloxifene and iodoxyfene), oestrogen receptor down regulators (for example fulvestratrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) Agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin™] and the anti-erbb1 antibody cetuximab [C225]), farnesyl transferase inhibitors, tyrosine kinase inhibitors and serine-threonine kinase inhibitors, for example inhibitors of the epidermal growth factor family (for example EGFR family tyrosine kinase inhibitors such as *N*-(3-chloro-4-fluorophenyl)-7-methoxy-6-(3-morpholinopropoxy)quinazolin-4-amine (gefitinib, AZD1839), *N*-(3-ethynylphenyl)-6,7-bis(2-methoxyethoxy)quinazolin-4-amine (erlotinib, OSI-774) and 6-acrylamido-*N*-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin (αvβ3 function and angiostatin);
(vi) vascular damaging agents such as Combretastatin A4 and compounds disclosed in International Patent Applications WO 99/02166, WO00/40529, WO 00/41669, WO01/92224, WO02/04434 and WO02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example *ex vivo* and *in vivo* approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

In addition a compound of the invention or a pharmaceutically acceptable salt, ester or prodrug thereof, may be used in combination with one or more cell cycle inhibitors. In particular with cell cycle inhibitors which inhibit bub1, bubR1 or CDK.

Such conjoint treatment may be achieved by way of the simultaneous, sequential or separate dosing of the individual components of the treatment. Such combination products employ the compounds of this invention within the dosage range described herein and the other pharmaceutically-active agent within its approved dosage range.

In addition to their use in therapeutic medicine, a compound of formula (I) and a pharmaceutically acceptable salt, ester or prodrug thereof are also useful as pharmacological tools in the development and standardisation of *in vitro* and *in vivo* test systems for the evaluation of the effects of inhibitors of cell cycle activity in laboratory animals such as cats, dogs, rabbits, monkeys, rats and mice, as part of the search for new therapeutic agents.

In the above other pharmaceutical composition, process, method, use and medicament manufacture features, the alternative and preferred embodiments of the compounds of the invention described herein also apply.

The compounds of the invention inhibit the serine-threonine kinase activity of the Aurora kinases, in particular Aurora-A kinase and/or Aurora-B kinase and thus inhibit the cell cycle and cell proliferation. These properties may be assessed for example, using one or more of the procedures set out below.

### (a) In Vitro Aurora-A kinase inhibition test

This assay determines the ability of a test compound to inhibit serine-threonine kinase activity. DNA encoding Aurora-A may be obtained by total gene synthesis or by cloning. This DNA may then be expressed in a suitable expression system to obtain polypeptide with serine-threonine kinase activity. In the case of Aurora-A, the coding sequence was isolated from cDNA by polymerase chain reaction (PCR) and cloned into the BamH1 and Not1 restriction endonuclease sites of the baculovirus expression vector pFastBac HTc (GibcoBRL/Life technologies). The 5' PCR primer contained a recognition sequence for the restriction endonuclease BamH1 5' to the Aurora-A coding sequence. This allowed the insertion of the Aurora-A gene in frame with the 6 histidine residues, spacer region and rTEV protease cleavage site encoded by the pFastBac HTc vector. The 3' PCR primer replaced the Aurora-A stop codon with additional coding sequence followed by a stop codon and a recognition sequence for the restriction endonuclease Not1. This additional coding sequence (5' TAC CCA TAC GAT GTT CCA GAT TAC GCT TCT TAA 3') encoded for the polypeptide sequence YPYDVPDYAS. This sequence, derived from the influenza hemagglutin protein, is frequently used as a tag epitope sequence that can be identified using specific monoclonal antibodies. The recombinant pFastBac vector therefore encoded for an N-terminally 6 his tagged, C terminally influenza hemagglutin epitope tagged Aurora-A protein. Details of the methods for the assembly of recombinant DNA molecules can be found in standard texts, for example Sambrook et al. 1989, Molecular Cloning - A Laboratory Manual, 2nd Edition, Cold Spring Harbor Laboratory press and Ausubel et al. 1999, Current Protocols in Molecular Biology, John Wiley and Sons Inc.

Production of recombinant virus can be performed following manufacturer's protocol from GibcoBRL. Briefly, the pFastBac-1 vector carrying the Aurora-A gene was transformed into E. coli DH10Bac cells containing the baculovirus genome (bacmid DNA) and via a transposition event in the cells, a region of the pFastBac vector containing gentamycin resistance gene and the Aurora-A gene including the baculovirus polyhedrin promoter was transposed directly into the bacmid DNA. By selection on gentamycin, kanamycin, tetracycline and X-gal, resultant white colonies should contain recombinant bacmid DNA encoding Aurora-A. Bacmid DNA was extracted from a small scale culture of several BH10Bac white colonies and transfected into Spodoptera frugiperda Sf21 cells grown in TC100 medium (GibcoBRL) containing 10% serum using CellFECTIN reagent (GibcoBRL) following manufacturer's instructions. Virus particles were harvested by collecting cell culture medium 72 hrs post transfection. 0.5 mls of medium was used to infect 100 ml suspension culture of Sf21s containing 1 x 10⁷ cells/ml. Cell culture medium was harvested 48 hrs post infection and virus titre determined using a standard plaque assay procedure. Virus stocks were used to infect Sf9 and "High 5" cells at a multiplicity of infection (MOI) of 3 to ascertain expression of recombinant Aurora-A protein.

For the large scale expression of Aurora-A kinase activity, Sf21 insect cells were grown at 28°C in TC100 medium supplemented with 10% foetal calf serum (Viralex) and 0.2% F68 Pluronic (Sigma) on a Wheaton roller rig at 3 r.p.m. When the cell density reached 1.2x10⁶ cells ml⁻¹ they were infected with plaque-pure Aurora-A recombinant virus at a multiplicity of infection of 1 and harvested 48 hours later. All subsequent purification steps were performed at 4°C. Frozen insect cell pellets containing a total of 2.0 x 10⁸ cells were thawed and diluted with lysis buffer (25 mM HEPES (N-[2-hydroxyethyl]piperazine-N'-[2-ethanesulphonic acid]) pH7.4 at 4°C , 100 mM KC1, 25 mM NaF, 1 mM Na₃VO₄, 1 mM PMSF (phenylmethylsulphonyl fluoride), 2 mM 2-mercaptoethanol, 2 mM imidazole, 1 µg/ml aprotinin, 1 µg/ml pepstatin, 1 µg/ml leupeptin), using 1.0 ml per 3 x 10⁷ cells. Lysis was achieved using a dounce homogeniser, following which the lysate was centrifuged at 41,000g for 35 minutes. Aspirated supernatant was pumped onto a 5 mm diameter chromatography column containing 500 µl Ni NTA (nitrilo-tri-acetic acid) agarose (Qiagen, product no. 30250) which had been equilibrated in lysis buffer. A baseline level of UV absorbance for the eluent was reached after washing the column with 12 ml of lysis buffer followed by 7 ml of wash buffer (25 mM HEPES pH7.4 at 4°C , 100 mM KCl, 20 mM imidazole, 2 mM 2-mercaptoethanol). Bound Aurora-A protein was eluted from the column using elution buffer (25 mM HEPES pH7.4 at 4°C , 100 mM KCI, 400 mM imidazole, 2 mM 2-mercaptoethanol). An elution fraction (2.5 ml) corresponding to the peak in UV absorbance was collected. The elution fraction, containing active Aurora-A kinase, was dialysed exhaustively against dialysis buffer (25 mM HEPES pH7.4 at 4°C , 45% glycerol (v/v), 100 mM KCl, 0.25% Nonidet P40 (v/v), 1 mM dithiothreitol).

Each new batch of Aurora-A enzyme was titrated in the assay by dilution with enzyme diluent (25mM Tris-HCl pH7.5, 12.5mM KCl, 0.6mM DTT). For a typical batch, stock enzyme is diluted 1 in 666 with enzyme diluent & 20µl of dilute enzyme is used for each assay well. Test compounds (at 10mM in dimethylsulphoxide (DMSO) were diluted with water & 10µl of diluted compound was transferred to wells in the assay plates. "Total" & "blank" control wells contained 2.5% DMSO instead of compound. Twenty microlitres of freshly diluted enzyme was added to all wells, apart from "blank" wells. Twenty microlitres of enzyme diluent was added to "blank" wells. Twenty microlitres of reaction mix (25mM Tris-HCl, 78.4mM KCl, 2.5mM NaF, 0.6mM dithiothreitol, 6.25mM MnCl₂, 6.25mM ATP, 7.5µM peptide substrate [biotin-LRRWSLGLRRWSLGLRRWSLGLRRWSLG]) containing 0.2µCi [γ³³P]ATP (Amersham Pharmacia, specific activity ≥2500Ci/mmol) was then added to all test wells to start the reaction. The plates were incubated at room temperature for 60 minutes. To stop the reaction 100µl 20% v/v orthophosphoric acid was added to all wells. The peptide substrate was captured on positively-charged nitrocellulose P30 filtermat (Whatman) using a 96-well plate harvester (TomTek) & then assayed for incorporation of ³³P with a Beta plate counter. "Blank" (no enzyme) and "total" (no compound) control values were used to determine the dilution range of test compound which gave 50% inhibition of enzyme activity. In this test, the compounds of the invention generally give 50% inhibition of enzyme activity at concentrations of 1nM to 1000nM and in particular compound 5 in Table 1 gave 50% inhibition of enzyme activity at a concentration of 5µM and compound 8 in Table 1 gave 50% inhibition of enzyme activity at a concentration of 6µM.

### (b) In Vitro Aurora-B kinase inhibition test

This assay determines the ability of a test compound to inhibit serine-threonine kinase activity. DNA encoding Aurora-B may be obtained by total gene synthesis or by cloning. This DNA may then be expressed in a suitable expression system to obtain polypeptide with serine-threonine kinase activity. In the case of Aurora-B, the coding sequence was isolated from cDNA by polymerase chain reaction (PCR) and cloned into the pFastBac system in a manner similar to that described above for Aurora-A (i.e. to direct expression of a 6-histidine tagged Aurora-B protein).

For the large scale expression of Aurora-B kinase activity, Sf21 insect cells were grown at 28°C in TC100 medium supplemented with 10% foetal calf serum (Viralex) and 0.2% F68 Pluronic (Sigma) on a Wheaton roller rig at 3 r.p.m. When the cell density reached 1.2x10⁶ cells ml⁻¹ they were infected with plaque-pure Aurora-B recombinant virus at a multiplicity of infection of 1 and harvested 48 hours later. All subsequent purification steps were performed at 4°C. Frozen insect cell pellets containing a total of 2.0 x 10⁸ cells were thawed and diluted with lysis buffer (50 mM HEPES (N-[2-hydroxyethyl]piperazine-N'-[2-ethanesulphonic acid]) pH7.5 at 4°C , 1 mM Na₃VO₄, 1 mM PMSF (phenylmethylsulphonyl fluoride), 1 mM dithiothreitol, 1 µg/ml aprotinin, 1 µg/ml pepstatin, 1 µg/ml leupeptin), using 1.0 ml per 2 x 10⁷ cells. Lysis was achieved using a sonication homogeniser, following which the lysate was centrifuged at 41,000g for 35 minutes. Aspirated supernatant was pumped onto a 5 mm diameter chromatography column containing 1.0 ml CM sepharose Fast Flow (Amersham Pharmacia Biotech) which had been equilibrated in lysis buffer. A baseline level of UV absorbance for the eluent was reached after washing the column with 12 ml of lysis buffer followed by 7 ml of wash buffer (50 mM HEPES pH7.4 at 4°C , 1 mM dithiothreitol). Bound Aurora-B B protein was eluted from the column using a gradient of elution buffer (50 mM HEPES pH7.4 at 4°C , 0.6 M NaCl, 1 mM dithiothreitol, running from 0% elution buffer to 100% elution buffer over 15 minutes at a flowrate of 0.5 ml/min). Elution fractions (1.0 ml) corresponding to the peak in UV absorbance was collected. Elution fractions were dialysed exhaustively against dialysis buffer (25 mM HEPES pH7.4 at 4°C , 45% glycerol (v/v), 100 mM KCl, 0.05% (v/v) IGEPAL CA630 (Sigma Aldrich), 1 mM dithiothreitol). Dialysed fractions were assayed for Aurora-B kinase activity.

Each new batch of Aurora-B enzyme was titrated in the assay by dilution with enzyme diluent (25mM Tris-HCl pH7.5, 12.5mM KCl, 0.6mM DTT). For a typical batch, stock enzyme is diluted 1 in 40 with enzyme diluent & 20µl of dilute enzyme is used for each assay well. Test compounds (at 10mM in dimethylsulphoxide (DMSO) were diluted with water & 10µl of diluted compound was transferred to wells in the assay plates. "Total" & "blank" control wells contained 2.5% DMSO instead of compound. Twenty microlitres of freshly diluted enzyme was added to all wells, apart from "blank" wells. Twenty microlitres of enzyme diluent was added to "blank" wells. Twenty microlitres of reaction mix (25mM Tris-HCl, 78.4mM KCl, 2.5mM NaF, 0.6mM dithiothreitol, 6.25mM MnCl₂, 37.5mM ATP, 25µM peptide substrate [biotin-LRRWSLGLRRWSLGLRRWSLGLRRWSLG]) containing 0.2µCi [γ³³P]ATP (Amersham Pharmacia, specific activity ≥2500Ci/mmol) was then added to all test wells to start the reaction. The plates were incubated at room temperature for 60 minutes. To stop the reaction 100µl 20% v/v orthophosphoric acid was added to all wells. The peptide substrate was captured on positively-charged nitrocellulose P30 filtermat (Whatman) using a 96-well plate harvester (TomTek) & then assayed for incorporation of ³³P with a Beta plate counter. "Blank" (no enzyme) and "total" (no compound) control values were used to determine the dilution range of test compound which gave 50% inhibition of enzyme activity. In this test, the compounds of the invention generally give 50% inhibition of enzyme activity at concentrations of 1nM to 1000nM and in particular compound 5 in Table 1 gave 50% inhibition of enzyme activity at a concentration of 2µM and compound 8 in Table 1 gave 50% inhibition of enzyme activity at a concentration of 1µM.

### (c) In Vitro cell proliferation assay

This and other assays can be used to determine the ability of a test compound to inhibit the growth of adherent mammalian cell lines, for example the human tumour cell line SW620 (ATCC CCL-227). This assay determines the ability of at test compound to inhibit the incorporation of the thymidine analogue, 5'-bromo-2'-deoxy-uridine (BrdU) into cellular DNA. SW620 or other adherent cells were typically seeded at 1x10⁵ cells per well in L-15 media (GIBCO) plus 5% foetal calf serum, 1% L-glutamine (100µl / well) in 96 well tissue culture treated 96 well plates (Costar) and allowed to adhere overnight. The following day the cells were dosed with compound (diluted from 10mM stock in DMSO using L-15 (with 5% FCS, 1% L-glutamine). Untreated control wells and wells containing a compound known to give 100% inhibition of BrdU incorporation were included on each plate. After 48 hours in the presence / absence of test compound the ability of the cells to incorporate BrdU over a 2 hour labelling period was determined using a Boehringer (Roche) Cell Proliferation BrdU ELISA kit (cat. No. 1 647 229) according to manufacturers directions. Briefly, 15 µl of BrdU labelling reagent (diluted 1:100 in media - L-15, 5% FCS, 1% L-glutamine) was added to each well and the plate returned to a humidified (+5% CO₂) 37°C incubator for 2 hours. After 2 hours the labelling reagent was removed by decanting and tapping the plate on a paper towel. FixDenat solution (50µl per well) was added and the plates incubated at room temperature for 45mins with shaking. The FixDenat solution was removed by decanting and tapping the inverted plate on a paper towel. The plate was then washed once with phosphate buffered saline (PBS) and 100µl /well of Anti-BrdU-POD antibody solution (diluted 1:100 in antibody dilution buffer) added. The plate was then incubated at room temperature with shaking for 90min. Unbound Anti-BrdU-POD antibody was removed by decanting and washing the plate 4 times with PBS before being blotted dry. TMB substrate solution was added (100µl/well) and incubated for approximately 10 minutes at room temperature with shaking until a colour change was apparent. The optical density of the wells was then determined at 690nm wavelength using a Titertek Multiscan plate reader. The values from compound treated, untreated and 100% inhibition controls were used to determine the dilution range of a test compound that gave 50% inhibition of BrdU incorporation. The compounds of the invention are generally active at 1nM to 100µM in this test.

### (d) In Vitro cell cycle analysis assay

This assay determines the ability of a test compound to arrest cells in specific phases of the cell cycle. Many different mammalian cell lines could be used in this assay and SW620 cells are included here as an example. SW620 cells were seeded at 7 x 10⁵ cells per T25 flask (Costar) in 5 ml L-15 (5% FCS, 1% L-glutamine). Flasks were then incubated overnight in a humidified 37°C incubator with 5% CO₂. The following day, 5µl of L-15 (5% FCS, 1% L-glutamine) carrying the appropriate concentration of test compound solubilised in DMSO was added to the flask. A no compound control treatment was also included (0.5% DMSO). The cells were then incubated for a defined time (24 hours) with compound. After this time the media was aspirated from the cells and they were washed with 5ml of prewarmed (37°C) sterile PBSA, then detached from the flask by brief incubation with trypsin and followed by resuspension in 5ml of 1% Bovine Serum Albumin (BSA, Sigma-Aldrich Co.) in sterile PBSA. The samples were then centrifuged at 2200rpm for 10 min. The supernatant was aspirated to leave 200µl of the PBS/BSA solution. The pellet was resuspended in this 200µl of solution by pipetting 10 times to create a single cell suspension. One ml of ice-cold 80% ethanol was slowly added to each cell suspension and the samples stored at -20°C overnight or until required for staining. Cells were pelleted by centrifugation, ethanol aspirated off and pellets resuspended in 200µl PBS containing 100µg/ml RNAse (Sigma Aldrich) & 10µg/ml Propidium Iodide (Sigma Aldrich). Cell suspensions were incubated at 37°C for 30min, a further 200µl PBS added and samples stored in the dark at 4°C overnight.

Each sample was then syringed 10 times using 21-guage needle. The samples were then transferred to LPS tubes and DNA content per cell analysed by Fluorescence activated cell sorting (FACS) using a FACScan flow cytometer (Becton Dickinson). Typically 30,000 events were counted and recorded using CellQuest v1.1 software (Verity Software). Cell cycle distribution of the population was calculated using Modfit software (Verity Software) and expressed as percentage of cells with 2N (G0/G1), 2N-4N (S phase) and with 4N (G2/M) DNA content.

The compounds of the invention are generally active in this test at 1nM to 10µM.

The invention will now be illustrated in the following examples, in which standard techniques known to the skilled chemist and techniques analogous to those described in these examples may be used where appropriate, and in which, unless otherwise stated:
(i) evaporations were carried out by rotary evaporation *in vacuo* and work up procedures were carried out after removal of residual solids such as drying agents by filtration;
(ii) operations were carried out at ambient temperature, typically in the range 18-25°C and in air unless stated, or unless the skilled person would otherwise operate under an atmosphere of an inert gas such as argon;
(iii) column chromatography (by the flash procedure) and medium pressure liquid chromatography (MPLC) were performed on Merck Kieselgel silica (Art. 9385);
(iv) yields are given for illustration only and are not necessarily the maximum attainable;
(v) the structures of the end products of the formula (I) were generally confirmed by nuclear (generally proton) magnetic resonance (NMR) and mass spectral techniques; proton magnetic resonance chemical shift values were measured in deuterated dimethyl sulphoxide (DMSO d₆) (unless otherwise stated) on the delta scale (ppm downfield from tetramethylsilane) using one of the following four instruments
   - Varian Gemini 2000 spectrometer operating at a field strength of 300 MHz
   - Bruker DPX300 spectrometer operating at a field strength of 300MHz
   - JEOL EX 400 spectrometer operating at a field strength of 400 MHz
   - Bruker Avance 500 spectrometer operating at a field strength of 500MHz
   Peak multiplicities are shown as follows: s, singlet; d, doublet; dd, double doublet; t, triplet; q, quartet; qu, quintet; m, multiplet; br s, broad singlet;
(vi) robotic synthesis was carried out using a Zymate XP robot, with solution additions via a Zymate Master Laboratory Station and stirred via a Stem RS5000 Reacto-Station at 25°C;
(vii) work up and purification of reaction mixtures from robotic synthesis was carried out as follows: evaporations were carried out *in vacuo* using a Genevac HT 4; column chromatography was performed using either an Anachem Sympur MPLC system on silica using 27 mm diameter columns filled with Merck silica (60 *µ*m, 25 g); the structures of the final products were confirmed by LCMS on a Waters 2890 / ZMD micromass system using the following and are quoted as retention time (RT) in minutes:
   - Column:: waters symmetry C18 3.5 µm 4.6x50 mm
   - Solvent A:: H₂O
   - Solvent B:: CH₃CN
   - Solvent C :: MeOH + 5% HCOOH
   - Flow rate:: 2.5 ml / min
   - Run time:: 5 minutes with a 4.5 minute gradient from 0-100% C
   - Wavelength:: 254 nm, bandwidth 10 nm
   - Mass detector:: ZMD micromass
   - Injection volume: 0.005 ml
(viii) Analytical LCMS for compounds which had not been prepared by robotic synthesis was performed on a Waters Alliance HT system using the following and are quoted as retention time (RT) in minutes:
   - Column:: 2.0 mm x 5 cm Phenomenex Max-RP 80A
   - Solvent A:: Water
   - Solvent B:: Acetonitrile
   - Solvent C:: Methanol / 1% formic acid or Water / 1% formic acid
   - Flow rate:: 1.1 ml/min
   - Run time:: 5 minutes with a 4.5 minute gradient from 0-95% B + constant 5% solvent C
   - Wavelength:: 254 nm, bandwidth 10 nm
   - Injection volume: 0.005 ml
   - Mass detector:: Micromass ZMD
(ix) Preparative high performance liquid chromatography (HPLC) was performed on either
   - Waters preparative LCMS instrument, with retention time (RT) measured in minutes:
      - Column:: β-basic Hypercil (21x100 mm) 5 *µ*m
      - Solvent A:: Water / 0.1 % Ammonium carbonate
      - Solvent B:: Acetonitrile
      - Flow rate:: 25 ml / min
      - Run time:: 10 minutes with a 7.5 minute gradient from 0-100% B
      - Wavelength:: 254 nm, bandwidth 10 nm
      - Injection volume: 1 - 1.5 ml
      - Mass detector :: Micromass ZMD
   - Gilson preparative HPLC instrument, with retention time (RT) measured in minutes:
      - Column:: 21 mm x 15 cm Phenomenex Luna2 C18
      - Solvent A:: Water +0.1% trifluoracetic acid,
      - Solvent B:: Acetonitrile + 0.1% trifluoracetic acid
      - Flow rate:: 21 ml / min
      - Run time:: 20 minutes with various 10 minute gradients from 5-100% B
      - Wavelength:: 254 nm, bandwidth 10 nm
      - Injection volume: 0.1-4.0 ml
(x) intermediates were not generally fully characterised and purity was assessed by thin layer chromatography (TLC), HPLC, infra-red (IR), MS or NMR analysis.

**Table 1**

| | | | |
|---|---|---|---|
| 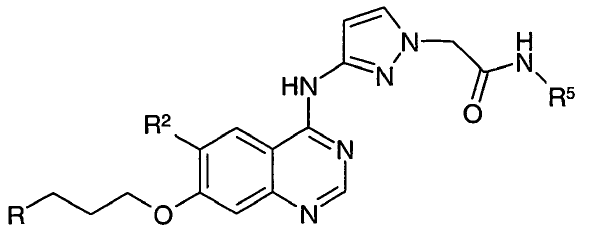 | | | |

| **Compound** | **R²** | **R** | **R⁵** |
|---|---|---|---|
| 1 | H | 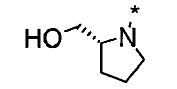 | 3-fluorophenyl |
| 2 | H | 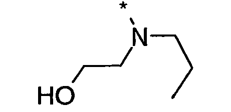 | 3-fluorophenyl |
| 3 | H | 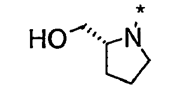 | 2,3- difluorophenyl |
| 4 | H | 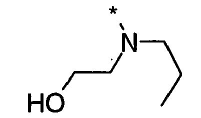 | 2,3- difluorophenyl |
| 5 | OMe | 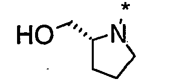 | 3-fluorophenyl |
| 6 | OMe | 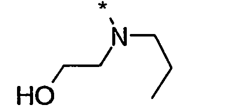 | 3-fluorophenyl |
| 7 | OMe | 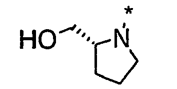 | 2,3- difluorophenyl |
| 8 | OMe | 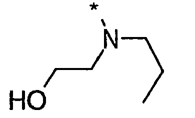 | 2,3- difluorophenyl |

**Table 2**

| | | | |
|---|---|---|---|
| 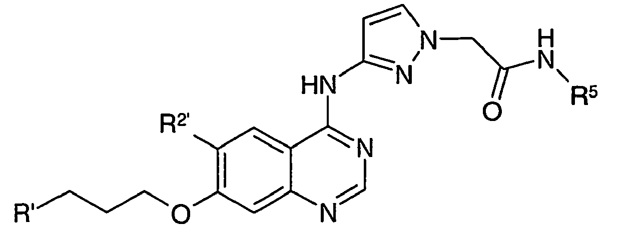 | | | |

| **Compound** | **R^{2'}** | **R'** | **R⁵** |
|---|---|---|---|
| 9 | H | 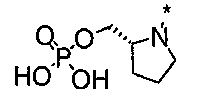 | 3-fluorophenyl |
| 10 | H | 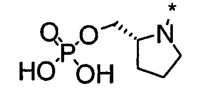 | 2,3- difluorophenyl |
| 11 | OMe | 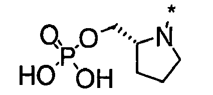 | 3-fluorophenyl |
| 12 | OMe | 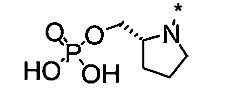 | 2,3- difluorophenyl |

### Example 1- Preparation of Compound 1 in Table 1- N-(3-fluorophenyl)-2-{3-[(7-{3-[(2R)-2-(hydroxymethyl)pyrrolidin-1-yl]propoxy}quinazolin-4-yl)amino]-1H-pyrazol-1-yl}acetamide

Potassium iodide (100 mg, 0.6 mmol), 2-(3-{[7-(3-chloropropoxy)quinazolin-4-yl]amino}-1*H*-pyrazol-1-yl)-*N*-(3-fluorophenyl)acetamide hydrochloride (147 mg, 0.30 mmol) and D-prolinol (152 mg, 1.5 mmol) were heated in dimethylacetamide (1 ml) for 2.5 hours at 90 °C. The reaction was cooled to ambient temperature, diluted with dichloromethane (10 ml) and purified by flash chromatography on silica gel, eluting with dichloromethane : methanol : aqueous ammonia (100:5:0.5) to (100:25:2). Evaporation of the fractions in vacuo afforded a clear oil which was crystallised by trituration with acetonitrile : dichloromethane : isohexane to yield compound 1 in table 1 (83 mg, 53 % yield) as a cream coloured solid :
¹H-NMR (DMSO d₆): 10.50 (br s, 1H), 10.27 (s, 1H), 8.53 (m, 2H), 7.75 (s, 1H), 7.59 (m, 1H), 7.34 (m, 2H), 7.14 (m, 2H), 6.92 (m, 1H), 6.86 (s, 1H), 4.98 (s, 2H), 4.30 (br s, 1H), 4.17 (t, 2H), 3.38 (m, 1H), 3.19 (m, 1H), 3.06 (m, 1H), 2.96 (m, 1H), 2.42 (m, 2H), 2.17 (m, 1H), 1.92 (m, 2H), 1.80 (m, 1H), 1.64 (m, 2H), 1.54 (m, 1H) :
MS (+ve ESI) : 520 (M+H)⁺,
MS (-ve ESI) : 518 (M-H)⁻.
2-(3- {[7-(3-chloropropoxy)quinazolin-4-yl]amino}-1*H*-pyrazol-1-yl)-*N*-(3-fluorophenyl)acetamide hydrochloride, used as the starting material, was obtained as follows:
a) 2-Amino-4-fluorobenzoic acid (15 g, 96 mmol) was dissolved in 2-methoxyethanol (97 ml). Formamidine acetate (20.13 g, 193.4 mmol) was added and the mixture heated to reflux for 18 hours. The reaction was cooled, concentrated and the residue stirred in aqueous ammonium hydroxide (0.01 N, 250 ml) for 1 hour. The suspension was filtered, washed with water and dried over phosphorus pentoxide to yield 7-fluoroquinazolin-4(3H)-one as an off-white solid (10.35 g, 65 % yield) :
   ¹H-NMR (DMSO d₆) : 12.32 (br s, 1H), 8.19 (dd, 1H), 8.14 (s, 1H), 7.45 (dd, 1H), 7.39 (m, 1H) :
   MS (-ve ESI) : 163 (M-H)⁻,
   MS (+ve ESI): 165 (M+H)⁺.
b) Sodium hydride (14.6 g, 365 mmol) was added at 0 °C to a solution of 1,3-propanediol (27.8 g, 365 mmol) in dimethylformamide (70 ml). 7-Fluoroquinazolin-4(3H)-one (10 g, 60.9 mmol) was added portionwise and the reaction mixture heated at 60 °C, then at 110 °C for 3 hours. The reaction was cooled to 0 °C, quenched with water (280 ml) and adjusted to pH 5.9. The resulting suspension was filtered, washed with water then diethyl ether and dried over phosphorus pentoxide to afford 7-(3-hydroxypropoxy)quinazolin-4(3H)-one as a white powder (12.41 g, 92 % yield) :
   ¹H-NMR (DMSO d₆) : 11.90 (br s, 1H), 8.04 (s, 1H), 8.00 (d, 1H), 7.10 (m, 2H), 4.17 (t, 2H), 3.58 (t, 2H), 1.92 (m, 2H) :
   MS (+ve ESI) : 221 (M+H)⁺.
c) 7-(3-hydroxypropoxy)quinazolin-4(3H)-one (10.5 g, 47.7 mmol) and thionyl chloride (100 ml, 137 mmol) were combined. Dimethylformamide (1 ml) was added and the reaction mixture heated to 85 °C for 1 hour. The mixture was cooled to room temperature, diluted with toluene and evaporated to dryness. This was repeated until all thionyl chloride was removed. The residue was dissolved in dichloromethane and washed with a saturated sodium bicarbonate solution. The aqueous layer was extracted with dichloromethane. The organics were combined, dried (magnesium sulphate) and concentrated to leave a yellow solid. Trituration with ether removed a less soluble impurity and the ether filtrate was concentrated to leave 4-chloro-7-(3-chloropropoxy)quinazoline as an off-white solid (8.5 g, 70 % yield) :
   ¹H-NMR (DMSO d₆) : 13.25 (br s, 1H), 8.34 (s, 1H), 8.06 (d, 1H), 7.17 (m, 2H), 4.21 (t, 2H), 3.83 (t, 2H), 2.23 (m, 2H) :
   MS (+ve ESI) : 257, 259 (M+H)⁺.
d) 4.0 N Hydrochloric acid in dioxane (0.46 ml, 1.85 mmol) was added dropwise to a stirred solution of 4-chloro-7-(3-chloropropoxy)quinazoline (475 mg, 1.85 mmol) and 2-(3-amino-1*H*-pyrazol-1-yl)-*N*-(3-fluorophenyl)acetamide (430 mg, 1.85 mmol) in dimethylacetamide (5 ml) at 50 °C. The reaction was heated at 70 °C for 1 hour, allowed to cool to ambient temperature and then stirred with diethyl ether (5 ml) at ambient temperature for 1 hour. Collection of the solid which precipitated by suction filtration, followed by washing with diethyl ether and acetonitrile and prolonged drying *in vacuo,* yielded 2-(3-{[7-(3-chloropropoxy)quinazolin-4-yl]amino}-1*H*-pyrazol-1-yl)-*N*-(3-fluorophenyl)acetamide hydrochloride (919 mg, 99 % yield) as a pale yellow solid :
   ¹H-NMR (DMSO d₆): 11.93 (br s, 1H), 10.96 (br s, 1H), 8.96 (s, 1H), 8.79 (d, 1H), 7.90 (d, 1H), 7.62 (m, 1H), 7.45 (m, 1H), 7.38 (m, 3H), 6.90 (m, 1H), 6.85 (d, 1H), 5.12 (s, 2H), 4.31 (t, 2H), 3.84 (t, 2H), 2.26 (m, 2H) :
   MS (+ve ESI): 455 (M+H)⁺,
   MS (-ve ESI) : 453 (M-H)⁻.
   2-(3-amino-1*H*-pyrazol-1-yl)-*N*-(3-fluorophenyl)acetamide, used as the starting material in reaction 1d), was obtained as follows:
e) 1.0 N Aqueous sodium hydroxide solution (54 ml, 54 mmol) was added to a stirred solution of 3-fluoroaniline (11.1 g, 100 mmol) in diethyl ether (100 ml) and the reaction cooled to °C. A solution of bromoacetyl bromide (10.2 ml, 117 mmol) in diethyl ether (100 ml) was added with vigorous stirring over 20 minutes keeping the temperature below 0 °C. The reaction was allowed to warm to ambient temperature over 10 minutes before the diethyl ether layer was separated, dried over magnesium sulphate and evaporated *in vacuo.* The product was purified by flash chromatography on a small pad of silica gel, eluting with dichloromethane : diethyl ether (95:5). Addition of isohexane to the fractions from the column, followed by concentration *in vacuo* caused precipitation of a white solid which was collected by suction filtration and dried *in vacuo* to yield 2-bromo-*N*-(3-fluorophenyl)-acetamide (21.7 g, 94 % yield) as a white crystalline solid:
   ¹H-NMR (DMSO d₆): 7.58 (m, 1H), 7.35 (m, 1H), 7.28 (m, 1H), 6.90 (m, 1H), 4.05 (s, 2H): MS (-ve ESI) : 230, 232 (M-H)⁻.
f) A solution of 2-bromo-*N*-(3-fluorophenyl)-acetamide (2.78 g, 12.0 mmol) in dichloromethane was added dropwise over 5 minutes to a stirred suspension of 1.0N aqueous sodium hydroxide (28.8 ml, 28.8 mmol), 3-nitro-1*H*-pyrazole (1.50 g, 13.2 mmol) and *tetra-n-*butylammonium sulphate (4.88 g, 14.4 mmol) in dichloromethane (10 ml) at 2 °C. The reaction was allowed to warm to ambient temperature over 4 hours after which time the organic layer was separated and passed through a short plug of silica gel (washed with diethyl ether). Evaporation of the combined organic layers *in vacuo* yielded *N*-(3-fluorophenyl)-2-(3-nitro-1*H*-pyrazol-1-yl)acetamide (1.41 g, 45 % yield) as a white solid:
   ¹H-NMR (DMSO d₆) : 10.62 (s, 1H), 8.08 (d, 1H), 7.55 (m, 1H), 7.38 (m, 1H), 7.31 (m, 1H), 7.10 (d, 1H), 6.93 (m, 1H), 5.23 (s, 2H) :
   MS (-ve ESI) : 263 (M-H)⁻.
g) A solution of *N*-(3-fluorophenyl)-2-(3-nitro-1*H*-pyrazol-1-yl)acetamide (1.33 g, 50.5 mmol) in ethyl acetate (50 ml) and ethanol (12.5 ml) was hydrogenated under an atmosphere of hydrogen in the presence of 10% palladium on carbon (130 mg) for 4 hours. The catalyst was removed by filtration through Celite and evaporation of the solvents *in vacuo* yielded 2-(3-amino-1*H*-pyrazol-1-yl)-*N*-(3-fluorophenyl)acetamide (898 mg, 76 % yield) as a white crystalline solid:
   ¹H-NMR (DMSO d₆) : 10.36 (br s, 1H), 7.58 (m, 1H), 7.38 (m, 2H), 7.32 (m, 1H), 5.45 (d, 1H), 4.69 (s, 2H), 4.57 (br s, 2H) :
   MS (+ve ESI) : 235 (M+H)⁺,
   MS (-ve ESI): 233 (M-H)⁻.

### Example 2- Preparation of Compound 2 in Table 1 - N-(3-fluorophenyl)-2-{3-[(7-{3-[(2-hydroxyethyl)(propyl)amino]propoxy}quinazolin-4-yl)amino]-1H-pyrazol-1-yl}acetamide

An analogous reaction to that described in example 1, but starting with 2-(3-{[7-(3-chloropropoxy)quinazolin-4-yl]amino}-1*H*-pyrazol-yl)-*N*-(3-fluorophenyl)acetamide hydrochloride (147 mg, 0.30 mmol) and 2-(*n*-propylamino)ethanol (155 mg, 1.5 mmol) yielded compound 2 in table 1 (96 mg, 61 % yield) as a pale yellow solid :
¹H-NMR (DMSO d₆): 10.50 (s, 1H), 10.27 (s, 1H), 8.53 (m, 2H), 7.75 (m, 1H), 7.60 (m, 1H), 7.34 (m, 2H), 7.14 (m, 2H), 6.91 (m, 1H), 6.87 (s, 1H), 4.99 (s, 2H), 4.32 (br s, 1H), 4.18 (t, 2H), 3.46 (m, 2H), 2.62 (m, 2H), 2.53 (m, 2H), 2.32 (m, 2H), 1.91 (m, 2H), 1.42 (m, 2H), 0.84 (t, 3H) :
MS (+ve ESI): 522 (M+H)⁺,
MS (-ve ESI): 520 (M-H)⁻.

### Example 3 - Preparation of Compound 3 in Table 1- N-(2,3-difluorophenyl)-2-{3-[(7-{3-[(2R)-2-(hydroxymethyl)pyrrolidin-1-yl]propoxy}quinazolin-4-yl)amino]-1H-pyrazol-1-yl}acetamide

An analogous reaction to that described in example 1, but starting with 2-(3-{[7-(3-chloropropoxy)quinazolin-4-yl]amino}-1*H*-pyrazol-1-yl)-*N*-(2,3-difluorophenyl)acetamide hydrochloride (153 mg, 0.30 mmol) and D-prolinol (152 mg, 1.5 mmol) yielded compound 3 in table 1 (123 mg, 76 % yield) as a pale yellow solid :
¹H-NMR (DMSO d₆) : 10.29 (br s, 2H), 8.52 (m, 2H), 7.74 (m, 2H), 7.18 (m, 4H), 6.87 (s, 1H), 5.09 (s, 2H), 4.28 (br s, 1H), 4.18 (t, 2H), 3.39 (m, 1H), 3.19 (m, 1H), 3.06 (m, 1H), 2.97 (m, 1H), 2.44 (m, 2H), 2.17 (m, 1H), 1.91 (m, 2H), 1.80 (m, 1H), 1.64 (m, 2H), 1.52 (m, 1H) :
MS (+ve ESI): 538 (M+H)⁺,
MS (-ve ESI): 536 (M-H)⁻.
2-(3-{[7-(3-chloropropoxy)quinazolin-4-yl]amino}-1*H*-pyrazol-1-yl)-*N*-(2,3-difluorophenyl)acetamide hydrochloride, used as the starting material, was obtained as follows:
a) An analogous reaction to that described in example 1e), but starting with 2,3-difluoroaniline (12.9 g, 100 mmol) yielded 2-bromo-*N*-(2,3-difluorophenyl)acetamide (23.9 g, 96 % yield) as a white solid :
   ¹H-NMR (DMSO d₆) : 7.68 (m, 1H), 7.20 (m, 2H), 4.17 (s, 2H) :
   MS (-ve ESI): 248, 250 (M-H)⁻.
b) An analogous reaction to that described in example 1f), but starting with 2-bromo-*N-*(2,3-difluorophenyl)acetamide (2.00 g, 8.00 mmol) and 3-nitro-1*H*-pyrazole (1.00 g, 8.85 mmol) yielded *N*-(2,3-difluorophenyl)-2-(3-nitro-1*H*-pyrazol-1-yl)acetamide (1.15 g, 51 % yield) as a white crystalline solid :
   ¹H-NMR (DMSO d₆) : 8.05 (d, 1H), 7.69 (m, 1H), 7.18 (m, 2H), 7.08 (d, 1H), 5.31 (s, 2H) :
   MS (-ve ESI): 281 (M-H)⁻.
c) An analogous reaction to that described in example 1g), but starting with *N*-(2,3-difluorophenyl)-2-(3-nitro-1*H*-pyrazol-1-yl)acetamide (1.13 g, 4.00 mmol) yielded 2-(3-amino-1*H*-pyrazol-1-yl)-*N*-(2,3-difluorophenyl)acetamide (934 mg, 93 % yield) as a white crystalline solid :
   ¹H-NMR (DMSO d₆): 10.08 (br s, 1H), 7.73 (m, 1H), 7.38 (m, 1H), 7.17 (m, 2H), 5.45 (s, 1H), 4.79 (s, 2H), 4.62 (br s, 2H) :
   MS (+ve ESI) : 253 (M+H)⁺,
   MS (-ve ESI) : 251 (M-H)⁻.
d) An analogous reaction to that described in example 1d), but starting with 4-chloro-7-(3-chloroxypropoxy)quinazoline (450 mg, 1.75 mmol) and 2-(3-amino-1*H*-pyrazol-1-yl)-*N-*(2,3-difluorophenyl)acetamide (440 mg, 1.75 mmol) yielded 2-(3-{[7-(3-chloropropoxy)quinazolin-4-yl]amino}-1*H*-pyrazol-1-yl)-*N*-(2,3-difluorophenyl)acetamide hydrochloride (800 mg, 90 % yield) as a white solid :
   ¹H-NMR (DMSO d₆) : 11.94 (br s, 1H), 10.50 (br s, 1H), 8.95 (s, 1H), 8.80 (d, 1H), 7.90 (s, 1H), 7.68 (m, 1H), 7.34 (m, 2H), 7.21 (m, 2H), 6.96 (s, 1H), 5.20 (s, 2H), 4.32 (t, 2H), 3.94 (t, 2H), 2.26 (m, 2H) :
   MS (+ve ESI): 473 (M+H)⁺,
   MS (-ve ESI): 471 (M-H)⁻.

### Example 4 - Preparation of Compound 4 in Table 1- N-(2,3-difluorophenyl)-2-{3-[(7-{3-[(2-hydroxyethyl)(propyl)amino]propoxy}quinazolin-4-yl)amino]-1H-pyrazol-1-yl}acetamide

An analogous reaction to that described in example 1, but starting with 2-(3-{[7-(3-chloropropoxy)quinazolin-4-yl]amino}-1*H*-pyrazol-1-yl)-*N*-(2,3-difluorophenyl)acetamide hydrochloride (153 mg, 0.30 mmol) and 2-(*n*-propylamino)ethanol (155 mg, 1.5 mmol) yielded compound 4 in table 1 (112 mg, 69 % yield) as a pale yellow solid:
¹H-NMR (DMSO d₆) : 10.29 (br s, 2H), 8.54 (m, 2H), 7.74 (m, 2H), 7.20 (m, 4H), 6.88 (s, 1H), 5.09 (s, 2H), 4.25 (br s, 1H), 4.18 (t, 2H), 3.46 (m, 2H), 2.56 (m, 6H), 1.91 (m, 2H), 1.40 (m, 2H), 0.83 (t, 3H) :
MS (+ve ESI): 540 (M+H)⁺,
MS (-ve ESI): 538 (M-H)⁻.

### Example 5 - Preparation of Compound 5 in Table 1 - N-(3-fluorophenyl)-2-{3-[(7-{3-[(2R)-2-(hydroxymethyl)pyrrolidin-1-yl]propoxy}-6-methoxyquinazolin-4-yl)amino]-1H-pyrazol-1-yl}acetamide

An analogous reaction to that described in example 1, but starting with 2-(3-{[7-(3-chloropropoxy)-6-methoxyquinazolin-yl]amino}-1*H*-pyrazol-1-yl)-*N*-(3-fluorophenyl)acetamide hydrochloride (156 mg, 0.30 mmol) and D-prolinol (152 mg, 1.5 mmol) yielded compound 5 in table 1 (129 mg, 78 % yield) as a pale yellow solid:
¹H-NMR (DMSO d₆): 10.50 (br s, 1H), 10.19 (s, 1H), 8.48 (s, 1H), 7.98 (s, 1H), 7.74 (m, 2H), 7.59 (m, 1H), 7.35 (m, 2H), 7.16 (s, 1H), 6.90 (m, 2H), 4.98 (s, 2H), 4.32 (br s, 1H), 4.18 (t, 2H), 3.91 (s, 3H), 3.40 (m, 1H), 3.23 (m, 1H), 3.10 (m, 1H), 3.00 (m, 1H), 2.46 (m, 2H), 2.20 (m, 1H), 1.96 (m, 2H), 1.81 (m, 1H), 1.67 (m, 2H), 1.56 (m, 1H):
MS (+ve ESI) : 550 (M+H)⁺,
MS (-ve ESI) : 548 (M-H)⁻.
2-(3-{[7-(3-chloropropoxy)-6-methoxyquinazolin-4-yl]amino}-1*H*-pyrazol-1-yl)-*N*-(3-fluorophenyl)acetamide, used as the starting material, was obtained as follows:
a) A mixture of 2-amino-4-benzyloxy-5-methoxybenzamide (10 g, 0.04 mol), (prepared according to J. Med. Chem. 1977, 20, 146-149), and Gold's reagent (7.4 g, 0.05 mol) in dioxane (100 ml) was stirred and heated at reflux for 24 hours. Sodium acetate (3.02 g, 0.037 mol) and acetic acid (1.65 ml, 0.029 mol) were added to the reaction mixture and it was heated for a further 3 hours. The volatiles were removed by evaporation, water was added to the residue, the solid was collected by filtration, washed with water and dried. Recrystallisation from acetic acid yielded 7-(benzyloxy)-6-methoxyquinazolin-4(3*H*)-one (8.7 g, 84 % yield) as a white solid.
b) Chloromethyl pivalate (225ml, 1.56 mol) was added dropwise to a stirred mixture of 7-(benzyloxy)-6-methoxyquinazolin-4(3*H*)-one (400 g, 1.42 mol) and potassium carbonate (783 g, 5.67 mol) in dimethylacetamide (5500 ml). The reaction was heated to 90 °C for 4 hours. The reaction was cooled and filtered to remove inorganic salts. The filtrate was concentrated *in vacuo* to yield, crude *tert*-butyl 2-[7-(benzyloxy)-6-methoxy-4-oxo-3(4H)-quinazolinyl]acetate (562 g, 100 % yield) :
   ¹H-NMR (DMSO-d₆): 8.33 (s, 1H), 7.30-7.50 (m, 6H), 7.25 (s, 1H), 5.90 (s, 2H), 5.25 (s, 2H), 3.88 (s,3H), 1.10 (s, 9H) :
   MS (+ve ESI): 397 (M+H)⁺
c) 10% palladium on carbon (56 g, 53 mmol) was added to a solution of *tert*-butyl 2-[7-(benzyloxy)-6-methoxy-4-oxo-3(4H)-quinazolinyl]acetate (562 g, 1.42 mmol) in dimethylacetamide (3500 ml) at ambient temperature and stirred for 3 hours under an atmosphere of hydrogen (1 bar). The reaction was filtered through a pad of celite and the solvent evaporated *in vacuo.* The residual solid was dissolved in 20% methanol in dichloromethane and passed through a pad of silica gel. Evaporation of the solvent *in vacuo* followed by trituration with methanol yielded, *tert*-butyl
   2-[7-hydroxy-6-methoxy-4-oxo-3(4H)-quinazolinyl]acetate (188 g, 43 % yield) :
   ¹H-NMR (DMSO-d₆) : 8.25 (s, 1H), 7.45 (s, 1H), 6.97 (s, 1H), 5.85 (s, 2H), 4.04 (s, 1H), 3.87 (s, 3H), 1.10 (s, 9H) :
   MS (+ve ESI): 307 (M+H)⁺
d) A mixture of *tert*-butyl 2-[7-hydroxy-6-methoxy-4-oxo-3(4H)-quinazolinyl]-acetate (100g, 0.327 mol), 3-bromopropanol (49.3 g, 0.355 mol) and potassium carbonate (133g, 0.967 mol) in dimethylformamide (500 ml) was stirred at 80 °C for 20 hours. The reaction was cooled and concentrated to quarter volume *in vacuo.* The residue was poured into ice/water (1500 ml) and the resulting solid collected by suction filtration. Purification by crystallisation from ethanol, yielded crude *tert*-butyl
   2-[7-(3-hydroxypropoxy)-6-methoxy-4-oxo-3(4H)-quinazolinyl]acetate (33.8 g, 41 % yield) as a beige solid :
   ¹H-NMR (DMSO-d₆) : 7.95 (s, 1H), 7.43 (s, 1H), 7.1 (s, 1H), 4.16 (t, 2H), 3.86 (m, 5H), 2.08 (t, 2H), 1.12 (s, 9H) :
   MS (+ve ESI): 365 (M+H)⁺
e) Aqueous sodium hydroxide solution (100 ml, 0.2 mol) was added to a solution of *tert*-butyl 2-[7-(3-hydroxypropoxy)-6-methoxy-4-oxo-3 (4H)-quinazolinyl] acetate (33.8 g, 93 mmol) in methanol (300 ml) and the solution heated to reflux for 1 hour. The methanol was evaporated *in vacuo* and the aqueous residue acidified with aqueous hydrochloric acid then sodium bicarbonate was added. Collection of the solid by suction filtration, washing with water and drying gave 7-(3-hydroxypropoxy)-6-methoxy-4-quinazolone (26 g, 95 % yield) :
   ¹H-NMR (DMSO-d₆) : 7.96 (s, 1H), 7.41 (s, 1H), 7.07 (s, 1H), 4.14 (t, 2H), 3.84 (s, 3H), 3.55 (t, 2H), 1.90 (t, 2H) :
   MS (+ve ESI): 251 (M+H)⁺
f) 7-(3-hydroxypropoxy)-6-methoxy-4-quinazolone (25 g, 100 mmol) was added slowly to a solution of dimethylformamide (1 ml) in thionyl chloride (250 ml). The mixture was heated to reflux for 4 hours then cooled and the solvents evaporated *in vacuo.* The residue was dissolved in dichloromethane and washed with aqueous sodium bicarbonate, brine, dried over magnesium sulphate and evaporated. Trituration and collection of the solid by suction filtration yielded, 4-chloro-6-methoxy-7-(3-chloroxypropoxy)quinazoline (19.5 g, 68 % yield) as a yellow solid :
   ¹H-NMR (CDCl₃) : 8.85 (s, 1H), 7.40 (s, 1H), 7.38 (s, 1H), 4.38 (t, 2H), 4.03 (s, 3H), 3.8 (t, 2H), 2.40 (m, 2H) :
   MS (+ve ESI): 287 (M+H)⁺
g) An analogous reaction to that described in example 1d), but starting with 4-chloro-6-methoxy-7-(3-chloroxypropoxy)quinazoline (531 mg, 1.85 mmol) and 2-(3-amino-1*H*-pyrazol-1-yl)-*N*-(3-fluorophenyl)acetamide (430 mg, 1.85 mmol) yielded 2-(3-{[7-(3-chloropropoxy)-6-methoxyquinazolin-4-yl]amino}-1*H*-pyrazol-1-yl)-*N*-(3-fluorophenyl)acetamide hydrochloride (841 mg, 86 % yield) as a white solid :
   ¹H-NMR (DMSO d₆) : 11.80 (br s, 1H), 10.94 (br s, 1H), 8.92 (s, 1H), 8.26 (s, 1H), 7.89 (s, 1H), 7.61 (m, 1H), 7.45 (s, 1H), 7.38 (m, 2H), 6.90 (m, 2H), 5.12 (s, 2H), 4.30 (t, 2H), 3.97 (s, 3H), 3.82 (t, 2H), 2.30 (m, 2H) :
   MS (+ve ESI): 485 (M+H)⁺,
   MS (-ve ESI): 483 (M-H)⁻.

### Example 6 - Preparation of Compound 6 in Table 1- N-(3-fluorophenyl)-2-{3-[(7-{3-[(2-hydroxyethyl)(propyl)amino]propoxy}-6-methoxyquinazolin-4-yl)amino]-1H-pyrazol-1-yl}acetamide

An analogous reaction to that described in example 1, but starting with 2-(3-{[7-(3-chloropropoxy)-6-methoxyquinazolin-4-yl]amino}-1*H*-pyrazol-1-yl)-*N*-(3-fluorophenyl)acetamide hydrochloride (156 mg, 0.30 mmol) and 2-(*n*-propylamino)ethanol (155 mg, 1.5 mmol) yielded compound 6 in table 1 (111 mg, 67 % yield) as a pale yellow solid:
¹H-NMR (DMSO d₆): 10.50 (s,1H), 10.19 (s, 1H), 8.47 (s,1H), 7.97 (s,1H), 7.74 (s,1H), 7.57 (m, 1H), 7.35 (m, 2H), 7.14 (s, 1H), 6.91 (m, 2H), 4.99 (s, 2H), 4.26 (m, 1H), 4.18 (t, 2H), 3.92 (s, 3H), 3.44 (m, 2H), 2.58 (t, 2H), 2.49 (m, 2H), 2.39 (t, 2H), 1.89 (m, 2H), 1.29 (m, 2H), 0.81 (t, 3H) :
MS (+ve ESI): 552 (M+H)⁺,
MS (-ve ESI) : 550 (M-H)⁻.

### Example 7- Preparation of Compound 7 in Table 1- N-(2,3-difluorophenyl)-2-{3-[(7-{3-[(2R)-2-(hydroxymethyl)pyrrolidin-1-yl]propoxy}-6-methoxyquinazolin-4-yl)amino]-1H-pyrazol-1-yl}acetamide

An analogous reaction to that described in example 1, but starting with 2-(3-{[7-(3-chloropropoxy)-6-methoxyquinazolin-4-yl]amino}-1*H*-pyrazol-1-yl)-*N*-(2,3-difluorophenyl)acetamide hydrochloride (162 mg, 0.30 mmol) and D-prolinol (152 mg, 1.5 mmol) yielded compound 7 in table 1 (132 mg, 78 % yield) as a pale yellow solid:
¹H-NMR (DMSO d₆): 10.29 (br s, 1H), 10.21 (s, 1H), 8.47 (s, 1H), 7.97 (s, 1H), 7.72 (m, 2H), 7.19 (m, 3H), 6.90 (s, 1H), 5.09 (s, 2H), 4.29 (br s, 1H), 4.16 (t, 2H), 3.93 (s, 3H), 3.40 (m, 1H), 3.18 (m, 1H), 3.08 (m, 1H), 2.96 (m, 1H), 2.42 (m, 2H), 2.16 (m, 1H), 1.95 (m, 2H), 1.80 (m, 1H), 1.64 (m, 2H), 1.55 (m, 1H):
MS (+ve ESI): 568 (M+H)⁺,
MS (-ve ESI): 566 (M-H)⁻.
2-(3-{[7-(3-chloropropoxy)-6-methoxyquinazolin-4-yl]amino}-1*H*-pyrazol-1-yl)-*N*-(2,3-difluorophenyl)acetamide hydrochloride, used as the starting material, was obtained as follows:
a) An analogous reaction to that described in example 1d), but starting with 4-chloro-6-methoxy-7-(3-chloroxypropoxy)quinazoline (502 mg, 1.75 mmol) and 2-(3-amino-1*H*-pyrazol-1-yl)-*N*-(2,3-difluorophenyl)acetamide (440 mg, 1.75 mmol) yielded 2-(3-{[7-(3-chloropropoxy)-6-methoxyquinazolin-4-yl]amino}-1*H*-pyrazol-1-yl)-*N*-(2,3-difluorophenyl)acetamide (650 mg, 69 % yield) as a white solid:
   ¹H-NMR (DMSO d₆) : 11.80 (br s, 1H), 10.50 (br s, 1H), 8.91 (s, 1H), 8.26 (s, 1H), 7.90 (s, 1H), 7.59 (m, 1H), 7.47 (s, 1M, 7.20 (m, 2H), 6.90 (s, 1H), 5.20 (s, 2H), 4.29 (t, 2H), 3.97 (s, 3H), 3.84 (t, 2H), 2.31 (m, 2H):
   MS (+ve ESI): 503 (M+H)⁺,
   MS (-ve ESI) : 501 (M-H)⁻.

### Example 8 - Preparation of Compound 8 in Table 1- N-(2,3-difluorophenyl)-2-{3-[(7-{3-[(2-hydroxyethyl)(propyl)amino]propoxy}-6-methoxyquinazolin-4-yl)alnino]-1H-pyrazol-1-yl}acetamide

An analogous reaction to that described in example 1, but starting with 2-(3-{[7-(3-chloropropoxy)-6-methoxyquinazolin-4-yl]amino}-1*H*-pyrazol-1-yl)-*N*-(2,3-difluorophenyl)acetamide (162 mg, 0.30 mmol) and 2-(*n*-propylamino)ethanol (155 mg, 1.5 mmol) yielded compound 8 in table 1 (127 mg, 75 % yield) as a pale yellow solid :
¹H-NMR (DMSO d₆) : 10.28 (br s, 1H), 10.21 (br s, 1H), 8.48 (s, 1H), 7.98 (s, 1H), 7.74 (m, 2H), 7.20 (m, 2H), 7.15 (s, 1H), 6.92 (s, 1H), 5.09 (s, 2H), 4.27 (t, 1H), 4.17 (t, 2H), 3.93 (s, 3H), 3.44 (m, 2H), 2.59 (m, 2H), 2.50 (m, 2H), 2.39 (t, 2H), 1.89 (m, 2H), 1.39 (m, 2H), 0.82 (t, 3H):
MS (+ve ESI): 570 (M+H)⁺,
MS (-ve ESI): 568 (M-H)⁻.

### Example 9 - Preparation of compound 9 in table 2 - {(2R)-1-[3-({4-[(1-{2-[(3-fluorophenyl)amino]-2-oxoethyl}-1H-pyrazol-4-yl)amino]quinazolin-7-yl)oxy)propyl]pyrrolidin-2-yl}methyl dihydrogen phosphate

Di-*tert*-butyl diethylphosphoramidite (483 mg, 1.94 mmol) was added slowly to a solution of *N*-(3-fluorophenyl)-2-{3-[(7-{3-[(2*R*)-2-(hydroxymethyl)pyrrolidin-1-yl]propoxy}quinazolin-4-yl)amino]-1*H*-pyrazol-1-yl}acetamide (252 mg, 0.486 mmol) and tetrazole (102 mg, 1.46 mmol) in dimethylacetamide (1.5 ml) under nitrogen. The mixture was stirred at ambient temperature for 1 hour. Hydrogen peroxide (248 µl of a 30% solution in water, 2.19 mmol) was added slowly at 0 °C and the reaction mixture stirred for 1.5 hours at ambient temperature. The mixture was cooled to 0 °C and a saturated solution of sodium metabisulfite (1.0 ml of 5.0N aqueous solution, 5 mmol) added slowly with vigorous stirring. The mixture was stirred at ambient temperature for 20 minutes, diluted with water and the pH adjusted to 7 with an aqueous solution of potassium hydrogen carbonate. The reaction mixture was extracted with dichloromethane (2 x 5 ml) and the combined organics dried and concentrated *in vacuo.* Purification by silica gel chromatography, eluting with dichloromethane followed by increased polarity to dichloromethane : methanol : ammonia (20:1:0.1 to 20: 3: 0.2) gave di(*tert*-butyl) {(2*R*)-1-[3-({4-[(1-{2-[(3-fluorophenyl)amino]-2-oxoethyl}-1*H*-pyrazol-4-yl)amino]-quinazolin-7-yl}oxy)propyl]pyrrolidin-2-yl}methyl phosphate as a pale yellow oil. This intermediate was dissolved in dioxane (3 ml) and a solution of 4.0 N hydrochloric acid in dioxane (0.73 µl, 2.92 mmol) was added. The mixture was stirred at ambient temperature for 18 hours. The solid was filtered, washed with dichloromethane and diethyl ether and dried *in vacuo* for 18 hours (50 °C, 0.1 mm Hg) to yield compound 9 in table 2 (213 mg, 62 % yield) :
¹H-NMR (DMSO d₆): 8.96 (s, 1H), 8.80 (d, 1H), 7.90 (s, 1H), 7.61 (m, 1H), 7.40 (m, 4H), 6.92 (m, 1H), 6.86 (s, 1H), 5.12 (s, 2H), 4.32 (t, 2H), 4.21 (m, 2H), 3.77 (m, 1H), 3.64 (m, 2H), 3.29 (m, 1H), 3.19 (m, 1H), 2.32 (m, 2H), 2.19 (m, 1H), 1.97 (m, 2H), 1.81 (m, 1H) :
MS (+ve ESI): 600 (M+H)⁺,
MS (-ve ESI): 598 (M-H)⁻

### Example 10 - Preparation of compound 10 in table 2 - {(2R)-1-[3-({4-[(1-{2-[(2,3-difluorophenyl)amino]-2-oxoethyl}-1H-pyrazol-3-yl)amino]quinazolin-7-yl}oxy)propyl]pyrrolidin-2-yl}methyl dihydrogen phosphate

An analogous reaction to that described in example 9, but starting with N-(2,3-difluorophenyl)-2-{3-[(7-{3-[(2*R*)-2-(hydroxymethyl)pyrrolidin-1-yl]propoxy}quinazolin-4-yl)amino]-1*H*-pyrazol-1-yl}acetamide (243 mg, 0.452 mmol), yielded compound 10 in table 2 (239 mg, 74 % yield) :
¹H-NMR (DMSO d₆): 8.94 (s, 1H), 8.80 (d, 1H), 7.89 (s, 1H), 7.68 (m, 1H), 7.45 (m, 2H), 7.21 (m, 2H), 6.86 (m, 2H), 5.20 (s, 2H), 4.33 (t, 2H), 4.21 (m, 2H), 3.77 (m, 1H), 3.62 (m, 2H), 3.30 (m, 1H), 3.18 (m, 1H), 2.33 (m, 2H), 2.18 (m, 1H), 1.98 (m, 2H), 1.81 (m, 1H) :
MS (+ve ESI): 618 (M+H)⁺,
MS (-ve ESI): 616 (M-H)⁻

### Example 11- Preparation of compound 11 in table 2 - {(2R)-1-[3-({4-[(1-{2-[(3-fluorophenyl)amino]-2-oxoethyl}-1H-pyrazol-3-yl)amino]-6-methoxyquinazolin-7-yl}oxy)propyl]pyrrollidin-2-yl}methyl dihydrogen phosphate

An analogous reaction to that described in example 9 but starting with N-(3-fluorophenyl)-2-{3-[(7-{3-[(2*R*)-2-(hydroxymethyl)pyrrolidin-1-yl]propoxy}-6-methoxyquinazolin-4-yl)amino]-1*H*-pyrazol-1-yl}acetamide (278 mg, 0.506 mmol), yielded compound 11 in table 2 (295 mg, 79 % yield) :
¹H-NMR (DMSO d₆): 8.92 (s, 1H), 8.28 (s, 1H), 7.90 (s, 1H), 7.62 (m, 1H), 7.49 (s, 1H), 7.38 (m, 2H), 6.91 (m, 2H), 5.12 (s, 2H), 4.31 (t, 2H), 4.22 (m, 2H), 3.98 (s, 3H), 3.78 (m, 1H), 3.68 (m, 1H), 3.58 (m, 1H), 3.27 (m, 1H), 3.20 (m, 1H), 2.34 (m, 2H), 2.19 (m, 1H), 2.03 (m, 1H), 1.96 (m, 1H), 1.82 (m, 1H) :
MS (+ve ESI): 630 (M+H)⁺,
MS (-ve ESI): 628 (M-H)⁻

### Example 12 - Preparation of compound 12 in table 2 - {(2R)-1-[3-({4-[(1-{2-[(2,3-difluorophenyl)amino]-2-oxoethyl}-1H-pyrazol-3-yl)amino]-6-methoxyquinazolin-7-yl}oxy)propyl]pyrrolidin-2-yl}methyl dihydrogen phosphate

An analogous reaction to that described in example 9 but starting with *N*-(2,3-difluorophenyl)-2-{3-[(7-{3-[(2*R*)-2-(hydroxymethyl)pyrrolidin-1-yl]propoxy}-6-methoxyquinazolin-4-yl)amino]-1*H*-pyrazol-1-yl}acetamide (241 mg, 0.439 mmol), yielded compound 12 in table 2 (161 mg, 49 % yield) :
¹H-NMR (DMSO d₆): 8.93 (s, 1H), 8.30 (s, 1H), 7.90 (s, 1H), 7.68 (m, 1H), 7.51 (s, 1H), 7.20 (m, 2H), 6.90 (s, 1H), 5.20 (s, 2H), 4.31 (t, 2H), 4.20 (m, 2H), 3.99 (s, 3H), 3.78 (m, 1H), 3.68 (m, 1H), 3.59 (m, 1H), 3.27 (m, 1H), 3.20 (m, 1H), 2.34 (m, 2H), 2.19 (m, 1H), 2.02 (m, 1H), 1.95 (m, 1H), 1.82 (m, 1H):
MS (+ve ESI): 648 (M+H)⁺,
MS (-ve ESI): 646 (M-H)⁻

## Claims

1. A compound of formula (I) 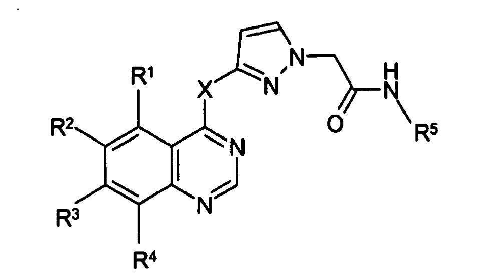 or a salt or ester thereof;
where:
**X** is O or NR⁶;
**R⁶** is hydrogen or C₁₋₄alkyl;
**R¹** is hydrogen, halo, or-X¹R¹¹;
**X¹** is a direct bond, -O-, -NH- or -N(C₁₋₆alkyl)-;
**R¹¹** is hydrogen, heterocyclyl or a group selected from C₁₋₆alkyl, C₂₋₆alkenyl, C₂₋₆ alkynyl, C₃₋₆cycloalkyl and C₃₋₆cycloalkenyl which group is optionally substituted by heterocyclyl, halo, hydroxy, C₁₋₄alkoxy or -NR⁹R¹⁰;
**R²** is hydrogen, halo, nitro, cyano or -X²R¹²;
**X²** is a direct bond, -O-, -NH- or -N(C₁₋₆alkyl)-;
**R¹²** is hydrogen, heterocyclyl or a group selected from aryl, C₁₋₆alkyl, C₂₋₆alkenyl, C₂₋₆ alkynyl, C₃₋₆cycloalkyl and C₃₋₆cycloalkenyl which group is optionally substituted by aryl, heterocyclyl, halo, hydroxy or -NR¹⁵R¹⁶;
**R³** is hydrogen, halo or-X³R¹³;
**X³** is a direct bond, -CH₂=CH₂-, -O-, -NH- or-N(C₁₋₆alkyl)-;
**R¹³** is hydrogen, heterocyclyl or a group selected from C₁₋₆alkyl, C₂₋₆alkenyl, C₂₋₆ alkynyl, C₃₋₆cycloalkyl and C₃₋₆cycloalkenyl which group is optionally substituted by -NR⁷R⁸, heterocyclyl, halo, hydroxy or C₁₋₄alkoxy;
**R⁷** and **R⁸** are independently selected from hydrogen, heterocyclyl, C₁₋₆alkyl, hydroxyC₁₋₆alkyl, C₁₋₃alkoxyC₁₋₆alkyl, C₃₋₆cycloalkyl, C₃₋₆cycloalkylC₁₋₃alkyl, hydroxyC₃₋₆cycloalkyl, hydroxyC₁₋₄alkylC₃₋₆cycloalkyl, hydroxyC₃₋₆cycloalkylC₁₋₃ alkyl, C₁₋₃alkoxyC₃₋₆cycloalkyl, C₁₋₃alkoxyC₃₋₆cycloalkylC₁₋₃alkyl, haloC₁₋₆alkyl, haloC₃₋₆cycloalkyl, haloC₃₋₆cycloalkylC₁₋₃alkyl, C₂₋₆alkenyl, C₂₋₆alkynyl, cyanoC₁₋₄ alkyl, aminoC₁₋₆alkyl, C₁₋₃alkylaminoC₁₋₆alkyl and bis(C₁₋₃alkyl)aminoC₁₋₆alkyl;
or **R⁷** and **R⁸** together with the nitrogen to which they are attached form a heterocyclic ring which ring comprises 4 to 7 ring atoms of which one is nitrogen and of which another is optionally selected from N, NH, O, S, SO and SO₂, and which ring is optionally substituted on carbon or nitrogen by 1 or 2 groups independently selected from C₁₋₄alkyl, hydroxy, C₁₋₄alkoxy, hydroxyC₁₋₄alkyl, hydroxyC₁₋₄alkoxyC₁₋₄alkyl and C₁₋₄alkoxyC₁₋₄alkoxy, and where a ring -CH₂- is optionally replaced with -C(O)-; **R⁴** is selected from hydrogen, halo or -X⁴R¹⁴;
**X⁴** is a direct bond, -O-, -NH- or -N(C₁₋₆alkyl)-;
**R¹⁴** is selected from hydrogen, C₁₋₆alkyl, C₂₋₆alkenyl and C₂₋₆alkynyl;
**R⁵** is aryl or heteroaryl optionally substituted by 1, 2 or 3 substituents independently selected from halo, hydroxy, cyano, nitro, amino, C₁₋₄alkylamino, bis(C₁₋₄ alkyl)amino, C₁₋₄alkyl, C₂₋₄alkenyl, C₂₋₄alkynyl, C₁₋₄alkoxy, C(O)NHR¹⁷, NHC(O)R¹⁸ and S(O)ₚR¹⁹ where p is 0, 1 or 2;
**R⁹**, **R¹⁰, R¹⁵** and **R¹⁶** are independently selected from hydrogen, C₁₋₆alkyl, C₃₋₆ cycloalkyl, C₃₋₆cycloalkylC₁₋₃alkyl, hydroxyC₁₋₆alkyl, haloC₁₋₆alkyl, aminoC₁₋₆alkyl, C₁₋₆alkylaminoC₁₋₆alkyl and bis(C₁₋₆alkyl)aminoC₁₋₆alkyl;
**R¹⁷**, **R¹⁸** and **R¹⁹** are independently selected from hydrogen, C₁₋₄alkyl, C₃₋₆cycloalkyl, C₂₋₄alkenyl and C₂₋₄alkynyl.

2. A compound according to claim 1, or a salt or ester thereof, wherein R⁵ is aryl optionally substituted by 1 or 2 halo.

3. A compound according to claim 2, or a salt or ester thereof, wherein R⁵ is 2,3-difluorophenyl or 3-fluorophenyl.

4. A compound according to any one of the preceding claims, or a salt or ester thereof, wherein X is NH.

5. A compound according to any one of the preceding claims, or a salt or ester thereof, wherein R¹ is hydrogen or -OR¹¹ and R¹¹ is hydrogen, heterocyclyl selected from piperidinyl and pyrrolidinyl or C₁₋₄alkyl (optionally substituted by hydroxy, C₁₋₄ alkoxy, amino, C₁₋₄alkylamino or bis(C₁₋₄alkyl)amino).

6. A compound according to any one of the preceding claims, or a salt or ester thereof, wherein R⁴ is hydrogen.

7. A compound according to claim 5, or a salt or ester thereof wherein R¹ is hydrogen and R⁴ is hydrogen.

8. A compound according to any one of the preceding claims, or a salt or ester thereof, wherein R² is hydrogen or -OR¹² and R¹² is hydrogen, C₁₋₄alkyl (optionally substituted by heterocyclyl) or heterocyclyl.

9. A compound according to claim 8, or a salt or ester thereof, wherein R² is hydrogen or methoxy.

10. A compound according to any one of the preceding claims, or a salt or ester thereof, wherein R³ is -X³R¹³, X³ is -CH₂=CH₂-, -O- or -NH- and R¹³ is C₁₋₆alkyl substituted by NR⁷R⁸, heterocyclyl or halo.

11. A compound according to claim 10, or a salt or ester thereof, wherein X³ is - O- and R¹³ is propyl substituted by chloro or -NR⁷R⁸.

12. A compound according to any one of the preceding claims, or a salt or ester thereof, wherein R⁷ and R⁸ are independently selected from hydrogen, heterocyclyl, C₁₋₆alkyl, hydroxyC₁₋₆alkyl, hydroxyC₁₋₄alkylC₃₋₆cycloalkyl, C₁₋₃alkoxyC₁₋₄alkyl, C₃₋₆ cycloalkyl, C₃₋₆cycloalkylC₁₋₃alkyl, haloC₁₋₆alkyl, C₂₋₆alkenyl, C₂₋₆alkynyl, cyanoC₁₋₄ alkyl and bis(C₁₋₃alkyl)aminoC₁₋₆alkyl; or R⁷ and R⁸ together with the nitrogen to which they are attached form a heterocyclic ring which ring comprises 4 to 7 ring atoms of which one is nitrogen and of which another is optionally NH and which ring is optionally substituted on carbon or nitrogen by a group selected from C₁₋₄alkyl, hydroxy, hydroxyC₁₋₄alkyl and hydroxyC₁₋₄alkoxyC₁₋₄alkyl, and where a ring -CH₂- is optionally replaced with -C(O)-.

13. A compound according to claim 12, or a salt or ester thereof wherein R⁷ and R⁸ are independently selected from hydrogen, methyl, ethyl, propyl, hydroxymethyl, 2-hydroxyethyl or 3-hydroxypropyl; or R⁷ and R⁸ together with the nitrogen to which they are attached form a heterocyclic ring selected from pyrrolidinyl, piperidinyl and piperazinyl, where the ring is optionally substituted by hydroxymethyl or 2-hydroxyethyl.

14. A compound of formula (IA) 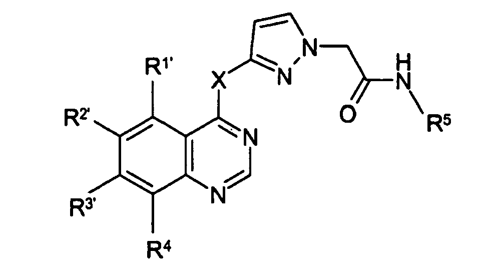 or a salt or ester thereof;
where X, X¹, X², X³, R⁴ and R⁵ are as defined in claim 1 and . **R^{1'}** is hydrogen, halo, or -X¹R^{11'};
**R^{11'}** is hydrogen, phosphonooxy, heterocyclyl or a group selected from C₁₋₆alkyl, C₂₋₆ alkenyl, C₂₋₆alkynyl, C₃₋₆cycloalkyl and C₃₋₆cycloalkenyl which group is optionally substituted by phosphonooxy, heterocyclyl, halo, hydroxy, C₁₋₄alkoxy or -NR^{9'}R^{10'}; **R^{2'}** is hydrogen, halo, nitro, cyano or-X²R^{12'};
**R^{12'}** is hydrogen, phosphonooxy, heterocyclyl or a group selected from aryl, C₁₋₆alkyl, C₂₋₆alkenyl, C₂₋₆alkynyl, C₃₋₆cycloalkyl and C₃₋₆cycloalkenyl which group is optionally substituted by phosphonooxy, aryl, heterocyclyl, halo, hydroxy or - NR^{15'}R^{16'};
**R^{3'}** is hydrogen, halo or -X³R^{13'} ;
**R^{13'}** is phosphonooxy or a group selected from C₁₋₆alkyl, C₂₋₆alkenyl, C₂₋₆alkynyl, C₃₋₆ cycloalkyl and C₃₋₆cycloalkenyl which group is substituted by -NR^{7'}R^{8'},
heterocyclyl, halo, hydroxy, phosphonooxy or C₁₋₄alkoxy;
**R**^{7'} and **R**^{8'} are independently selected from hydrogen, heterocyclyl, C₁₋₆alkyl, hydroxyC₁₋₆alkyl, phosphonooxyC₁₋₆alkyl, C₁₋₃alkoxyC₁₋₆alkyl, phosphonooxyC₁₋₄ alkoxyC₁₋₄alkyl, C₃₋₆cycloalkyl, C₃₋₆cycloalkylC₁₋₃alkyl, hydroxyC₃₋₆cycloalkyl, phosphonooxyC₃₋₆cycloalkyl, hydroxyC₁₋₄alkylC₃₋₆cycloalkyl, phosphonooxyC₁₋₄ alkylC₃₋₆cycloalkyl, hydroxyC₃₋₆cycloalkylC₁₋₃alkyl, phosphonooxyC₃₋₆cycloalkylC₁₋₃ alkyl, C₁₋₃alkoxyC₃₋₆cycloalkyl, C₁₋₃alkoxyC₃₋₆cycloalkylC₁₋₃alkyl, haloC₁₋₆alkyl, haloC₃₋₆cycloalkyl, haloC₃₋₆cycloalkylC₁₋₃alkyl, C₂₋₆alkenyl, C₂₋₆alkynyl, cyanoC₁₋₄ alkyl, aminoC₁₋₆alkyl, C₁₋₃alkylaminoC₁₋₆alkyl and bis(C₁₋₃alkyl)aminoC₁₋₆alkyl;
or **R^{7'}** and **R^{8'}** together with the nitrogen to which they are attached form a heterocyclic ring which ring comprises 4 to 7 ring atoms of which one is nitrogen and of which another is optionally selected from N, NH, O, S, SO and SO₂, and which ring is substituted on carbon or nitrogen by 1 or 2 groups independently selected from C₁₋₄alkyl, hydroxy, phosphonooxy, C₁₋₄alkoxy, hydroxyC₁₋₄alkyl, phoshonooxyC₁₋₄ alkyl, hydroxyC₁₋₄alkoxyC₁₋₄alkyl, phosphonooxyC₁₋₄alkoxyC₁₋₄alkyl and C₁₋₄ alkoxyC₁₋₄alkoxy, and where a ring -CH₂- is optionally replaced with a -C(O)-;
**R^{9'}, R^{10'}, R^{15'}** and **R^{16'}** are independently selected from hydrogen, C₁₋₆alkyl, C₃₋₆ cycloalkyl, C₃₋₆cycloalkylC₁₋₃alkyl, hydroxyC₁₋₆alkyl, phosphonooxyC₁₋₆alkyl, haloC₁₋₆alkyl, aminoC₁₋₆alkyl, C₁₋₆alkylaminoC₁₋₆alkyl and bis(C₁₋₆alkyl)aminoC₁₋₆ alkyl;
provided that a compound of formula (IA) contains at least one phosphonooxy group.

15. A compound according to claim 14 or a salt or ester thereof wherein the compound comprises only one phosphonooxy group.

16. A compound according to claim 14 or claim 15 or a salt or ester thereof wherein R⁵ is aryl optionally substituted by 1 or 2 halo.

17. A compound according to claim 16 or a salt or ester thereof wherein R⁵ is 2,3-difluorophenyl or 3-fluorophenyl.

18. A compound according to any one claims 14 to 17 or salt or ester thereof wherein X is NH.

19. A compound according to any one of claims 14 to 18 or a salt or ester thereof wherein R^{1'} is hydrogen or-OR^{11'} and R^{11'} is hydrogen, heterocyclyl selected from piperidinyl or pyrrolidinyl or C₁₋₄alkyl (optionally substituted by hydroxy, phosphonooxy, C₁₋₄alkoxy, amino, C₁₋₄alkylamino or bis(C₁₋₄alkyl)amino).

20. A compound according to any one of claims 14 to 19 or a salt or ester thereof wherein R⁴ is hydrogen.

21. A compound according to claim 19 or a salt or ester thereof wherein R^{1'} is hydrogen and R⁴ is hydrogen.

22. A compound according to any one of claims 14 to 21 or a salt or ester thereof wherein R^{2'} is hydrogen or -OR^{12'} and R^{12'} is hydrogen, C₁₋₄alkyl (optionally substituted by heterocyclyl) or heterocyclyl.

23. A compound according to claim 22 or salt or ester thereof wherein R^{2'} is hydrogen or methoxy.

24. A compound according to any one of claims 14 to 23 or a salt or ester thereof wherein R^{3'} is -X³R^{13'}, X³ is -CH₂=CH₂-, -O- or -NH- and R^{13'} is C₁₋₆alkyl substituted by -NR^{7'}R^{8'}, heterocyclyl or halo.

25. A compound according to any one of claims 14 to 24 or a salt or ester thereof wherein R^{7'} is selected from hydrogen, heterocyclyl, C₁₋₆alkyl, C₁₋₃alkoxyC₁₋₆alkyl, cyanoC₁₋₄alkyl and C₃₋₆cycloalkyl; R^{8'} is phosphonooxyC₁₋₄alkyl or phosphonooxyC₁₋₄alkylC₃₋₆cycloalkyl; or R^{7'} and R^{8'} together with the nitrogen to which they are attached form a heterocyclic ring selected from pyrrolidine, piperidine and piperazine which ring is substituted on carbon or nitrogen by a group selected from phosphonooxy, phosponooxymethyl and 2-phoshonooxyethyl.

26. A compound selected from:
*N*-(3-fluorophenyl)-2-{3-[(7-{3-[(2*R*)-2-(hydroxymethyl)pyrrolidin-1-yl]propoxy}quinazolin-4-yl)amino]-1*H*-pyrazol-1-yl}acetamide;
*N*-(3-fluorophenyl)-2-{3-[(7-{3-[(2-hydroxyethyl)(propyl)amino]propoxy}quinazolin-4-yl)amino]-1*H*-pyrazol-1-yl}acetamide;
*N*-(2,3-difluorophenyl)-2-{3-[(7-{3-[(2*R*)-2-(hydroxymethyl)pyrrolidin-1-yl]propoxy}quinazolin-4-yl)amino]-1*H*-pyrazol-1-yl}acetamide;
*N*-(2,3-difluorophenyl)-2-{3-[(7-{3-[(2-hydroxyethyl)(propyl)amino]propoxy}quinazolin4-yl)amino]-1*H*-pyrazol-1-yl}acetamide;
*N*-(3-fluorophenyl)-2-{3-[(7-{3-[(2*R*)-2-(hydroxymethyl)pyrrolidin-l-yl]propoxy} -6-methoxyquinazolin-4-yl)amino]-1*H*-pyrazol-1-yl}acetamide;
*N*-(3-fluorophenyl)-2-(3-[(7-{3-[(2-hydroxyethyl)(propyl)amino]propoxy}-6-methoxyquinazolin-4-yl)amino]-1*H*-pyrazol-1-yl}acetamide;
*N*-(2,3-difluorophenyl)-2-{3-[(7-{3-[(2*R*)-2-(hydroxymethyl)pyrrolidin-1-yl]propoxy}-6-methoxyquinazolin-4-yl)amino]-1*H*-pyrazol-1-yl}acetamide; and
*N*-(2,3-difluorophenyl)-2-{3-[(7-{3-[(2-hydroxyethyl)(propyl)amino]propoxy}-6-methoxyquinazolin-4-yl)amino]-1*H*-pyrazol-1-yl}acetamide;
{(2*R*)-1-[3-({4-[(1-{2-[(3-fluorophenyl)amino]-2-oxoethyl}-1*H*-pyrazol-4-yl)amino]quinazolin-7-yl}oxy)propyl]pynolidin-2-yl}methyl dihydrogen phosphate;
{(2*R*)-1-[3-({4-[(1-{2-[(2,3-difluorophenyl)amino]-2-oxoethyl}-1*H*-pyrazol-3-yl)amino]quinazolin-7-yl}oxy)propyl]pyrrolidin-2-yl}methyl dihydrogen phosphate;
{(2*R*)-1-[3-({4-[(1-{2-[(3-fluorophenyl)amino]-2-oxoethyl}-1*H*-pyrazol-3-yl)amino]-6-methoxyquinazolin-7-yl}oxy)propyl]pyrrolidin-2-yl)methyl dihydrogen phosphate; and
{(2*R*)-1-[3-({4-[(1-{2-[(2,3-difluorophenyl)ammo]-2-oxoethyl}-1*H*-pyrazol-3-yl)amino]-6-methoxyquinazolin-7-yl}oxy)propyl]pyrrolidin-2-yl}methyl dihydrogen phosphate;
or a pharmaceutically acceptable salt thereof.

27. A pharmaceutical composition comprising a compound of formula (I) as defined in any one of claims 1 to 13 or a pharmaceutically acceptable salt or ester thereof; a compound of formula (IA) as defined in any one of claims 14 to 25 or a pharmaceutically acceptable salt or ester thereof; or a compound as defined in claim 26 or a pharmaceutically acceptable salt thereof; in association with a pharmaceutically acceptable diluent or carrier.

28. A compound of formula (I) as defined in any one of claims 1 to 13 or a pharmaceutically acceptable salt or ester thereof; a compound of formula (IA) as defined in any one of claims 14 to 25 or a pharmaceutically acceptable salt or ester thereof; or a compound as defined in claim 26 or a pharmaceutically acceptable salt thereof; for use in therapy.

29. The use of a compound of formula (I) as defined in any one of claims 1 to 13 or a pharmaceutically acceptable salt or ester thereof; a compound of formula (IA) as defined in any one of claims 14 to 25 or a pharmaceutically acceptable salt or ester thereof; or a compound as defined in claim 26 or a pharmaceutically acceptable salt thereof; in the preparation of a medicament for the treatment of a hyperproliferative disease such as cancer.

30. The use as defined in claim 29 wherein the cancer is colorectal, breast, lung, prostate, bladder, renal or pancreatic cancer or leukaemia or lymphoma.

31. A process for the preparation of a compound of formula (I) as claimed in claim 1 or a salt or ester thereof, which process comprises reacting a compound of formula (II) 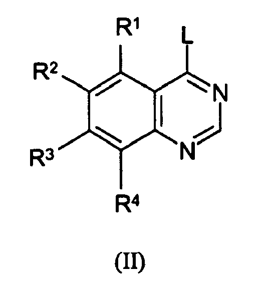 where L is a suitable leaving group such as chloro, bromo, SMe etc.
with a compound of formula (III) 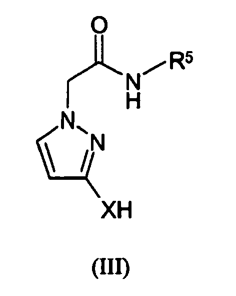 in the presence of hydrochloric acid in dioxane,
and thereafter if necessary:
i) converting a compound of the formula (I) into another compound of the formula (I); and/or
ii) removing any protecting groups; and/or
iii) forming a salt or ester thereof.

32. A process for the preparation of a compound of formula (IA) as defined in claim 14 or a salt or ester thereof, which process comprises phosphorylation of a suitable compound of formula (I) followed by deprotection of the phosphate group.

## Patentansprüche

1. Verbindung der Formel (I) 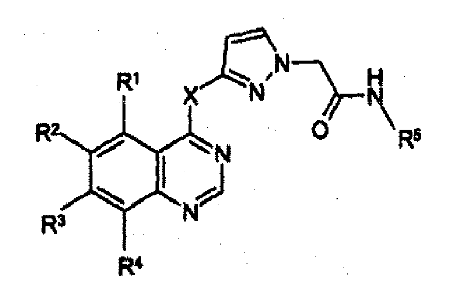 oder ein Salz oder Ester davon;
wobei:
X für O oder NR⁶ steht;
R⁶ für Wasserstoff oder C₁₋₄-Alkyl steht;
R¹ für Wasserstoff, Halogen oder -X¹R¹¹ steht;
X¹ für eine direkte Bindung, -O-, -NH- oder -N(C₁₋₆-Alkyl)- steht;
R¹¹ für Wasserstoff, Heterocyclyl oder eine unter C₁₋₆-Alkyl, C₂₋₆-Alkenyl, C₂₋₆-Alkinyl, C₃₋₆-Cycloalkyl und C₃₋₆-Cycloalkenyl ausgewählte Gruppe, die gegebenenfalls durch Heterocyclyl, Halogen, Hydroxy, C₁₋₄-Alkoxy oder -NR⁹R¹⁰ substituiert ist, steht;
R² für Wasserstoff, Halogen, Nitro, Cyano oder -X²R¹² steht;
X² für eine direkte Bindung, -O-, -NH- oder -N(C₁₋₆-Alkyl)- steht;
R¹² für Wasserstoff, Heterocyclyl oder eine unter Aryl, C₁₋₆-Alkyl, C₂₋₆-Alkenyl, C₂₋₆-Alkinyl, C₃₋₆-Cycloalkyl und C₃₋₆-Cycloalkenyl ausgewählte Gruppe, die gegebenenfalls durch Aryl, Heterocyclyl, Halogen, Hydroxy, oder -NR¹⁵R¹⁶ substituiert ist, steht;
R³ für Wasserstoff, Halogen oder -X³R¹³ steht;
X³ für eine direkte Bindung, -CH₂=CH₂-, -O-, -NH- oder -N (C₁₋₆-Alkyl)- steht;
R¹³ für Wasserstoff, Heterocyclyl oder eine unter C₁₋₆-Alkyl, C₂₋₆-Alkenyl, C₂₋₆-Alkinyl, C₃₋₆-Cycloalkyl und C₃₋₆-Cycloalkenyl ausgewählte Gruppe, die gegebenenfalls durch -NR⁷R⁸, Heterocyclyl, Halogen, Hydroxy oder C₁₋₄-Alkoxy substituiert ist, steht; R⁷ und R⁸ unabhängig voneinander unter Wasserstoff, Heterocyclyl, C₁₋₆-Alkyl, Hydroxy-C₁₋₆-alkyl, C₁₋₃-Alkoxy-C₁₋₆-alkyl, C₃₋₆-Cycloalkyl, C₃₋₆-Cycloalkyl-C₁₋₃-alkyl, Hydroxy-C₃₋₆-cycloalkyl, Hydroxy-C₁₋₄-alkyl-C₃₋₆-cycloalkyl, Hydroxy-C₃₋₆-Cycloalkyl-C₁₋₃-alkyl, C₁₋₃-Alkoxy-C₃₋₆-Cycloalkyl, C₁₋₃-Alkoxy-C₃₋₆-cycloalkyl-C₁₋₃-alkyl, Halogen-C₁₋₆-alkyl, Halogen-C₃₋₆-cycloalkyl, Halogen-C₃₋₆-Cycloalkyl-C₁₋₃-alkyl, C₂₋₆-Alkenyl, C₂₋₆-Alkinyl, Cyano-C₁₋₄-alkyl, Amino-C₁₋₆-alkyl, C₁₋₃-Alkylamino-C₁₋₆-alkyl und Bis (C₁₋₄-alkyl) amino-C₁₋₆-alkyl ausgewählt sind;
oder R⁷ und R⁸ gemeinsam mit dem Stickstoffatom, an das sie gebunden sind, einen heterocyclischen Ring bilden, der 4 bis 7 Ringatome, von denen eines Stickstoff ist und ein anderes gegebenenfalls unter N, NH, O, S, SO and SO₂ ausgewählt ist, enthält und gegebenenfalls an Kohlenstoff oder Stickstoff durch 1 oder 2 unabhängig voneinander unter C₁₋₄-Alkyl, Hydroxy, C₁₋₄-Alkoxy, Hydroxy-C₁₋₄-alkyl, Hydroxy-C₁₋₄-alkoxy-C₁₋₄-alkyl und C₁₋₄-Alkoxy-C₁₋₄-alkoxy ausgewählte Substituenten substituiert ist, und wobei ein Ring-CH₂-gegebenenfalls durch -C(O)- ersetzt ist;
R⁴ unter Wasserstoff, Halogen oder -X⁴R¹⁴ ausgewählt ist;
X⁴ für eine direkte Bindung, -O-, -NH- oder -N(C₁₋₆-Alkyl)- steht;
R¹⁴ unter Wasserstoff, C₁₋₆-Alkyl, C₂₋₆-Alkenyl und C₂₋₆-Alkinyl ausgewählt ist;
R⁵ für gegebenenfalls durch 1, 2 oder 3 unabhängig voneinander unter Halogen, Hydroxy, Cyano, Nitro, Amino, C₁₋₄-Alkylamino, Bis (C₁₋₄-alkyl) amino, C₁₋₄-Alkyl, C₂₋₄-Alkenyl, C₂₋₄-Alkinyl, C₁₋₄-Alkoxy, -C(O)NHR¹⁷, -NHC (O) R¹⁸ und -S (O)ₚR¹⁹, wobei p für 0, 1 oder 2 steht, ausgewählte Substituenten substituiertes Aryl oder Heteroaryl steht;
R⁹, R¹⁰, R¹⁵ und R¹⁶ unabhängig voneinander unter Wasserstoff, C₁₋₆-Alkyl, C₃₋₆-Cycloalkyl, C₃₋₆-Cycloalkyl-C₁₋₃-alkyl, Hydroxy-C₁₋₆-alkyl, Halogen-C₁₋₆-alkyl, Amino-C₁₋₆-alkyl, C₁₋₆-Alkylamino-C₁₋₆-alkyl und Bis (C₁₋₆-alkyl) amino-C₁₋₆-alkyl ausgewählt sind;
R¹⁷, R¹⁸ und R¹⁹ unabhängig voneinander unter Wasserstoff, C₁₋₄-Alkyl, C₃₋₆-Cycloalkyl, C₂₋₄-Alkenyl und C₂₋₄-Alkinyl ausgewählt sind.

2. Verbindung nach Anspruch 1 oder ein Salz oder Ester davon, worin R⁵ für gegebenenfalls durch 1 oder 2 Halogene substituiertes Aryl steht.

3. Verbindung nach Anspruch 2 oder ein Salz oder Ester davon, worin R⁵ für 2,3-Difluorphenyl oder 3-Fluorphenyl steht.

4. Verbindung nach einem der vorhergehenden Ansprüche oder ein Salz oder Ester davon, worin X für NH steht.

5. Verbindung nach einem der vorhergehenden Ansprüche oder ein Salz oder Ester davon, worin R¹ für Wasserstoff oder -OR¹¹ steht und R¹¹ für Wasserstoff, unter Piperidinyl und Pyrrolidinyl ausgewähltes Heterocyclyl oder C₁₋₄-Alkyl (gegebenenfalls substituiert durch Hydroxy, C₁₋₄-Alkoxy, Amino, C₁₋₄-Alkylamino oder Bis (C₁₋₄-alkyl) amino) steht.

6. Verbindung nach einem der vorhergehenden Ansprüche oder ein Salz oder Ester davon, worin R⁴ für Wasserstoff steht.

7. Verbindung nach Anspruch 5 oder ein Salz oder Ester davon, worin R¹ für Wasserstoff steht und R⁴ für Wasserstoff steht.

8. Verbindung nach einem der vorhergehenden Ansprüche oder ein Salz oder Ester davon, worin R² für Wasserstoff oder -OR¹² steht und R¹² für Wasserstoff, C₁₋₄-Alkyl (gegebenenfalls substituiert durch Heterocyclyl) oder Heterocyclyl steht.

9. Verbindung nach Anspruch 8 oder ein Salz oder Ester davon, worin R² für Wasserstoff oder Methoxy steht.

10. Verbindung nach einem der vorhergehenden Ansprüche oder ein Salz oder Ester davon, worin R³ für -X³R¹³ steht, X³ für -CH₂=CH₂-, -O- oder -NH- steht und R¹³ für C₁₋₆-Alkyl, das durch -NR⁷R⁸, Heterocyclyl oder Halogen substituiert ist, steht.

11. Verbindung nach Anspruch 10 oder ein Salz oder Ester davon, worin X³ für -O- steht und R¹³ für Propyl, das durch Chlor oder -NR⁷R⁸ substituiert ist, steht.

12. Verbindung nach einem der vorhergehenden Ansprüche oder ein Salz oder Ester davon, worin R⁷ und R⁸ unabhängig voneinander unter Wasserstoff, Heterocyclyl, C₁₋₆-Alkyl, Hydroxy-C₁₋₆-alkyl, Hydroxy-C₁₋₄-alkyl-C₃₋₆-Cycloalkyl, C₁₋₃-Alkoxy-C₁₋₄-alkyl, C₃₋₆-Cycloalkyl, C₃₋₆-Cycloalkyl-C₁₋₃-alkyl, Halogen-C₁₋₆-alkyl, C₂₋₆-Alkenyl, C₂₋₆-Alkinyl, Cyano-C₁₋₄-alkyl und Bis (C₁₋₃-alkyl) amino-C₁₋₆-alkyl, ausgewählt sind; oder R⁷ und R⁸ gemeinsam mit dem Stickstoffatom, an das sie gebunden sind, einen heterocyclischen Ring bilden, der 4 bis 7 Ringatome, von denen eines Stickstoff ist und ein anderes gegebenenfalls NH ist, enthält und gegebenenfalls an Kohlenstoff oder Stickstoff durch 1 oder 2 unabhängig voneinander unter C₁₋₄-Alkyl, Hydroxy, Hydroxy-C₁₋₄-alkyl und Hydroxy-C₁₋₄-alkoxy-C₁₋₄-alkyl ausgewählte Substituenten substituiert ist, und wobei ein Ring-CH₂- gegebenenfalls durch -C(O)- ersetzt ist.

13. Verbindung nach Anspruch 12 oder ein Salz oder Ester davon, worin R⁷ und R⁸ unabhängig voneinander unter Wasserstoff, Methyl, Ethyl, Propyl, Hydroxymethyl, 2-Hydroxyethyl oder 3-Hydroxypropyl ausgewählt sind; oder R⁷ und R⁸ gemeinsam mit dem Stickstoffatom, an das sie gebunden sind, einen unter Pyrrolidinyl, Piperidinyl und Piperazinyl ausgewählten heterocyclischen Ring bilden, der gegebenenfalls durch Hydroxymethyl oder 2-Hydroxyethyl substituiert ist.

14. Verbindung der Formel (IA) 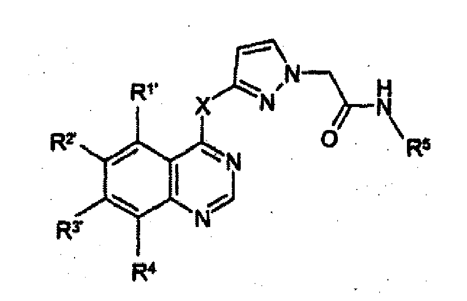 oder ein Salz oder Ester davon;
wobei:
X, X¹, X², X³, R⁴ und R⁵ die in Anspruch 1 angegebene Bedeutung besitzen und
R^{1'} für Wasserstoff, Halogen oder -X¹R^{11'} steht;
R^{11'} für Wasserstoff, Phosphonooxy, Heterocyclyl oder eine unter C₁₋₆-Alkyl, C₂₋₆-Alkenyl, C₂₋₆-Alkinyl, C₃₋₆-Cycloalkyl und C₃₋₆-Cycloalkenyl ausgewählte Gruppe, die gegebenenfalls durch Phosphonooxy, Heterocyclyl, Halogen, Hydroxy, C₁₋₄-Alkoxy oder -NR^{9'}R^{10'} substituiert ist, steht;
R^{2'} für Wasserstoff, Halogen, Nitro, Cyano oder -X²R^{12'} steht;
R^{12'} für Wasserstoff, Phosphonooxy, Heterocyclyl oder eine unter Aryl, C₁₋₆-Alkyl, C₂₋₆-Alkenyl, C₂₋₆-Alkinyl, C₃₋₆-Cycloalkyl und C₃₋₆-Cycloalkenyl ausgewählte Gruppe, die gegebenenfalls durch Phosphonooxy, Aryl, Heterocyclyl, Halogen, Hydroxy oder -NR^{15'}R^{16'} substituiert ist, steht;
R^{3'} für Wasserstoff, Halogen oder -X³R^{13'} steht;
R^{13'} für Phosphonooxy oder eine unter C₁₋₆-Alkyl, C₂₋₆-Alkenyl, C₂₋₆-Alkinyl, C₃₋₆-Cycloalkyl oder C₃₋₆-Cycloalkenyl ausgewählte Gruppe, die durch -NR^{7'}R^{8'}, Heterocycyl, Halogen, Hydroxy, Phosphonooxy oder C₁₋₄-Alkoxy substituiert ist, steht;
R^{7'} und R^{8'} unabhängig voneinander unter Wasserstoff, Heterocyclyl, C₁₋₆-Alkyl, Hydroxy-C₁₋₆-alkyl, Phosphonooxy-C₁₋₆-alkyl, C₁₋₃-Alkoxy-C₁₋₆-alkyl, Phosphonooxy-C₁₋₄-alkoxy-C₁₋₄-alkyl, C₃₋₆-Cycloalkyl, C₃₋₆-Cycloalkyl-C₁₋₃-alkyl, Hydroxy-C₃₋₆-cycloalkyl, Phosphonooxy-C₃₋₆-cycloalkyl, Hydroxy-C₁₋₄-alkyl-C₃₋₆-cycloalkyl, Phosphonooxy-C₁₋₄-alkyl-C₃₋₆-cycloalkyl, Hydroxy-C₃-₆-Cycloalkyl-C₁₋₃-alkyl, Phosphonooxy-C₃₋₆-cycloalkyl-C₁₋₃-alkyl, C₁₋₃-Alkoxy-C₃₋₆-cycloalkyl, C₁₋₃-Alkoxy-C₃₋₆-cycloalkyl-C₁₋₃-alkyl, Halogen-C₁₋₆-alkyl, Halogen-C₃₋₆-cycloalkyl, Halogen-C₃₋₆-cycloalkyl-C₁₋₃-alkyl, C₂₋₆-Alkenyl, C₂₋₆-Alkinyl, Cyano-C₁₋₄-alkyl, Amino-C₁₋₆-alkyl, C₁₋₃-Alkylamino-C₁₋₆-alkyl und Bis(C₁₋₃-alkyl)amino-C₁₋₆-alkyl ausgewählt sind;
oder R^{7'} und R^{8'} gemeinsam mit dem Stickstoffatom, an das sie gebunden sind, einen heterocyclischen Ring bilden, der 4 bis 7 Ringatome, von denen eines Stickstoff ist und ein anderes gegebenenfalls unter N, NH, O, S, SO and SO₂ ausgewählt ist, enthält und gegebenenfalls an Kohlenstoff oder Stickstoff durch 1 oder 2 unabhängig voneinander unter C₁₋₄-Alkyl, Hydroxy, Phosphonooxy, C₁₋₄-Alkoxy, Hydroxy-C₁₋₄-alkyl, Phosphonooxy-C₁₋₄-alkyl, Hydroxy-C₁₋₄-alkoxy-C₁₋₄-alkyl, Phosphonooxy-C₁₋₄-alkoxy-C₁₋₄-alkyl und C₁₋₄-Alkoxy-C₁₋₄-alkoxy ausgewählte Substituenten substituiert ist, und wobei ein Ring-CH₂-gegebenenfalls durch -C(O)- ersetzt ist;
R^{9'}, R^{10'}, R^{15'} und R^{16'} unabhängig voneinander unter Wasserstoff, C₁₋₆-Alkyl, C₃₋₆-Cycloalkyl, C₃₋₆-Cycloalkyl-C₁₋₃-alkyl, Hydroxy-C₁₋₆-alkyl, Phosphonooxy-C₁₋₆-alkyl, Halogen-C₁₋₆-alkyl, Amino-C₁₋₆-alkyl, C₁₋₆-Alkylamino-C₁₋₆-alkyl und Bis(C₁₋₆-alkyl)amino-C₁₋₆-alkyl ausgewählt sind;
mit der Maßgabe, daß eine Verbindung der Formel (IA) mindestens eine Phosphonooxygruppe enthält.

15. Verbindung nach Anspruch 14 oder ein Salz oder Ester davon mit nur einer Phosphonooxygruppe.

16. Verbindung nach Anspruch 14 oder 15 oder ein Salz oder Ester davon, worin R⁵ für gegebenenfalls durch 1 oder 2 Halogene substituiertes Aryl steht.

17. Verbindung nach Anspruch 16 oder ein Salz oder Ester davon, worin R⁵ für 2,3-Difluorphenyl oder 3-Fluorphenyl steht.

18. Verbindung nach einem der Ansprüche 14 bis 17 oder ein Salz oder Ester davon, worin X für NH steht.

19. Verbindung nach einem der Ansprüche 14 bis 18 oder ein Salz oder Ester davon, worin R^{1'} für Wasserstoff oder -OR^{11'} steht und R^{11'} für Wasserstoff, unter Piperidinyl und Pyrrolidinyl ausgewähltes Heterocyclyl oder C₁₋₄-Alkyl (gegebenenfalls substituiert durch Hydroxy, Phosphonooxy, C₁₋₄-Alkoxy, Amino, C₁₋₄-Alkylamino oder Bis (C₁₋₄-alkyl)amino) steht.

20. Verbindung nach einem der Ansprüche 14 bis 19 oder ein Salz oder Ester davon, worin R⁴ für Wasserstoff steht.

21. Verbindung nach Anspruch 19 oder ein Salz oder Ester davon, worin R^{1'} für Wasserstoff steht und R⁴ für Wasserstoff steht.

22. Verbindung nach einem der Ansprüche 14 bis 21 oder ein Salz oder Ester davon, worin R^{2'} für Wasserstoff oder -OR^{12'} steht und R^{12'} für Wasserstoff, C₁₋₄-Alkyl (gegebenenfalls substituiert durch Heterocyclyl) oder Heterocyclyl steht.

23. Verbindung nach Anspruch 22 oder ein Salz oder Ester davon, worin R^{2'} für Wasserstoff oder Methoxy steht.

24. Verbindung nach einem der Ansprüche 14 bis 23 oder ein Salz oder Ester davon, worin R^{3'} für -X³R^{13'} steht, X³ für -CH₂=CH₂-, -O- oder -NH- steht und R^{13'} für C₁₋₆-Alkyl, das durch -NR^{7'}R^{8'}, Heterocyclyl oder Halogen substituiert ist, steht.

25. Verbindung nach einem der Ansprüche 14 bis 24 oder ein Salz oder Ester davon, worin R^{7'} unter Wasserstoff, Heterocyclyl, C₁₋₆-Alkyl, C₁₋₃-Alkoxy-C₁₋₆-alkyl, Cyano-C₁₋₄-alkyl und C₃₋₆-Cycloalkyl ausgewählt ist; R^{8'} für Phosphonooxy-C₁₋₄-alkyl oder Phosphonooxy-C₁₋₄-alkyl-C₃₋₆-cycloalkyl steht; oder R^{7'} und R^{8'} gemeinsam mit dem Stickstoffatom, an das sie gebunden sind, einen unter Pyrrolidin, Piperidin und Piperazin ausgewählten heterocyclischen Ring bilden, der gegebenenfalls an Kohlenstoff oder Stickstoff durch eine unter Phosphonooxy, Phosphonooxymethyl und 2-Phosphonooxyethyl ausgewählte Gruppe substituiert ist.

26. Verbindung, ausgewählt unter:
*N*-(3-Fluorphenyl)-2-{3-[(7-{3-[(2*R*)-2-(hydroxymethyl)pyrrolidin-1-yl]propoxy}chinazolin-4-yl)-amino]-1*H*-pyrazol-1-yl}acetamid;
*N*-(3-Fluorphenyl)-2-{3-[(7-{3-[(2-hydroxyethyl)(propyl)amino]propoxy}chinazolin-4-yl)-amino]-1*H*-pyrazol-1-yl}acetamid;
*N*-(2,3-Difluorphenyl)-2-{3-[(7-{3-[(2*R*)-2-(hydroxymethyl)pyrrolidin-1-yl]propoxy}chinazolin-4-yl)amino]-1*H*-pyrazol-1-yl}acetamid;
*N*-(2,3-Difluorphenyl)-2-{3-[(7-{3-[(2-hydroxyethyl)(propyl)amino]propoxy}chinazolin-4-yl)-amino]-1*H*-pyrazol-1-yl}acetamid;
*N*-(3-Fluorphenyl)-2-{3-[(7-{3-[(2*R*)-2-(hydroxymethyl)pyrrolidin-1-yl]propoxy}-6-methoxychinazolin-4-yl)amino]-1*H*-pyrazol-1-yl}acetamid;
*N*-(3-Fluorphenyl)-2-{3-[(7-{3-[(2-hydroxyethyl)(propyl)amino]propoxy}-6-methoxychinazolin-4-yl)amino]-1*H*-pyrazol-1-yl}acetamid;
*N*-(2,3-Difluorphenyl)-2-{3-[(7-{3-[(2*R*)-2-(hydroxymethyl)pyrrolidin-1-yl]propoxy}-6-methoxy-chinazolin-4-yl)amino]-1*H*-pyrazol-1-yl}acetamid und
*N*-(2,3-Difluorphenyl)-2-{3-[(7-{3-[(2-hydroxyethyl)(propyl)amino]propoxy}-6-methoxychinazolin-4-yl)amino]-1*H*-pyrazol-1-yl}acetamid;
{(2*R*)-1-[3-({4-[(1-{2-[(3-Fluorphenyl)amino]-2-oxoethyl}-1*H*-pyrazol-4-yl)amino]chinazolin-7-yl}oxy)propyl]pyrrolidin-2-yl}methyldihydrogenphosphat;
{(2*R*)-1-[3-({4-[(1-{2-[(2,3-Difluorphenyl)amino]-2-oxoethyl}-1*H*-pyrazol-3-yl)amino]chinazolin-7-yl}oxy)propyl]pyrrolidin-2-yl}methyldihydrogenphosphat;
{(2R)-1-[3-({4-[(1-{2-[(3-Fluorphenyl)amino]-2-oxoethyl}-1*H*-pyrazol-3-yl)amino]-6-methoxychinazolin-7-yl}oxy)propyl]pyrrolidin-2-yl}methyldihydrogenphosphat und
{(2*R*)-1-[3-({4-[(1-{2-[(2,3-Difluorphenyl)amino]-2-oxoethyl}-1*H*-pyrazol-3-yl)amino]-6-methoxychinazolin-7-yl}oxy)propyl]pyrrolidin-2-yl}methyldihydrogenphosphat;
oder ein pharmazeutisch annehmbares Salz davon.

27. Pharmazeutische Zusammensetzung, enthaltend eine Verbindung der Formel (I) nach einem der Ansprüche 1 bis 13 oder ein pharmazeutisch annehmbares Salz oder einen pharmazeutisch annehmbaren Ester davon, eine Verbindung der Formel (IA) nach einem der Ansprüche 14 bis 25 oder ein pharmazeutisch annehmbares Salz oder einen pharmazeutisch annehmbaren Ester davon oder eine Verbindung nach Anspruch 26 oder ein pharmazeutisch annehmbares Salz davon zusammen mit einem pharmazeutisch annehmbaren Verdünnungsmittel oder Träger.

28. Verbindung der Formel (I) nach einem der Ansprüche 1 bis 13 oder ein pharmazeutisch annehmbares Salz oder einen pharmazeutisch annehmbaren Ester davon, Verbindung der Formel (IA) nach einem der Ansprüche 14 bis 25 oder ein pharmazeutisch annehmbares Salz oder einen pharmazeutisch annehmbaren Ester davon oder Verbindung nach Anspruch 26 oder ein pharmazeutisch annehmbares Salz davon zur Verwendung bei der Therapie.

29. Verwendung einer Verbindung der Formel (I) nach einem der Ansprüche 1 bis 13 oder eines pharmazeutisch annehmbaren Salzes oder Esters davon, einer Verbindung der Formel (IA) nach einem der Ansprüche 14 bis 25 oder eines pharmazeutisch annehmbaren Salzes oder Esters davon oder einer Verbindung der Formel (IA) nach Anspruch 26 oder eines pharmazeutisch annehmbaren Salzes oder Esters davon bei der Herstellung eines Arzneimittels zur Behandlung einer hyperproliferativen Erkrankung wie Krebs.

30. Verwendung nach Anspruch 29, bei der es sich bei dem Krebs um Kolorektal-, Brust-, Lungen-, Prostata-, Blasen-, Nieren- oder Bauchspeicheldrüsenkrebs oder Leukämie oder Lymphom handelt.

31. Verfahren zur Herstellung einer Verbindung der Formel (I) nach Anspruch 1 oder eines Salzes oder Esters davon, bei dem man eine Verbindung der Formel (II) 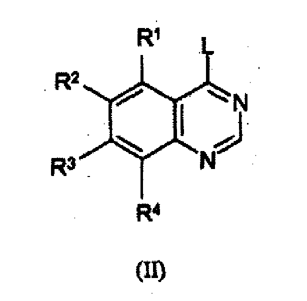 worin L für eine geeignete Abgangsgruppe wie Chlor, Brom, SMe usw. steht, in Gegenwart von Salzsäure in Dioxan mit einer Verbindung der Formel (III) 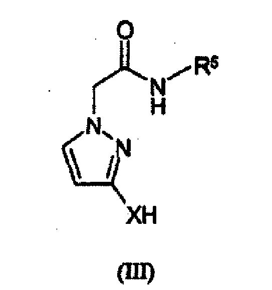 umsetzt
und danach gegebenenfalls:
i) eine Verbindung der Formel (I) in eine andere Verbindung der Formel (I) umwandelt und/oder
ii) gegebenenfalls vorhandene Schutzgruppen abspaltet und/oder
iii) ein Salz oder einen Ester davon bildet.

32. Verfahren zur Herstellung einer Verbindung der Formel (IA) nach Anspruch 14 oder eines Salzes oder Esters davon, bei dem man eine geeignete Verbindung der Formel (I) phosphoryliert und danach die Phosphatgruppe entschützt.

## Revendications

1. Composé de formule (I) 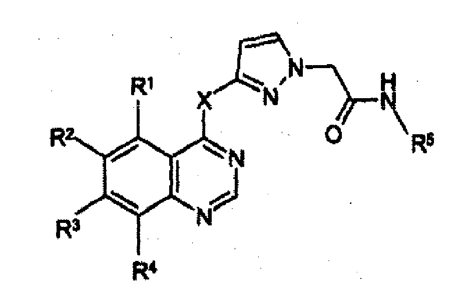 ou un sel ou ester de celui-ci ;
dans laquelle :
X est O ou NR⁶ ;
R⁶ est hydrogène ou C₁₋₄alkyle ;
R¹ est hydrogène, halogéno ou -X¹R¹¹ ;
X¹ est une liaison directe, -O-, -NH- ou -N(C₁₋₆ alkyl)- ;
R¹¹ est hydrogène, hétérocyclyle ou un groupement choisi parmi C₁₋₆alkyle, C₂₋₆alcényle, C₂₋₆alcynyle, C₃₋₆cycloalkyle et C₃₋₆cycloalcényle, lequel groupement est éventuellement substitué par hétérocyclyle, halogéno, hydroxy, C₁₋₄alcoxy ou -NR⁹R¹⁰;
R² est hydrogène, halogéno, nitro, cyano ou -X²R¹² ; X² est une liaison directe, -O-, -NH- ou -N(C₁₋₆ alkyl)- ;
R¹² est hydrogène, hétérocyclyle ou un groupement choisi parmi aryle, C₁₋₆alkyle, C₂₋₆alcényle, C₂₋₆ alcynyle, C₃₋₆cycloalkyle et C₃₋₆cycloalcényle, lequel groupement est éventuellement substitué par aryle, hétérocyclyle, halogéno, hydroxy, ou -NR¹⁵R¹⁶ ;
R³ est hydrogène, halogéno ou -X³R¹³ ;
X³ est une liaison directe, -CH₂=CH₂-, -O-, -NH- ou -N (C₁₋₆alkyl) - ;
R¹³ est hydrogène, hétérocyclyle ou un groupement choisi parmi C₁₋₆alkyle, C₂₋₆alcényle, C₂₋₆alcynyle, C₃₋₆cycloalkyle et C₃₋₆cycloalcényle, lequel groupement est éventuellement substitué par -NR⁷R⁸, hétérocyclyle, halogéno, hydroxy ou C₁₋₄alcoxy ;
R⁷ et R⁸ sont choisis indépendamment parmi hydrogène, hétérocyclyle, C₁₋₆alkyle, hydroxyC₁₋₆ alkyle, C₁₋₃alCOxyC₁₋₆alkyle, C₃₋₆cycloalkyle, C₃₋₆ cycloalkylC₁₋₃alkyle, hydroxyC₃₋₆cycloalkyle, hydroxyC₁₋₄al kylC₃₋₆cycloalkyle, hydroxyC₃₋₆ cycloalkylC₁₋₃alkyle, C₁₋₃alcoxyC₃₋₆cycloalkyle, C₁₋₃ alcoxyC₃₋₆cycloalkylC₁₋₃alkyle, halogénoC₁₋₆alkyle, halogénoC₃₋₆cycloalkyle, halogénoC₃₋₆cycloalkylC₁₋₃ alkyle, C₂₋₆alcényle, C₂₋₆alcynyle, cyanoC₁₋₄alkyle, aminoC₁₋₆alkyle, C₁₋₃alkylaminoC₁₋₆alkyle et bis (C₁₋₃ alkyl)aminoC₁₋₆alkyle ;
ou R⁷ et R⁸ ensemble avec l'azote auquel ils sont liés forment un cycle hétérocyclique, lequel cycle comprend de 4 à 7 atomes du cycle dont l'un est azote et dont l'autre est éventuellement choisi parmi N, NH, O, S, SO et SO₂, et lequel cycle est éventuellement substitué sur le carbone ou l'azote par 1 ou 2 groupements choisis indépendamment parmi C₁₋₄alkyle, hydroxy, C₁₋₄alcoxy, hydroxyC₁₋₄ alkyle, hydroxyC₁₋₄alcoxyC₁₋₄alkyle et C₁₋₄alcoxyC₁₋₄ alcoxy et où un -CH₂- du cycle est éventuellement remplacé par -C (O) - ;
R⁴ est choisi parmi hydrogène, halogéno ou -X⁴R¹⁴ ; X⁴ est une liaison directe, -O-, -NH- ou -N(C₁₋₆ alkyl)- ;
R¹⁴ est choisi parmi hydrogène, C₁₋₆alkyle, C₂₋₆ alcényle et C₂₋₆alcynyle ;
R⁵ est aryle ou hétéroaryle éventuellement substitué par 1, 2 ou 3 substituants choisis indépendamment parmi halogéno, hydroxy, cyano, nitro, amino, C₁₋₄alkylamino, bis (C₁₋₄alkyl) amino, C₁₋₄alkyle, C₂₋₄alcényle, C₂₋₄alcynyle, C₁₋₄alcoxy, C(O) NHR¹⁷, NHC(O)R¹⁸ et S(O)ₚR¹⁹, où p est 0, 1 ou 2 ;
R⁹, R¹⁰, R¹⁵ et R¹⁶ sont choisis indépendamment parmi hydrogène, C₁₋₆alkyle, C₃₋₆cycloalkyle, C₃₋₆ cycloalkylC₁₋₃alkyle, hydroxyC₁₋₆alkyle, halogénoC₁₋₆ alkyle, aminoC₁₋₆alkyle, C₁₋₄alkylaminoC₁₋₆alkyle et bis (C₁₋₆alkyl) aminoC₁₋₆alkyle ;
R¹⁷, R¹⁸ et R¹⁹ sont choisis indépendamment parmi hydrogène, C₁₋₄alkyle, C₃₋₆cycloalkyle, C₂₋₄alcényle et C₂₋₄alcynyle.

2. Composé selon la revendication 1, ou un sel ou ester de celui-ci, **caractérisé en ce que** R⁵ est aryle éventuellement substitué par 1 ou 2 halogéno.

3. Composé selon la revendication 2, ou un sel ou ester de celui-ci, **caractérisé en ce que** R⁵ est 2,3-difluorophényle ou 3-fluorophényle.

4. Composé selon l'une quelconque des revendications précédentes, ou un sel ou ester de celui-ci, **caractérisé en ce que** X est NH.

5. Composé selon l'une quelconque des revendications précédentes, ou un sel ou ester de celui-ci, **caractérisé en ce que** R¹ est hydrogène ou -OR¹¹ et R¹¹ est hydrogène, hétérocyclyle choisi parmi pipéridinyle et pyrrolidinyle ou C₁₋₄alkyle (éventuellement substitué par hydroxy, C₁₋₄alcoxy, amino, C₁₋₄alkylamino ou bis (C₁₋₄alkyl) amino).

6. Composé selon l'une quelconque des revendications précédentes, ou un sel ou ester de celui-ci, **caractérisé en ce que** R⁴ est hydrogène.

7. Composé selon la revendication 5, ou un sel ou ester de celui-ci, **caractérisé en ce que** R¹ est hydrogène et R⁴ est hydrogène.

8. Composé selon l'une quelconque des revendications précédentes, ou un sel ou ester de celui-ci, **caractérisé en ce que** R² est hydrogène ou -OR¹² et R¹² est hydrogène, C₁₋₄alkyle (éventuellement substitué par hétérocyclyle) ou hétérocyclyle.

9. Composé selon la revendication 8, ou un sel ou ester de celui-ci, **caractérisé en ce que** R² est hydrogène ou méthoxy.

10. Composé selon l'une quelconque des revendications précédentes, ou un sel ou ester de celui-ci, **caractérisé en ce que** R³ est -X³R¹³, X³ est -CH₂=CH₂-, -O- ou -NH- et R¹³ est C₁₋₆alkyle substitué par -NR⁷R⁸, hétérocyclyle ou halogéno.

11. Composé selon la revendication 10, ou un sel ou ester de celui-ci, **caractérisé en ce que** X³ est -O- et R¹³ est propyle substitué par chloro ou -NR⁷R⁸.

12. Composé selon l'une quelconque des revendications précédentes, ou un sel ou ester de celui-ci, **caractérisé en ce que** R⁷ et R⁸ sont choisis indépendamment parmi hydrogène, hétérocyclyle, C₁₋₆ alkyle, hydroxyC₁₋₆alkyle, hydroxyC₁₋₄alkylC₃₋₆ cycloalkyle, C₁₋₃alcoxyC₁₋₄alkyle, C₃₋₆cycloalkyle, C₃₋₆cycloalkylC₁₋₃alkyle, halogénoC₁₋₆alkyle, C₂₋₆ alcényle, C₂₋₆alcynyle, cyanoC₁₋₄alkyle et bis (C₁₋₃ alkyl) aminoC₁₋₆alkyle ; ou R⁷ et R⁸ ensemble avec l'azote auquel ils sont liés forment un cycle hétérocyclique, lequel cycle comprend de 4 à 7 atomes du cycle dont l'un est azote et dont l'autre est éventuellement NH et lequel cycle est éventuellement substitué sur le carbone ou l'azote par un groupement choisi parmi C₁₋₄alkyle, hydroxy, hydroxyC₁₋₄alkyle et hydroxyC₁₋₄alcoxyC₁₋₄alkyle, et où un -CH₂- du cycle est éventuellement remplacé par -C (O) -.

13. Composé selon la revendication 12, ou un sel ou ester de celui-ci, **caractérisé en ce que** R⁷ et R⁸ sont choisis indépendamment parmi hydrogène, méthyle, éthyle, propyle, hydroxyméthyle, 2-hydroxyéthyle ou 3-hydroxypropyle ; ou R⁷ et R⁸ ensemble avec l'azote auquel ils sont liés forment un cycle hétérocyclique choisi parmi pyrrolidinyle, pipéridinyle et pipérazinyle, où le cycle est éventuellement substitué par hydroxyméthyle ou 2-hydroxyéthyle.

14. Composé de formule (IA) 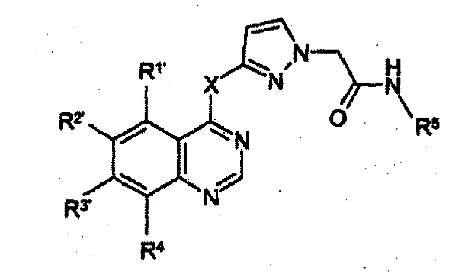 ou un sel ou ester de celui-ci ;
dans laquelle :
X, X¹, X², X³, R⁴ et R⁵ sont tels que définis dans la revendication 1 et
R^{1'} est hydrogène, halogéno ou -X¹R^{11'} ;
R^{11'} est hydrogène, phosphonooxy, hétérocyclyle ou un groupement choisi parmi C₁₋₆alkyle, C₂₋₆alcényle, C₂₋₆alcynyle, C₃₋₆cycloalkyle et C₃₋₆cycloalcényle, lequel groupement est éventuellement substitué par phosphonooxy, hétérocyclyle, halogéno, hydroxy, C₁₋₄ alcoxy ou -NR^{9'}R^{10'} ;
R^{2'} est hydrogène, halogéno, nitro, cyano ou -X²R^{12'} ;
R^{12'} est hydrogène, phosphonooxy, hétérocyclyle ou un groupement choisi parmi aryle, C₁₋₆alkyle, C₂₋₆ alcényle, C₂₋₆alcynyle, C₃₋₆cycloalkyle et C₃₋₆ cycloalcényle, lequel groupement est éventuellement substitué par phosphonooxy, aryle, hétérocyclyle, halogéno, hydroxy ou -NR^{15'} R^{16'} ;
R^{3'} est hydrogène, halogéno ou -X³R^{13'} ;
R^{13'} est phosphonooxy ou un groupement choisi parmi C₁₋₆alkyle, C₂₋₆alcényle, C₂₋₆alcynyle, C₃₋₆ cycloalkyle ou C₃₋₆cycloalcényle, lequel groupement est substitué par -NR^{7'}R^{8'}, hétérocycyle, halogéno, hydroxy, phosphonooxy ou C₁₋₄alcoxy ;
R^{7'} et R^{8'} sont choisis indépendamment parmi hydrogène, hétérocyclyle, C₁₋₆alkyle, hydroxyC₁₋₆ alkyle, phosphonooxyC₁₋₆alkyle, C₁₋₃alcoxyC₁₋₆alkyle, phosphonooxyC₁₋₄alcoxyC₁₋₄alkyle, C₃₋₆cycloalkyle, C₃₋₆ cycloalkylC₁₋₃alkyle, hydroxyC₃₋₆cycloalkyle, phosphonooxyC₃₋₆cycloalkyle, hydroxyC₁₋₄alkylC₃₋₆ cycloalkyle, phosphonooxyC₁₋₄alkylC₃₋₆cycloalkyle, hydroxyC₃₋₆cycloalkylC₁₋₃alkyle, phosphonooxyC₃₋₆ cycloalkylC₁₋₃alkyle, C₁₋₃alcoxyC₃₋₆cycloalkyle, C₁₋₃ alcoxyC₃₋₆cycloalkylC₁₋₃alkyle, halogénoC₁₋₆alkyle, halogènoC₃₋₆cycloalkyle, halogènoC₃₋₆cycloalkylC₁₋₃ alkyle, C₂₋₆alcényle, C₂₋₆alcynyle, cyanoC₁₋₄alkyle, aminoC₁₋₆alkyle, C₁₋₃alkylaminoC₁₋₆alkyle et bis (C₁₋₃ alkyl) aminoC₁₋₆alkyle ;
ou R^{7'} et R^{8'} ensemble avec l'azote auquel ils sont liés forment un cycle hétérocyclique, lequel cycle comprend de 4 à 7 atomes du cycle dont l'un est l'azote et dont un autre est éventuellement choisi parmi N, NH, O, S, SO et SO₂, et lequel cycle est substitué sur le carbone ou l'azote par 1 ou 2 groupements choisis indépendamment parmi C₁₋₄ alkyle, hydroxy, phosphonooxy, C₁₋₄alcoxy, hydroxyC₁₋₄alkyle, phosphonooxyC₁₋₄alkyle, hydroxyC₁₋₄ alcoxyC₁₋₄alkyle, phosphonooxyC₁₋₄alcoxyC₁₋₄alkyle et C₁₋₄alcoxyC₁₋₄alcoxy, et où un -CH₂- du cycle est éventuellement remplacé par un -C(O)- ;
R^{9'}, R^{10'}, R^{15'} et R^{16'} sont choisis indépendamment parmi hydrogène, C₁₋₆alkyle, C₃₋₆cycloalkyle, C₃₋₆ cycloalkylC₁₋₃alkyle, hydroxyC₁₋₆alkyle, phosphonooxyC₁₋₆alkyle, halogénoC₁₋₆alkyle, aminoC₁₋₆ alkyle, C₁₋₆alkylaminoC₁₋₆alkyle et bis (C₁₋₆ alkyl)aminoC₁₋₆alkyle ;
à condition qu'un composé de formule (IA) contienne au moins un groupement phosphonooxy.

15. Composé selon la revendication 14, ou un sel ou ester de celui-ci, **caractérisé en ce que** le composé ne comprend qu'un seul groupement phosphonooxy.

16. Composé selon la revendication 14 ou la revendication 15, ou un sel ou ester de celui-ci, **caractérisé en ce que** R⁵ est aryle éventuellement substitué par 1 ou 2 halogéno.

17. Composé selon la revendication 16, ou un sel ou ester de celui-ci, **caractérisé en ce que** R⁵ est 2,3-difluorophényle ou 3-fluorophényle.

18. Composé selon l'une quelconque des revendications 14 à 17, ou un sel ou ester de celui-ci, **caractérisé en ce que** X est NH.

19. Composé selon l'une quelconque des revendications 14 à 18, ou un sel ou ester de celui-ci, **caractérisé en ce que** R^{1'} est hydrogène ou -OR^{11'} et R^{11'} est hydrogène, hétérocyclyle choisi parmi pipéridinyle ou pyrrolidinyle ou C₁₋₄-alkyle (éventuellement substitué par hydroxy, phosphonooxy, C₁₋₄alcoxy, amino, C₁₋₄alkylamino ou bis(C₁₋₄alkyl)amino).

20. Composé selon l'une quelconque des revendications 14 à 19, ou un sel ou ester de celui-ci, **caractérisé en ce que** R⁴ est hydrogène.

21. Composé selon la revendication 19, ou un sel ou ester de celui-ci, **caractérisé en ce que** R^{1'} est hydrogène et R⁴ est hydrogène.

22. Composé selon l'une quelconque des revendications 14 à 21, ou un sel ou ester de celui-ci, **caractérisé en ce que** R^{2'} est hydrogène ou -OR^{12'} et R^{12'} est hydrogène, C₁₋₄alkyle (éventuellement substitué par hétérocyclyle) ou hétérocyclyle.

23. Composé selon la revendication 22, ou un sel ou ester de celui-ci, **caractérisé en ce que** R^{2'} est hydrogène ou méthoxy.

24. Composé selon l'une quelconque des revendications 14 à 23, ou un sel ou ester de celui-ci, **caractérisé en ce que** R^{3'} est -X³R^{13'}, X³ est -CH₂=CH₂-, -O- ou -NH- et R^{13'} est C₁₋₆alkyle substitué par -NR^{7'}R^{8'}, hétérocyclyle ou halogéno.

25. Composé selon l'une quelconque des revendications 14 à 24, ou un sel ou ester de celui-ci, **caractérisé en ce que** R^{7'} est choisi parmi hydrogène, hétérocyclyle, C₁₋₆alkyle, C₁₋₃alcoxyC₁₋₆ alkyle, cyanoC₁₋₄alkyle et C₃₋₆cycloalkyle ; R^{8'} est phosphonooxyC₁₋₄alkyle ou phosphonooxyC₁₋₄alkylC₃₋₆ cycloalkyle ; ou R^{7'} et R^{8'} ensemble avec l'azote auquel ils sont liés forment un cycle hétérocyclique choisi parmi pyrrolidine, pipéridine et pipérazine, lequel cycle est substitué sur le carbone ou l'azote par un groupement choisi parmi phosphonooxy, phosphonooxyméthyle et 2-phosphonooxyéthyle.

26. Composé choisi parmi :
le *N*-(3-fluorophényl)-2-{3-[(7-{3-[(2*R*)-2-(hydroxyméthyl)pyrrolidin-1-yl]propoxy}quinazolin-4-yl)amino]-1*H*-pyrazol-1-yl}acétamide ;
le *N*-(3-fluorophényl)-2-{3-[(7-{3-[(2-hydroxyéthyl)(propyl)amino]propoxy}quinazolin-4-yl)amino]-1*H*-pyrazol-1-yl}acétamide;
le *N*-(2,3-difluorophényl)-2-{3-[(7-{3-[(2*R*)-2-(hydroxyméthyl)pyrrolidin-1-yl]propoxy}quinazolin-4-yl)amino]-1*H*-pyrazol-1-yl}acétamide ;
le *N*-(2,3-difluorophényl)-2-{3-[(7-{3-[(2-hydroxyéthyl)(propyl)amino]propoxy}quinazolin-4-yl)amino]-1*H*-pyrazol-1-yl}acétamide ;
le *N*-(3-fluorophényl)-2-{3-[(7-{3-[(2*R*)-2-(hydroxyméthyl)pyrrolidin-1-yl]propoxy}-6-méthoxyquinazolin-4-yl)amino]-1*H*-pyrazol-1-yl}acétamide ;
le *N*-(3-fluorophényl)-2-{3-[(7-{3-[(2-hydroxyéthyl)(propyl)amino]propoxy}-6-méthoxyquinazolin-4-yl)amino]-1*H*-pyrazol-1-yl}acétamide ;
le *N*-(2,3-difluorophényl)-2-{3-[(7-{3-[(2*R*)-2-(hydroxyméthyl)pyrrolidin-1-yl]propoxy}-6-méthoxyquinazolin-4-yl)amino]-1*H*-pyrazol-1-yl}acétamide et
le *N*-(2,3-difluorophényl)-2-{3-[(7-{3-[(2-hydroxyéthyl)(propyl)amino]propoxy}-6-méthoxyquinazolin-4-yl)amino]-1*H*-pyrazol-1-yl}acétamide ;
le dihydrogénophosphate de {(2*R*)-1-[3-({4-[(1-{2-[(3-fluorophényl)amino]-2-oxoéthyl}-1*H*-pyrazol-4-yl)amino]quinazolin-7-yl}oxy)propyl]pyrrolidin-2-yl}méthyle ;
le dihydrogénophosphate de {(2*R*)-1-[3-({4-[(1-{2-[(2,3-difluorophényl)amino]-2-oxoéthyl}-1*H-*pyrazol-3-yl)amino]quinazolin-7-yl}oxy)propyl]pyrrolidin-2-yl}méthyle ;
le dihydrogénophosphate de {(2*R*)-1-[3-({4-[(1-{2-[(3-fluorophényl)amino]-2-oxoéthyl}-1*H*-pyrazol-3-yl)amino]-6-méthoxyquinazolin-7-yl}oxy)propyl]pyrrolidin-2-yl}méthyle et
le dihydrogénophosphate de {(2*R*)-1-[3-({4-[(1-{2-[(2,3-difluorophényl)amino]-2-oxoéthyl}-1*H-*pyrazol-3-yl)amino]-6-méthoxyquinazolin-7-yl}oxy)propyl]pyrrolidin-2-yl}méthyle ;
ou un sel pharmaceutiquement acceptable de celui-ci.

27. Composition pharmaceutique comprenant un composé de formule (I) telle que définie dans l'une quelconque des revendications 1 à 13 ou un sel ou ester pharmaceutiquement acceptable de celui-ci ; un composé de formule (IA) telle que définie dans l'une quelconque des revendications 14 à 25 ou un sel ou ester pharmaceutiquement acceptable de celui-ci ; ou un composé tel que défini dans la revendication 26 ou un sel pharmaceutiquement acceptable de celui-ci ; en association avec un diluant ou un support pharmaceutiquement acceptable.

28. Composé de formule (I) tel que défini dans l'une quelconque des revendications 1 à 13 ou un sel ou ester pharmaceutiquement acceptable de celui-ci ; composé de formule (IA) telle que définie dans l'une quelconque des revendications 14 à 25 ou un sel ou ester pharmaceutiquement acceptable de celui-ci ; ou composé tel que défini dans la revendication 26 ou un sel pharmaceutiquement acceptable de celui-ci ; destinés à être utilisés en thérapie.

29. Utilisation d'un composé de formule (I) telle que définie dans l'une quelconque des revendications 1 à 13 ou d'un sel ou d'un ester pharmaceutiquement acceptable de celui-ci ; d'un composé de formule (IA) telle que définie dans l'une quelconque des revendications 14 à 25 ou d'un sel ou d'un ester pharmaceutiquement acceptable de celui-ci ; ou d'un composé tel que défini dans la revendication 26 ou d'un sel pharmaceutiquement acceptable de celui-ci ; dans la préparation d'un médicament destiné au traitement d'une maladie hyperproliférative telle que le cancer.

30. Utilisation telle que définie dans la revendication 29, **caractérisée en ce que** le cancer est le cancer colorectal, du sein, du poumon, de la prostate, de la vessie, du rein ou du pancréas, ou les leucémies ou les lymphomes.

31. Procédé de préparation d'un composé de formule (I) selon la revendication 1, ou d'un sel ou d'un ester de celui-ci, lequel procédé comprend la réaction d'un composé de formule (II) 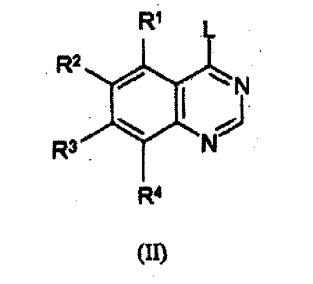 dans laquelle L est un groupement partant convenable tel que chloro, bromo, SMe et similaires, avec un composé de formule (III) 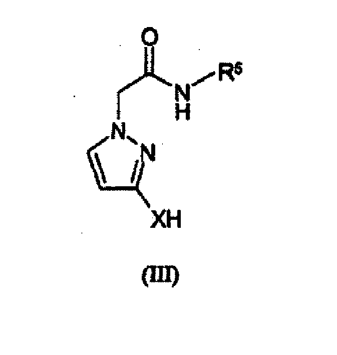 en présence d'acide chlorhydrique dans du dioxane, et ensuite le cas échéant :
i) la transformation d'un composé de formule (I) en un autre composé de formule (I) ; et/ou
ii) l'élimination de tous groupements protecteurs ; et/ou
iii) la formation d'un sel ou d'un ester de celui-ci.

32. Procédé de préparation d'un composé de formule (IA) telle que définie dans la revendication 14 ou d'un sel ou d'un ester de celui-ci, lequel procédé comprend la phosphorylation d'un composé convenable de formule (I), suivie de la déprotection du groupement phosphate.
